(19)

**Europäisches Patentamt**

**European Patent Office**

**Office européen des brevets**

(11)     **EP 3 901 288 A1**

(12)     **EUROPEAN PATENT APPLICATION**

(43) Date of publication:
**27.10.2021  Bulletin 2021/43**

(51) Int Cl.:
*C12Q 1/6886* (2018.01)     *G16B 25/10* (2019.01)

(21) Application number: **20170321.2**

(22) Date of filing: **20.04.2020**

| | |
|---|---|
| (84) Designated Contracting States:<br>**AL AT BE BG CH CY CZ DE DK EE ES FI FR GB<br>GR HR HU IE IS IT LI LT LU LV MC MK MT NL NO<br>PL PT RO RS SE SI SK SM TR**<br>Designated Extension States:<br>**BA ME**<br>Designated Validation States:<br>**KH MA MD TN**<br><br>(71) Applicants:<br>• **Fraunhofer-Gesellschaft zur Förderung der<br>  angewandten Forschung e.V.<br>  80686 München (DE)**<br>• **Universität Leipzig<br>  04109 Leipzig (DE)**<br><br>(72) Inventors:<br>• **Reiche, Kristin<br>  04416 Markkleeberg (DE)**<br>• **Blumert, Conny<br>  04109 Leipzig (DE)** | • **Horn, Friedemann<br>  04316 Leipzig (DE)**<br>• **Kreuz, Markus<br>  04299 Leipzig (DE)**<br>• **Otto, Dominik<br>  04107 Leipzig (DE)**<br>• **Bertram, Catharina<br>  04229 Leipzig (DE)**<br>• **Hackermüller, Jörg<br>  04105 Leipzig (DE)**<br>• **Wirth, Manfred<br>  01326 Dresden (DE)**<br>• **Füssel, Susanne<br>  01309 Dresden (DE)**<br>• **Fröhner, Michael<br>  01307 Dresden (DE)**<br><br>(74) Representative: **CH Kilger Anwaltspartnerschaft<br>  mbB<br>  Fasanenstraße 29<br>  10719 Berlin (DE)** |

(54)     **MULTI-GENE EXPRESSION ASSAY FOR PROSTATE CARCINOMA**

(57)     Prostate cancer is the most prevalent solid cancer among men in Western Countries. The clinical behavior of localized prostate cancer is highly variable. Hence, there is a high clinical need for precise biomarkers for identification of aggressive disease in addition to established clinical parameters. The present invention relates to a prognostic multi-gene expression score based on transcriptome-wide gene expression analysis providing a method for calculating a score (ProstaTrend score) that allows the prediction of death of disease (DoD) and/or biochemical recurrence (BCR), long-term prognosis and outcome of prostate cancer patients. The present invention is an independent predictor of prognosis and is suitable for the identification of high-risk patients among patients with a clinical classification of low or intermediate risk prior and after surgery, such as radical prostatectomy.

**EP 3 901 288 A1**

## Description

FIELD OF THE INVENTION

The present invention is directed to a method for predicting the outcome of prostate carcinoma patients. The present invention (ProstaTrend) provides a method for molecular patient risk stratification after treatment, such as radical prostatectomy. The present invention also offers a method that can be used in the clinical decision-making process for potential scheduling of adjuvant therapy or evaluating biopsies in the context of active surveillance and focal therapies.

BACKGROUND OF THE INVENTION

Prostate cancer is the most common solid cancer among men both in the US and in Europe. Existing diagnostic methods are often unable to precisely classify risk groups, thereby leading similarly to overdiagnosis and overtreatment as well as to insufficient detection of high-risk patients. An underlying reason is the highly variable clinical behavior of localized prostate cancer and that common classification methods of prostate cancer are often based on clinical and histological parameters only. Aggressive types of prostate cancer require radical treatment while indolent ones may be suitable for active surveillance or organ preserving focal therapies. Yet, stratification by clinical risk categories or available molecular tests lack sufficient precision. Therefore, additional molecular markers are needed to improve the stratification and outcome prediction in men with prostate cancer. Currently, tools for risk stratification based on gene expression are available and have been shown to outperform risk assessment by clinical parameters alone. An example for risk stratification tools are Oncotype (Cullen et al., 2015; Eur Urol), which predicts adverse pathology after biopsy, Decipher (Erho et al., 2013; PLoS ONE), which predicts metastasis-free survival after radical prostatectomy (RP) and Prolaris (Cuzick et al., 2015; Br J Cancer and 2011; Lancet Oncol), which is the only assay suitable for long-term prognosis after surgery, such as RP. However, none of the previously cited tools evaluate all tumor-relevant signaling pathways and therefore provide a limited molecular risk discrimination. Therefore, the problem to be solved is the persisting high clinical need for improved methods using gene expression signatures for long-term prognosis for prostate cancer patients prior and after surgery.

The present invention aims to solve the problem described above, which can be summarized as the urgent need for improved classification and risk stratification of prostate cancer patients. The solution provided by the present invention comprises the calculation of a predictive score on the basis of the expression values of up to 1396 gene transcripts (RNA biomarkers) from a set of genes associated with (prostate) cancer and (prostate) cancer patient outcome.

The inventors developed a method for calculating and applying a prognostic multi-gene expression score based on transcriptome-wide gene expression analysis (ProstaTrend), which allows improved molecular risk stratification, improved prediction of long-term prognosis and counselling of prostate cancer patients after surgery, such as radical prostatectomy, regarding adjuvant therapy options as well as evaluating biopsies prior to radical prostatectomy in the context of active surveillance and focal therapies.

SUMMARY OF THE INVENTION

The present invention provides a method (ProstaTrend) for molecular risk stratification and improved prediction of long-term prognosis for prostate cancer patients with the method comprising calculating and applying a prognostic multi-gene expression score (herein also referred to as ProstaTrend score) based on the expression of a set of genes that are significantly associated with prostate cancer prognosis in a patient sample. The calculation of the prognostic ProstaTrend score comprises the normalization and standardization of the expression values (abundancies) of marker genes (Table 2) in a patient sample and multiplication with the respective logHR values (listed in Table 2).

The method disclosed by the present invention further allows the prediction of death of disease (DoD) and biochemical recurrence (BCR) for long-term prognosis and outcome for prostate cancer patients. The method provided by the invention (ProstaTrend) is independent from clinical parameters after diagnosis and facilitates the identification of high-risk patients among patients with low or intermediate histological risk profiles. The herein disclosed invention also relates to a method for use in the treatment of prostate cancer, wherein the outcome or classification of the disease of a prostate carcinoma patient from whom the sample was derived is predicted based on the calculated gene expression score value.

The invention also relates to a method for predicting the outcome or classifying the disease of a prostate carcinoma patient, the method comprising the steps of: a) obtaining a sample from said patient, wherein the sample comprises a plurality of nucleic acids, b) analyzing the plurality of nucleic acids, thereby determining the abundancy of at least one or more different target nucleic acid sequences, c) normalizing of the target nucleic acid sequence abundancies against a reference dataset and standardization, d) weighting the abundancy of the at least one or more different target nucleic acid using a corresponding reference value and e) calculating a score value, and wherein, if the score value is above a threshold value of 0, the patient is classified as having a high risk of death of disease and/or biochemical recurrence and/or if the score value is below a threshold value of 0, the patient is classified as having a low risk of death of disease and/or biochemical recurrence.

Herein, each of the one or more different target nucleic acid sequences may be identical to, without being limited to, the nucleic acid sequence of a corresponding specific reference gene, a transcript or an isoform of a reference gene, which may be selected, without being limited to, from the group of reference genes listed in Table 2.

Herein, the weighting of said abundancy of the target nucleic acids may be performed, without being limited to, by weighting said abundancy of the target nucleic acids by the logHR value of a corresponding reference gene listed in Table 2, wherein the nucleic acid sequence of said reference gene, or of a transcript or isoform thereof, is identical to the nucleic acid sequence of one or more of said target nucleic acid sequences from said sample.

Herein, a biological sample may be derived from, without being limited to, a human patient, a cell culture, an "organ-on-a-chip" model, a microphysiological system or an animal. Herein, a biological sample may be an organ, a tissue, a biopsy, a liquid biopsy, a blood sample, a sample from a Patient Derived Xenograft, a sample from an "organ-on-a-chip" model, a sample from a microphysiological system, a cell line or cell culture of a patient-derived sample of a human patient, an animal or an animal model, of a cell line or of an organoid.

Furthermore, herein the analysis of the nucleic acid sequences may comprise, but is not limited to, optional lysis of the tissue and/or the cells and optional subsequent purification of respective nucleic acids followed by optional fragmentation, enzymatic digestion or ligation of respective nucleic acids, reverse transcription, microarray analysis, Next Generation Sequencing analysis and any other Sequencing method such as, but not limited to, nanopore sequencing, third-generation sequencing, Sanger sequencing analysis, digital or droplet PCR, quantitative Real-time PCR analysis and/or PCR amplification of the nucleic acids derived from respective sample and/or a sequential or parallel combination of previously listed steps and techniques.

Herein, Next Generation Sequencing relates to, without being limited to, RNA sequencing, DNA sequencing, Whole Genome Sequencing, Whole Exome Sequencing, Single Cell Sequencing, Targeted Sequencing, NanoString Hyb & Seq NGS analysis, nCounteranalysis, sequencing methods allowing two-dimensional resolution of transcriptome or epigenome patterns in tissues such as, but not limited to, spatial sequencing or sequencing methods allowing the characterization of chromatin status such as, but not limited to, ATAC-Seq or ChIP-Seq.

Herein, the gene expression of said reference genes in said sample can be analyzed by, without being limited to, transcriptome-wide gene expression analysis comprising, without limitation to, the analysis techniques and methods of nucleic acids and nucleic acid sequences described herein. Herein, the gene expression of reference genes in said sample can be calculated, without being limited to, using the abundancies of target nucleic acids and/or target nucleic acid sequences in or obtained from a patient sample.

The score, herein also referred to as ProstaTrend score, may be calculated herein, without being limited to, by using the expression values of said target genes and/or the abundancies of each one or more target nucleic acid sequences in said sample or derived from said sample, which each possess an identical nucleic acid sequence to one of the genes, or of a transcript or isoform thereof, that are listed in Table 2.

In greater detail, in the herein disclosed method for predicting the outcome or classifying the disease of prostate carcinoma patients said score value for said patient may be calculated by, without being limited to, using the abundancies of said target nucleic acid sequences in said sample to calculate expression values for all corresponding reference genes in said sample, and wherein for each reference gene the abundancies of all target nucleic sequences that are identical to the nucleic acid sequence of at least one or more transcripts and/or isoforms of said reference gene are summarized for each reference gene, and wherein for each patient k the median over the weighted standardized gene expression values of all reference genes selected for said calculation $g_i$ is calculated applying:

$$score_k = Median(logHR_i * g_i),$$

and

wherein the weight for each significant gene i is the estimated univariate log hazard ratio *(logHR)* from the Cox regression meta-analysis of a training cohort to account for the direction of the prognostic effect of said gene *i* as well as for the effect size, and wherein a normalization of said gene expression values is done cohort-wise, if a set of samples from the same cohort is provided, or, in case of a single sample, an add-on normalization with respect to the normalized gene expression values of a training cohort is conducted, and wherein a negative value of said score characterizes said patient with lower risk of an adverse prognosis and/or death of disease and an increased chance of extended biochemical recurrence-free and/or death of disease-free survival, and a positive value of said score characterizes said patient with a higher risk of an adverse prognosis and/or death of disease and a decreased chance of extended biochemical recurrence-free and/or death of disease-free survival.

The herein disclose invention may also relate to a method for use in the treatment of prostate cancer, with the method comprising the steps of: a) obtaining a sample from a patient, wherein the sample comprises a plurality of nucleic acids, b) analyzing the plurality of nucleic acids, thereby determining the abundancy of at least one or more different target nucleic acid sequences, c) normalizing of the target nucleic acid sequence abundancies against a reference dataset and standardization, d) weighting the abundancy of the at least one or more different target nucleic acid using a corresponding reference value and calculating a score value, and wherein the outcome or classification of the disease of a prostate carcinoma patient from whom said sample was derived is predicted based on said score value, and wherein, if the score value is above a threshold value of 0, the patient is classified as having a high risk of death of disease and/or biochemical recurrence and/or if the score value is below a threshold value of 0, the patient is classified as having a low risk of death of disease and/or biochemical recurrence.

BRIEF DESCRIPTION OF THE DRAWINGS

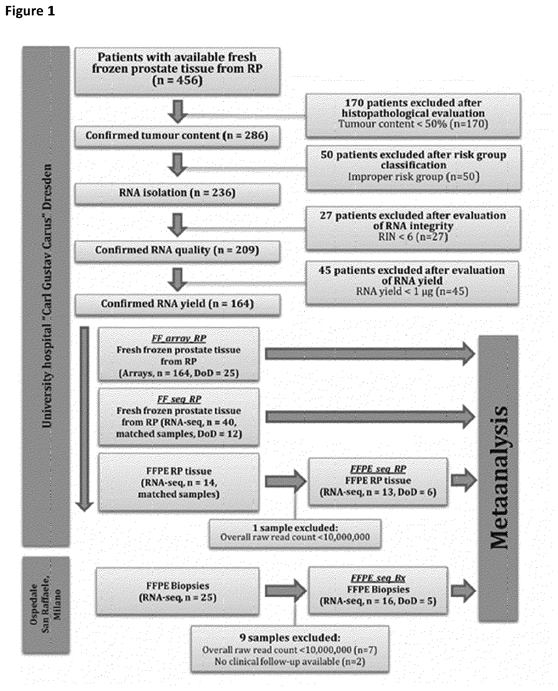

**Figure 1: Overview of tissue specimens included in the training cohort for the ProstaTrend score calculation.**
A flowchart depicting the number of patients included in the training cohort and reasons for exclusion according to REMARK (McShane et al., 2005; J Natl Cancer Inst.). For 164 patients who underwent RP fresh-frozen (FF) tumor specimens fulfilled all inclusion criteria and gene expression variation by custom gene expression microarrays (FF_array_RP) was assessed. A subset of 40 patients was selected to perform a strand-specific transcriptome-wide sequencing (FF_seq_RP). For 14 of those patients, differential gene expression was confirmed in an FFPE tissue specimen by strand-specific RNA-seq. All but one sample passed the quality filter and entered the meta-analysis (FFPE_seq_RP). Gene expression variation in FFPE biopsies was assessed for 25 patients from an independent cohort with strand-specific RNA-seq. Of those, 16 samples passed the quality filter and entered the meta-analysis (FFPE_seq_Bx). n denotes the number of patients for which tissue specimens were processed. The number of DoD (death because of disease) events (primary endpoint) included in the meta-analysis is presented for each cohort. RIN corresponds to the RNA integrity number.

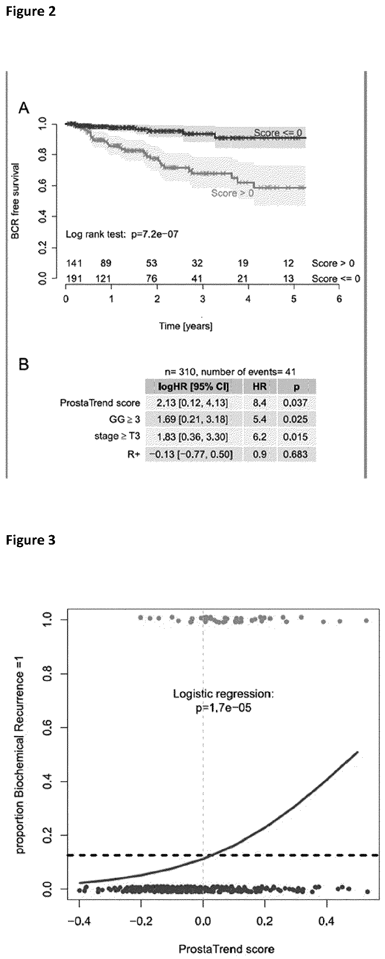

**Figure 2: Significant discrimination of different prostate cancer risk groups, characterized by percentage of BCR-free survival, when applying the method described by the present invention.**
Figure 2 shows the prognostic value of the herein disclosed gene expression signature (which is also shown in Table 2 and herein also referred to as gene expression values of reference genes) in an independent patient cohort ("TCGA PRAD", n=332 samples) with the primary endpoint of BCR. The Figure 2A depicts Kaplan-Meier analysis and log-rank test of patients with a ProstaTrend score > 0 (increased risk) compared to patients with ProstaTrend score $\leq$ 0 (reduced risk). Grey shades depict the 95%-confidence interval for Kaplan-Meier curves. The curves were truncated if the number of patients at risk dropped below ten in any arm. Figure 2B further assesses the prognostic value of the herein disclosed score by multivariable Cox proportional hazard regression using the score on a continuous scale and adjusting for Gleason grading groups (GG) $\geq$3, pathological stage $\geq$T3 and positive resection status.

**Figure 3: Statistical analysis of performance of the continuous ProstaTrend score.**
Statistical analysis was carried out evaluating the performance of the continuous ProstaTrend score, relating to the herein described invention, distinguishing patients with and without BCR confirming a significant correlation of the invention (ProstaTrend score) with BCR ($p=1.7e^{-05}$) and providing the estimation of the conditional probability of BCR at a given score. The figure depicts logistic regression analysis for the ProstaTrend score and BCR. Patients with BCR are plotted in grey scattered around the horizontal axis at 1. Patients without BCR are depicted in black at the bottom of the graph scattered around 0. The dashed line indicates the overall frequency of BCR in the "TCGA PRAD" patient cohort (13%). The logistic regression curve represents the estimated conditional probability of BCR at a given ProstaTrend score.

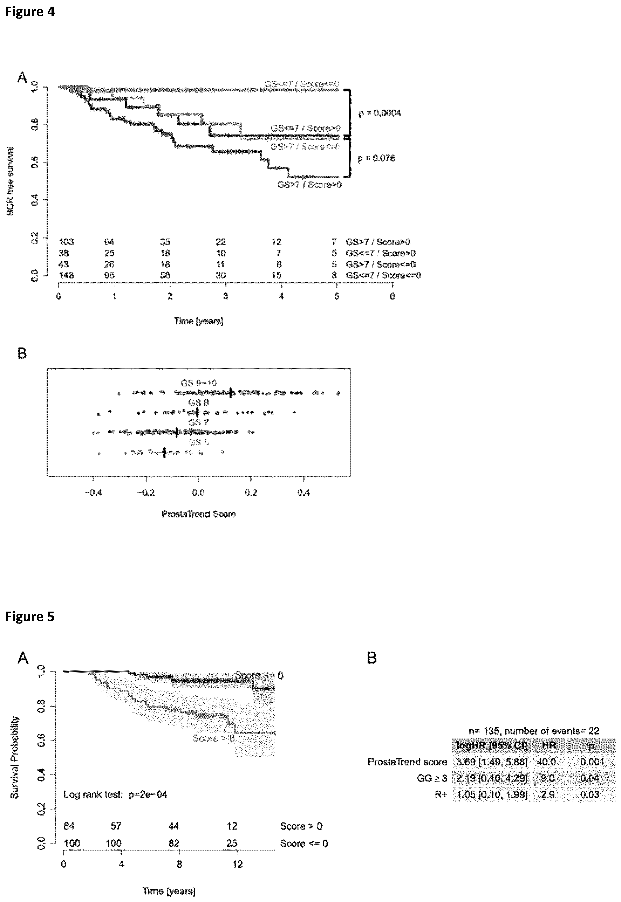

**Figure 4: Prognostic value of the gene expression signature of the ProstaTend reference gene set in comparison to standard Gleason scoring.**
The present invention (ProstaTrend score) allows for significant discrimination of patients with low/intermediate and high Gleason score (GS; n=186), characterized by percentage of BCR-free survival. Figure 4A shows the prognostic value of the herein disclosed reference gene expression signature (see also Table 2) for patients with a ProstaTrend score >0 (increased risk) compared to patients with ProstaTrend score $\leq$0 (reduced risk) separately for patients with GS >7 and GS $\leq$7. For only ~20% (38/186) of the patients in the low/intermediate GS group, the gene expression indicated an increased risk (ProstaTrend score >0). Patients at risk are displayed at the bottom of the graphic. The curves were truncated if the number of patients at risk dropped below five in any arm. Figure 4B shows individual ProstaTrend scores for patients within different Gleason subgroups. A vertical black line indicates the median ProstaTrend score for each group.

**Figure 5: Prognostic value of the ProstaTrend**

reference gene expression signature (listed in Table 2) in the training cohort (n=164 samples) with the primary endpoint DoD.

The present invention (ProstaTrend score) allows for significant discrimination of different risk groups, characterized by percentage of "death of disease" (DoD)-free survival. The figure 5A depicts Kaplan-Meier analysis and log-rank test of patients with a ProstaTrend score > 0 (increased risk) compared to patients with ProstaTrend score $\leq$ 0 (reduced risk). Grey shades depict the 95%-confidence interval for Kaplan-Meier curves. The curves were truncated if the number of patients at risk dropped below ten in any arm. Figure 5B further assesses the prognostic value of the score by multivariable Cox proportional hazard regression using the ProstaTrend score on a continuous scale and adjusting for Gleason grading groups (GG) $\geq$3 and positive resection status. Adjusting the model for pathological stage was not reasonable, because no DoD cases were observed for low risk pathological stage (pT2). Since the cohort was part of the training dataset leave-one-out cross validation (LOOCV) was applied for the ProstaTrend score calculation.

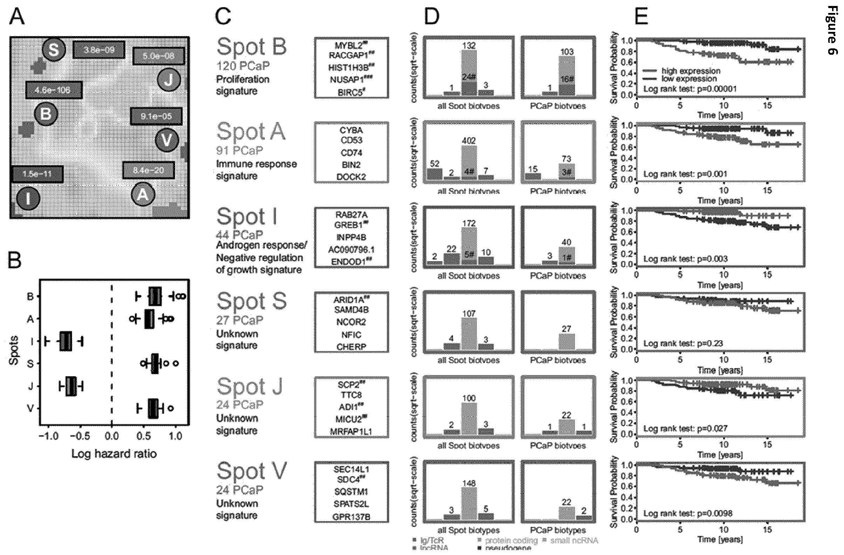

**Figure 6: Biological characterization of the ProstaTrend reference gene signature (Table 2) for DoD.**

(A) Representation of the "FF_array_RP" dataset using Self-organizing maps (R-package oposSOM v.1.16). The map illustrates the distribution of overexpressed sets of correlated genes (spots) across the SOM landscape in tumor samples. Spots that significantly enrich prognostic genes (i.e. genes listed in Table 2) after adjusting for multiple testing by Bonferroni correction (adjusted p-value < 0.05) are color-coded and annotated. The adjusted p-values for the enrichment of prognostic genes are $1.1*10^{-104}$ for spot B, $1.9*10^{-18}$ for spot A, $3.4*10^{-10}$ for spot I, $8.7*10^{-08}$ for spot S, $1.2*10^{-06}$ for spot J and $2.1*10^{-03}$ for spot V. (B) Box plot of the logHRs for the prognostic genes of significantly enriched spots. (C) Spots enriched with prognostic genes are sorted according to the number of included prognostic genes. If genes of specific biological pathways were enriched, spots were assigned to a functional signature of gene ontology (GO) or gene-sets collected from publications and independent analyses that are part of the oposSOM package. The top five genes (ranked decreasingly by correlation with mean spot expression profile) are shown for each spot. "#" indicates genes only included in Prolaris, Oncotype and/or Decipher signatures but not in the ProstaTrend gene signature (Table 2). "##" indicates genes included in the ProstaTrend signature, but not in the Prolaris, Oncotype and/or Decipher signatures, and "###" indicates genes that are occurring in the ProstaTrend signature and in the Prolaris, Oncotype and/or Decipher signatures. (D) Bar plots, depicting the distribution of gene types (biotypes) among all genes in the spots, significantly enriched with prognostic genes (left panel) and the distribution of gene types (biotypes) included in the spot and the ProstaTrend score (right panel). The y-axis is square-root-scaled to ensure readability for small groups. (E) Kaplan-Meier curves for DoD for patients with mean expression of the genes in the respective spot > 0 compared to patients with mean expression of genes in the respective spot $\leq$ 0. The prognostic value of the spots was assessed with a log-rank test. Ig/TcR: immunoglobulin/T-cell receptor; lncRNA: long non-coding RNA; small ncRNA: non-coding RNA.

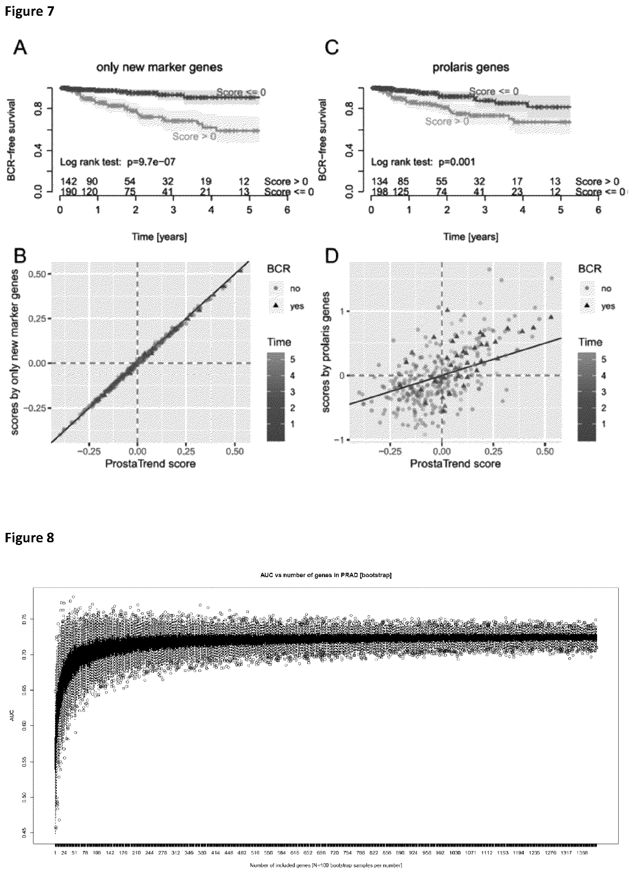

**Figure 7: Comparison of different prognostic scores in the "TCGA PRAD" cohort (n=332 samples).**

The different scores were computed exactly as the herein disclosed prognostic ProstaTrend score but for different selections of ProstaTrend reference genes (selected from Table 2) and the calculated scores were applied to the "TCGA PRAD" patient cohort to predict BCR-free survival. Kaplan-Meier curves were truncated if the number of patients at risk dropped below ten in any arm. (A) The same Kaplan-Meier curve as presented in Figure 2A but based only on genes that are not included in the Prolaris, Oncotype or Decipher marker sets. (B) The score values of each sample in the "TCGA PRAD" patient cohort compared between the complete reference gene set (Table 2) and the reduced set selected from Table 2, but without genes that are part of the Prolaris, Oncotype or Decipher marker gene sets. (C) Kaplan-Meier curve using only expression data of the genes in the Prolaris marker set for score calculation. (D) The score values of each sample in "TCGA PRAD" patient cohort compared between the reference gene set disclosed by the present invention (Table 2) and the Prolaris marker gene set.

**Figure 8: Impact of number of reference genes selected from Table 2 for the classification of patients from the "TCGA PRAD" patient cohort.**

AUC for classification of biochemical recurrence (yes/no) in the "TCGA_PRAD" validation cohort is shown. AUC is estimated for n=1 to n=1396 genes from the complete set of the herein disclosed gene set (Table 2), which is used to calculate the ProstaTrend score. For each set N=100 bootstrap samples (with replacement) were evaluated, AUC for classification of biochemical recurrence calculated and plotted. With increasing size of the gene set the AUC improves and the variation decreases. For roughly n~[100-300] the AUC is ~0.7 and the increase of AUC decreases.

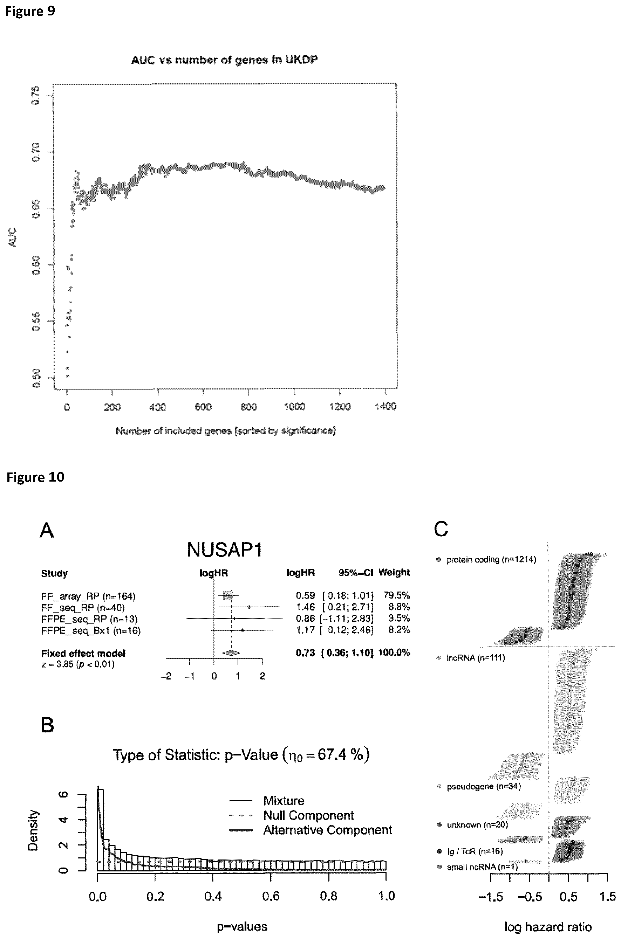

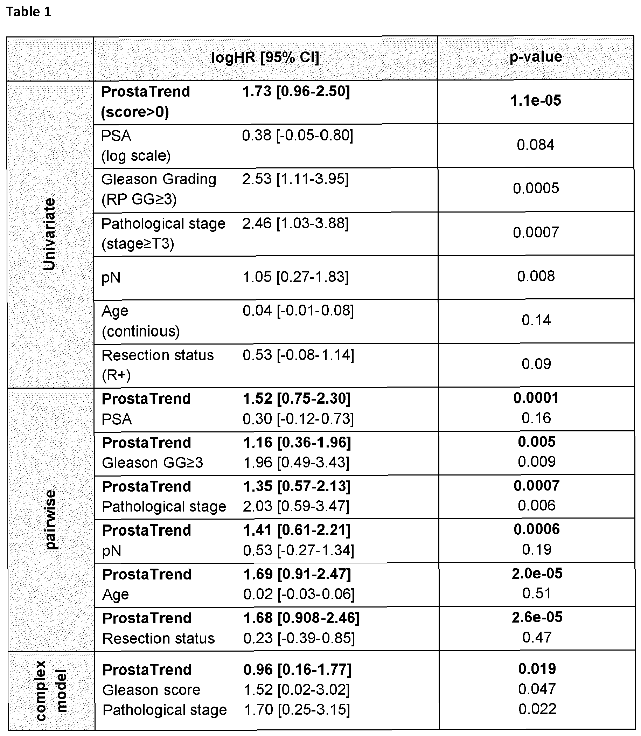

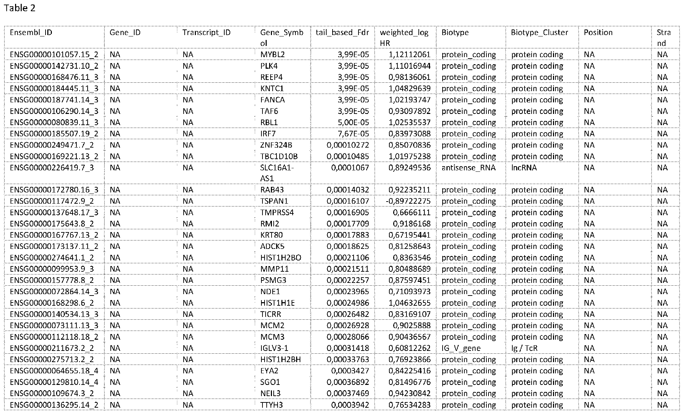

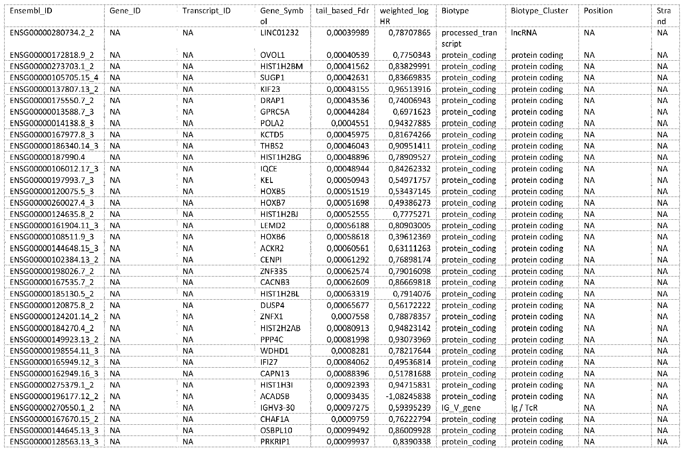

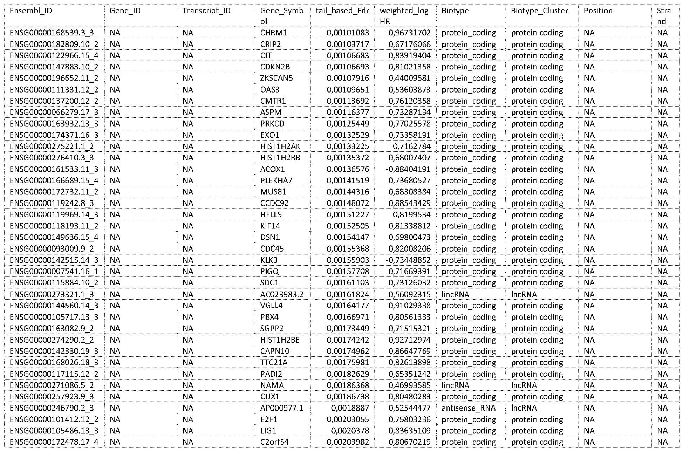

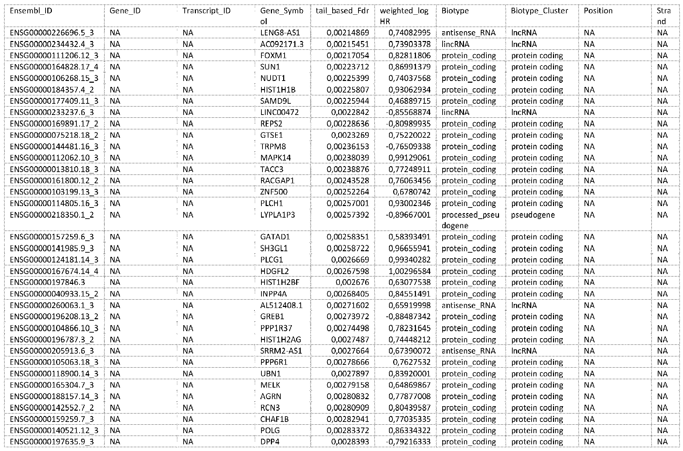

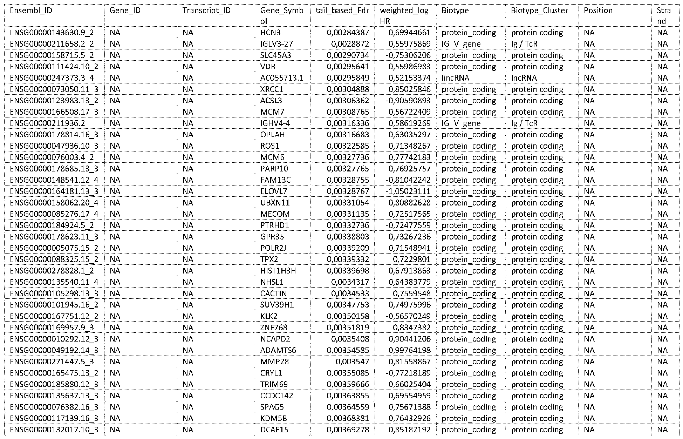

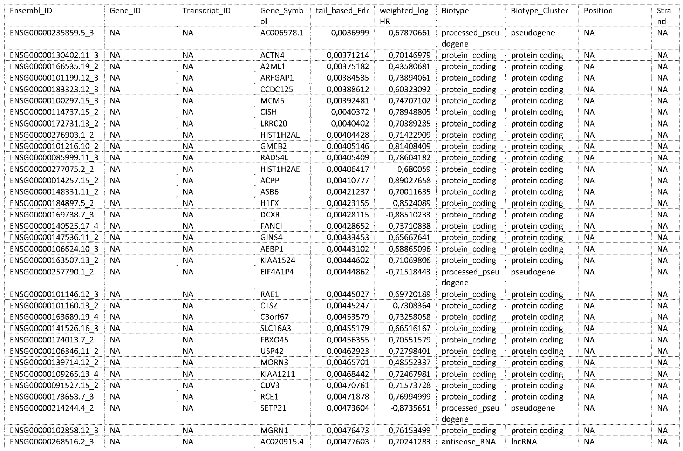

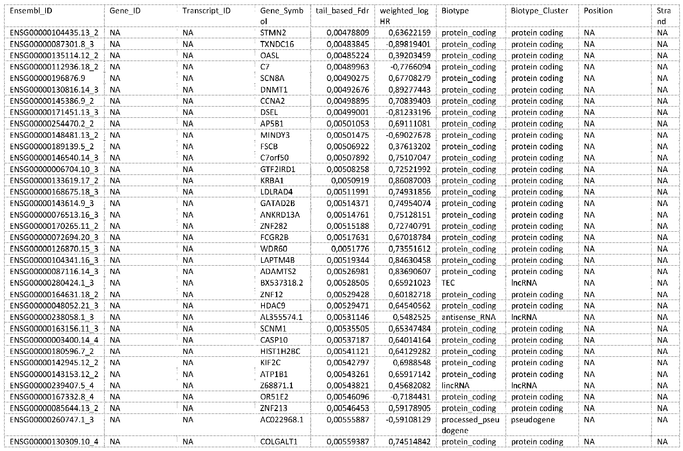

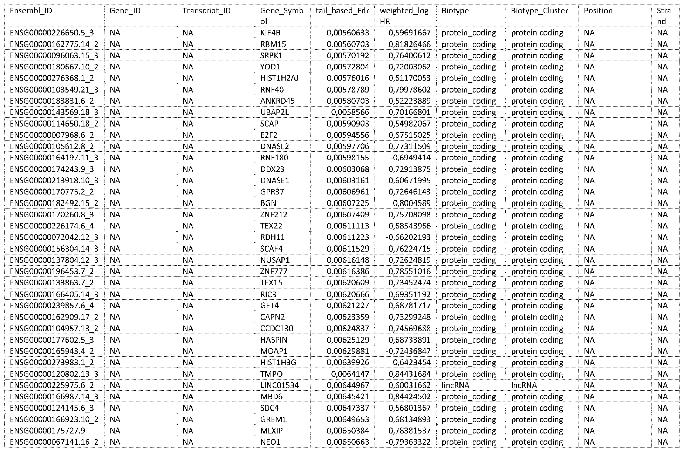

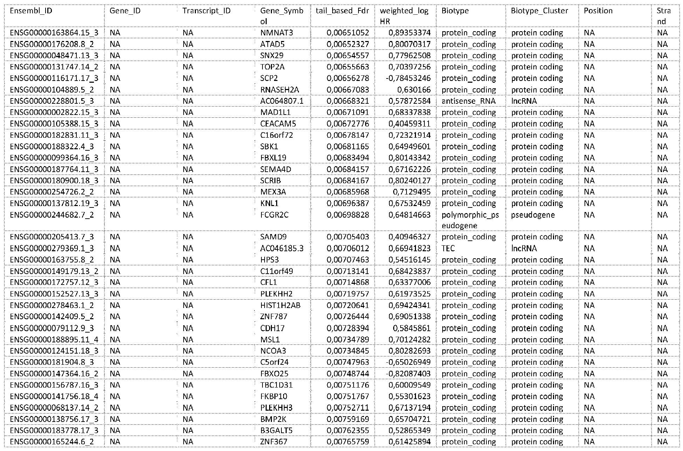

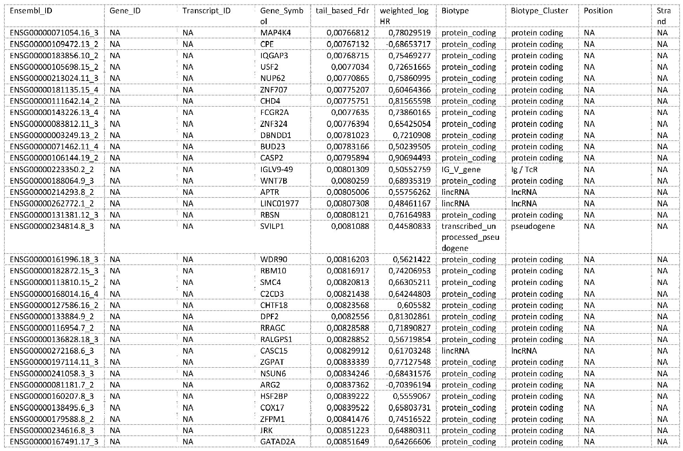

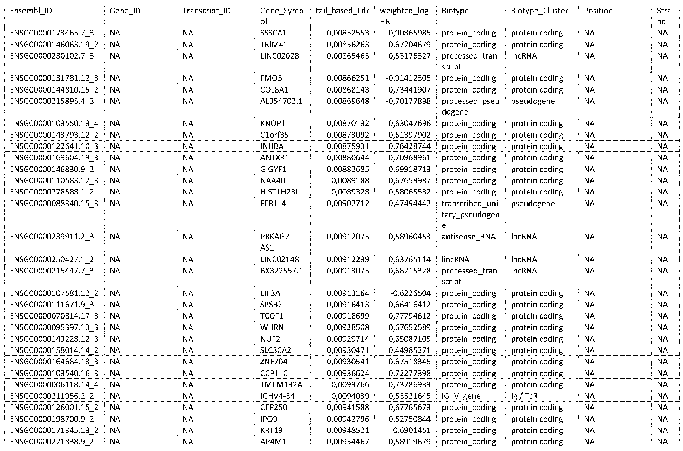

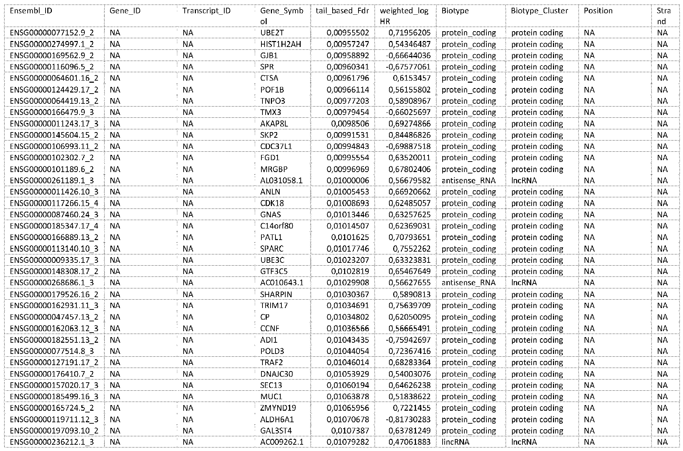

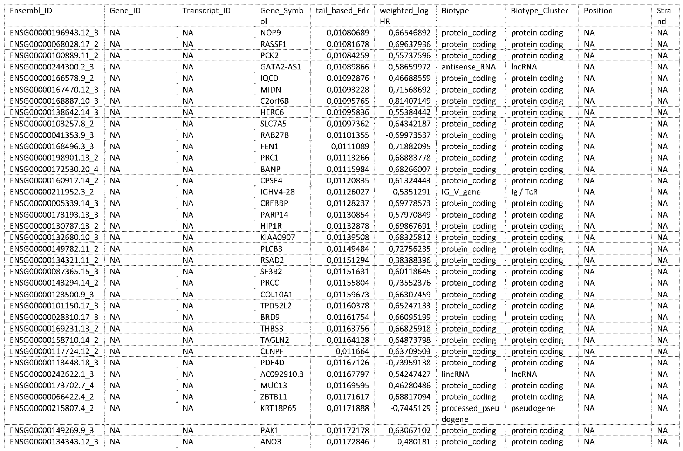

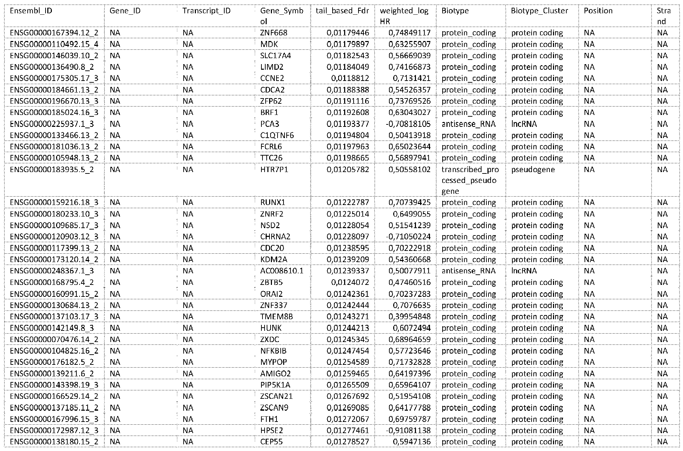

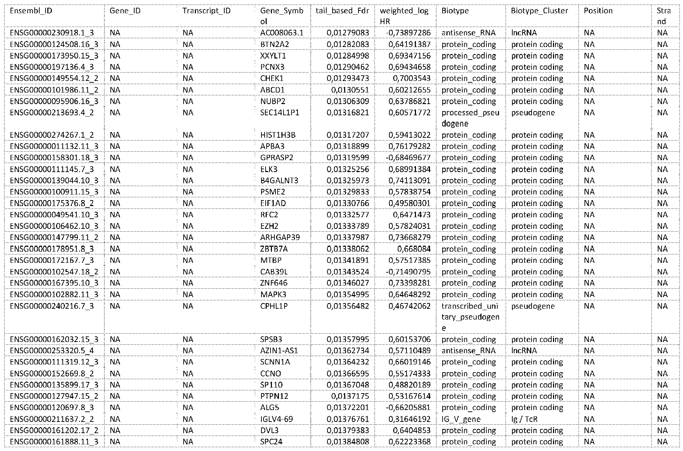

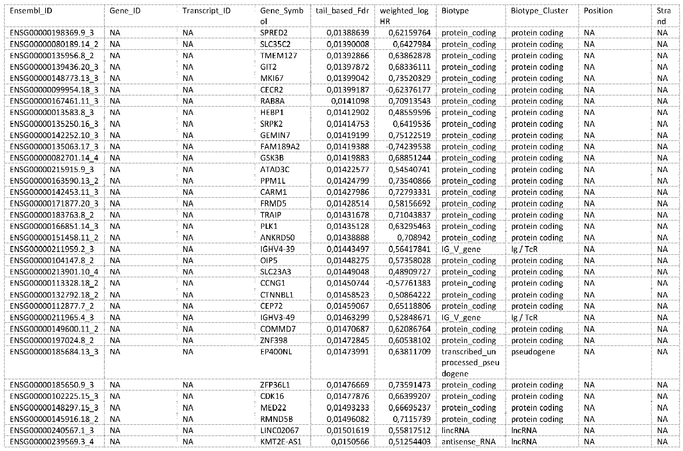

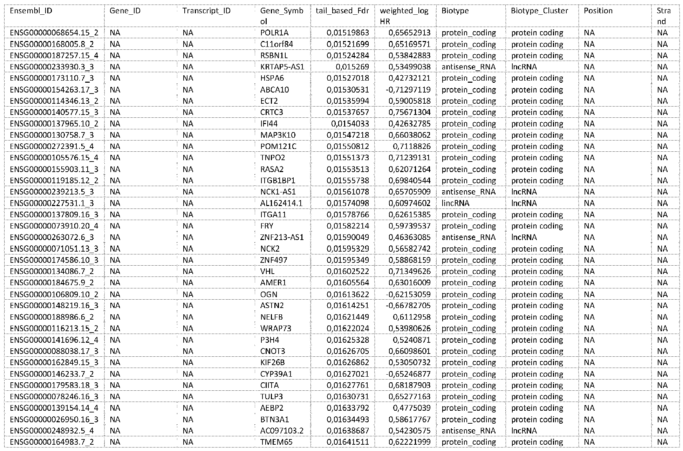

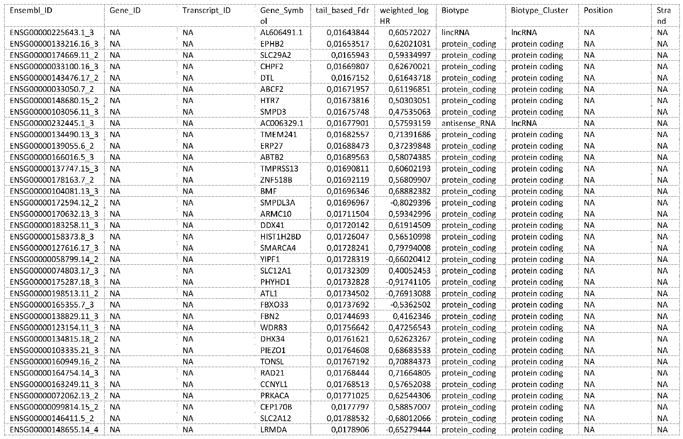

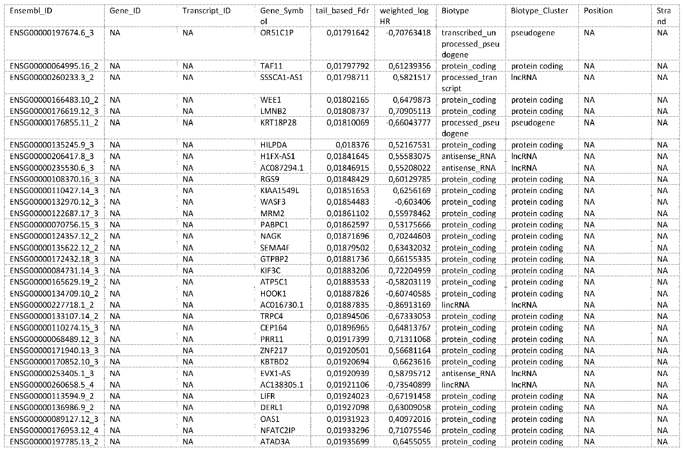

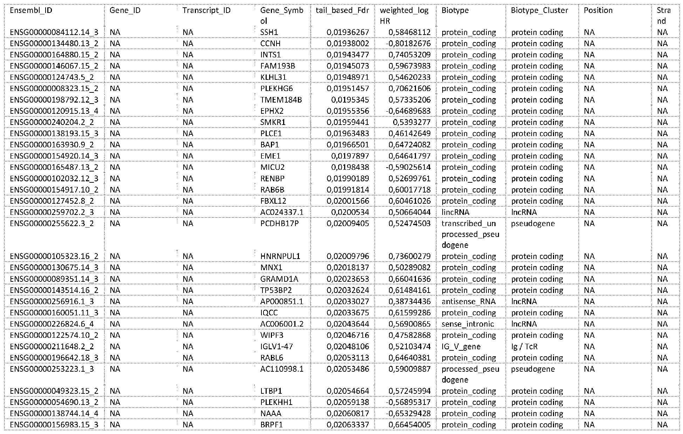

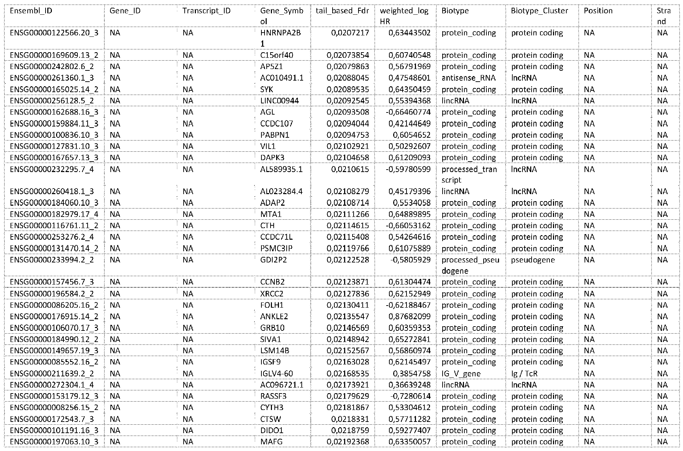

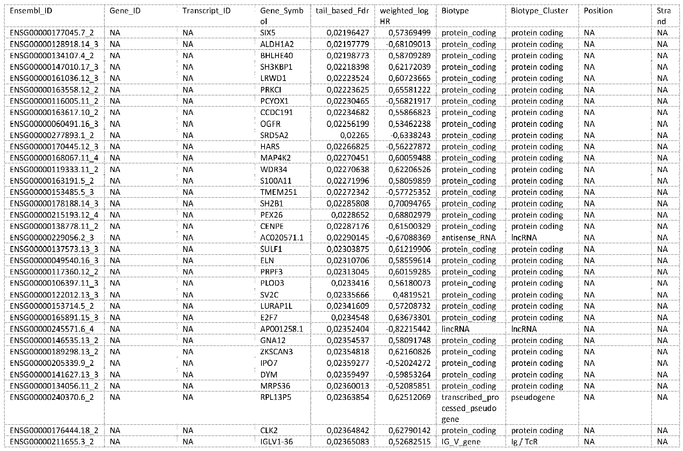

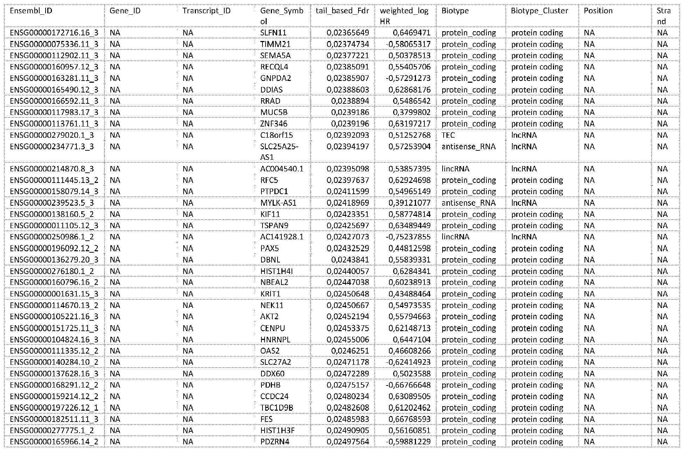

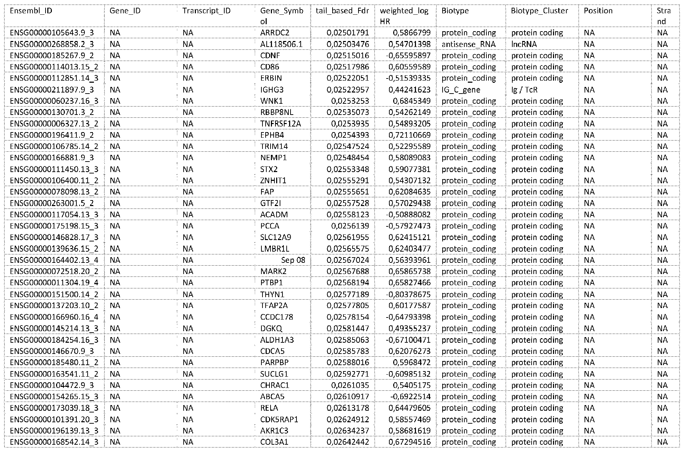

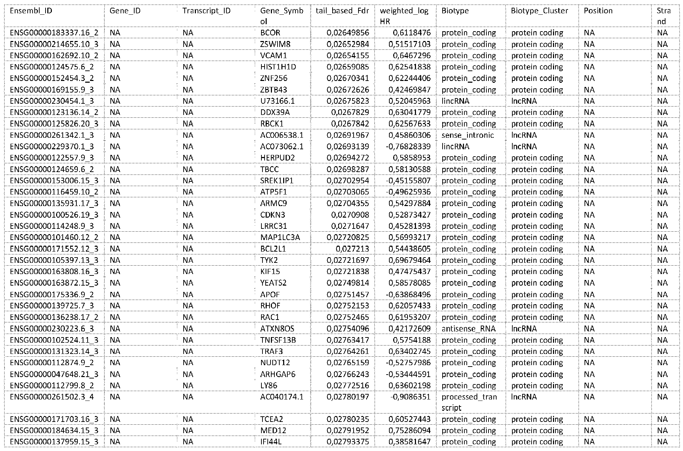

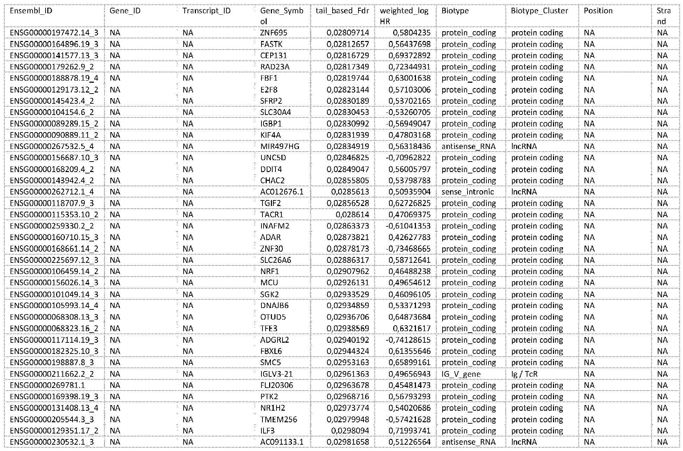

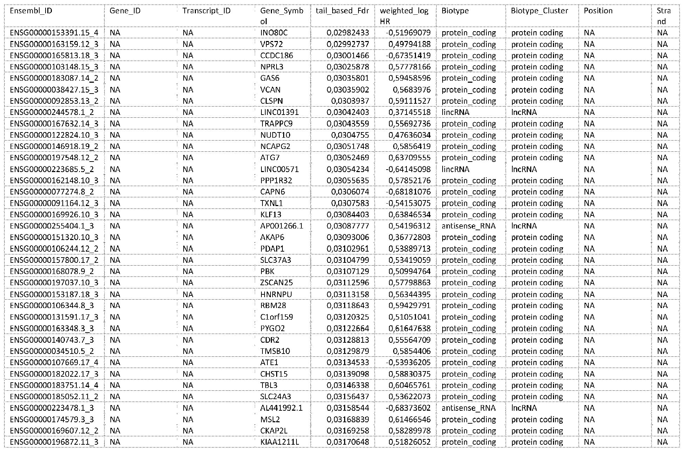

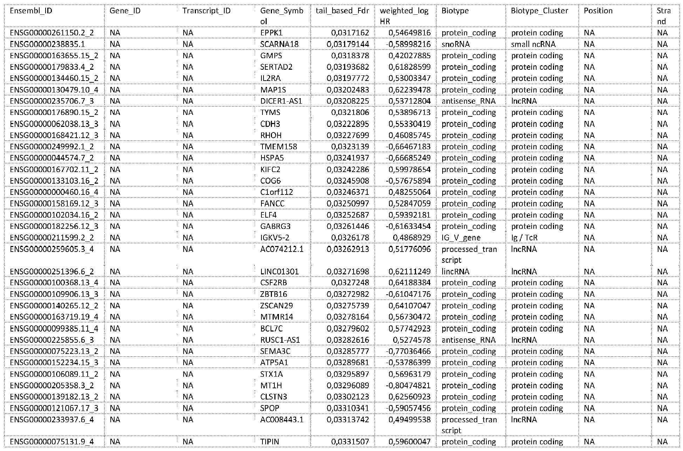

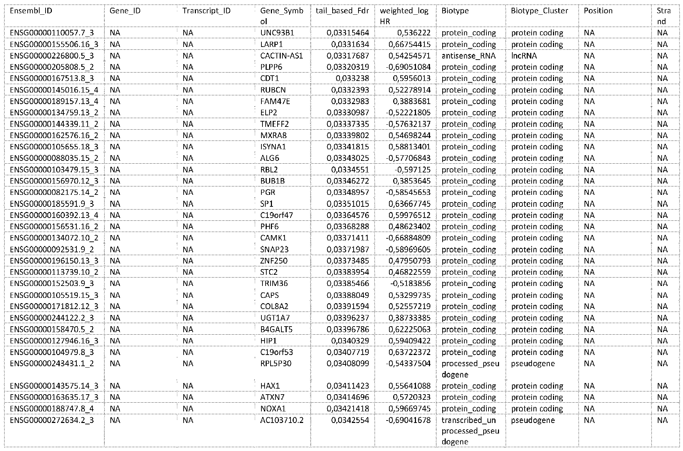

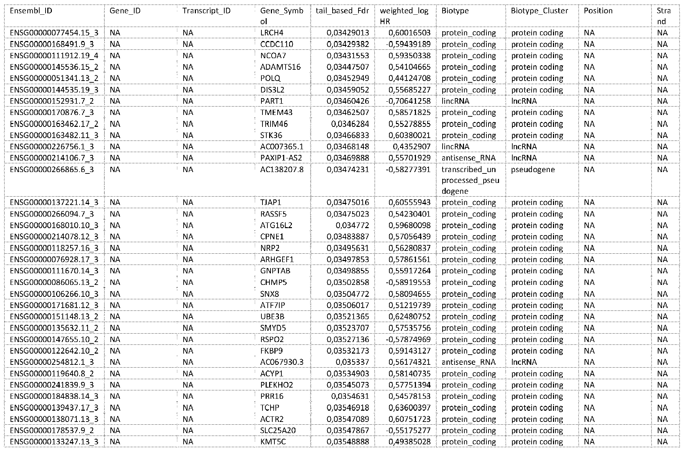

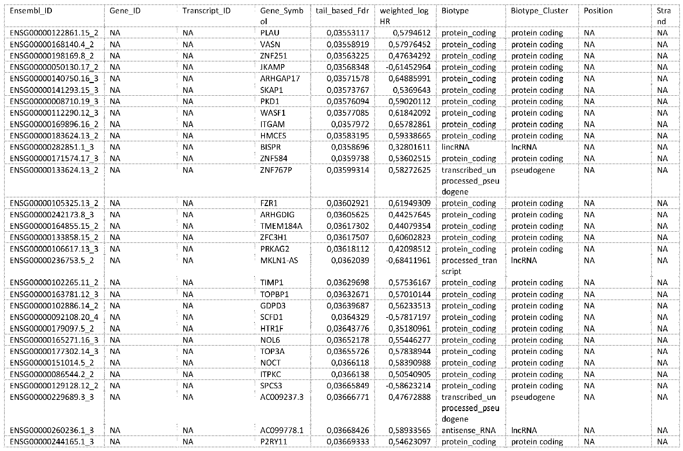

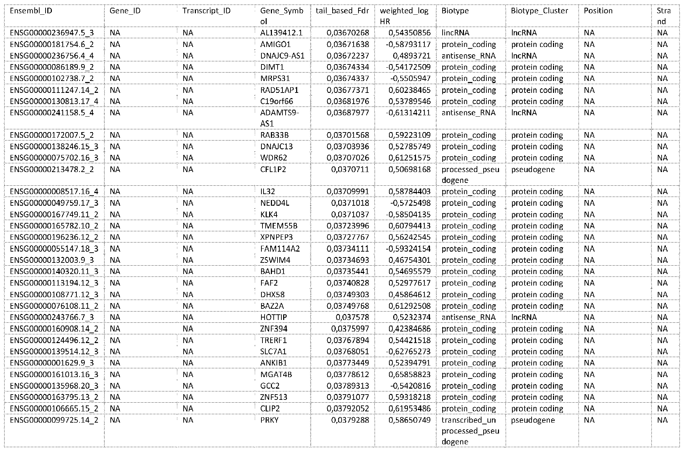

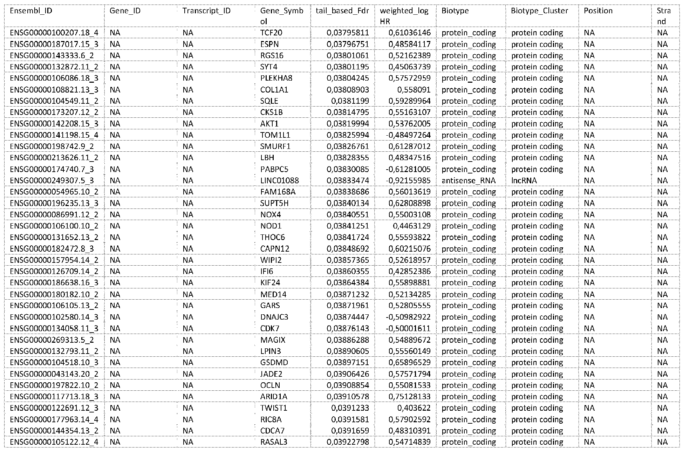

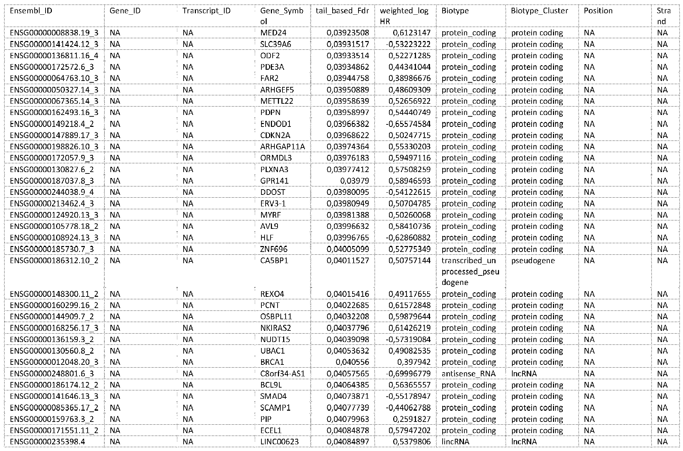

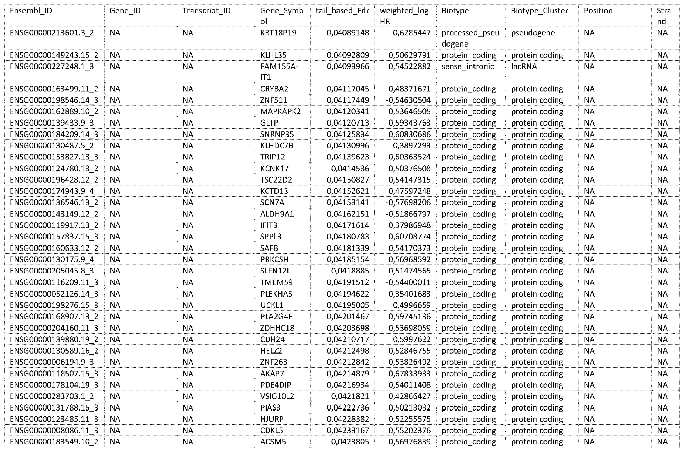

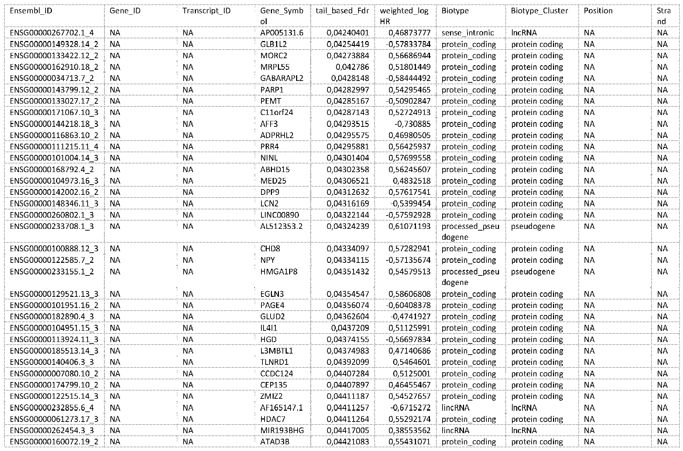

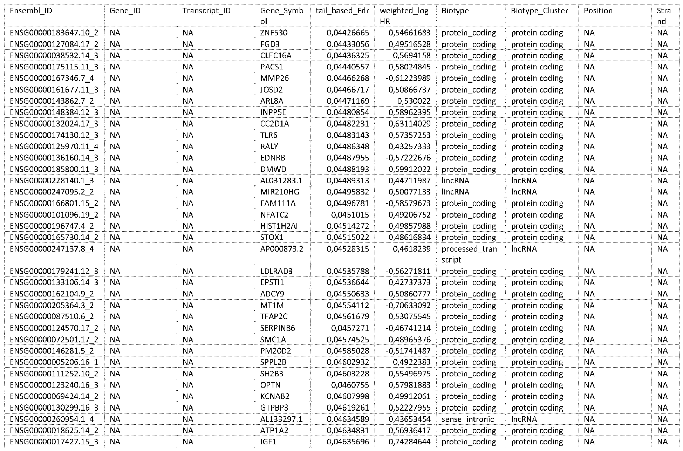

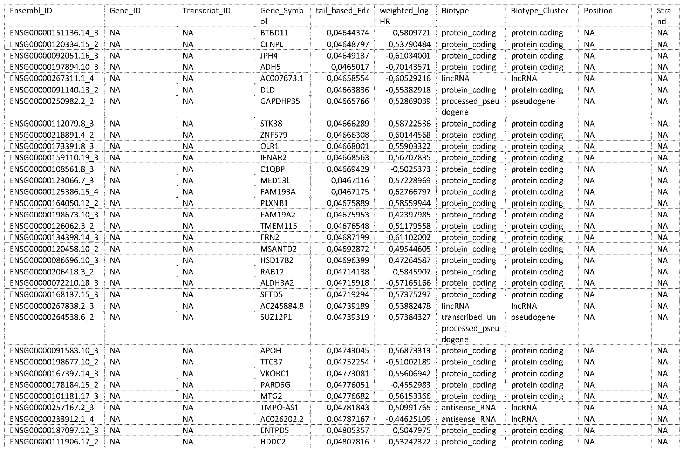

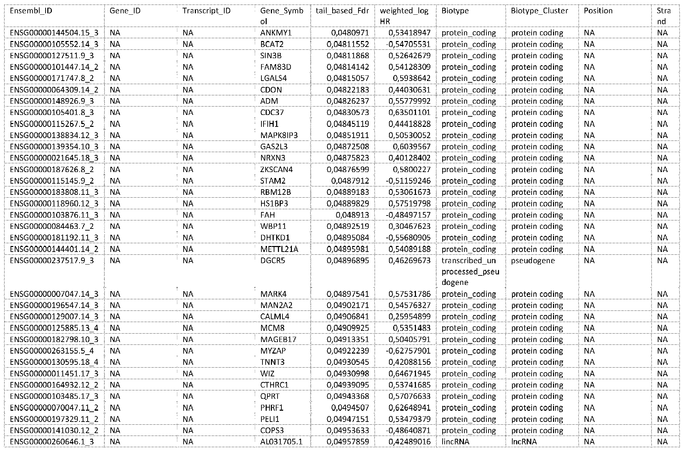

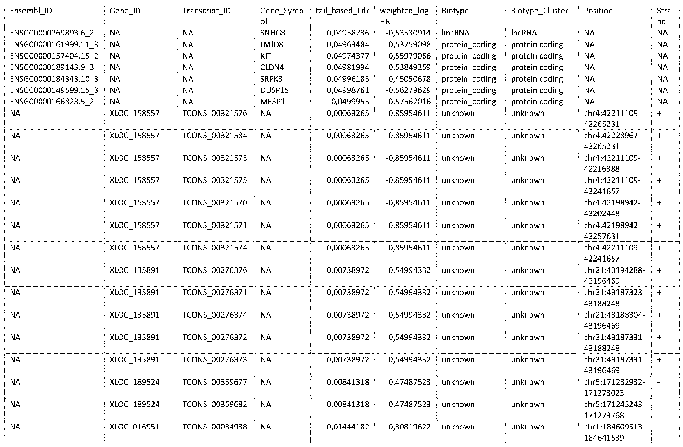

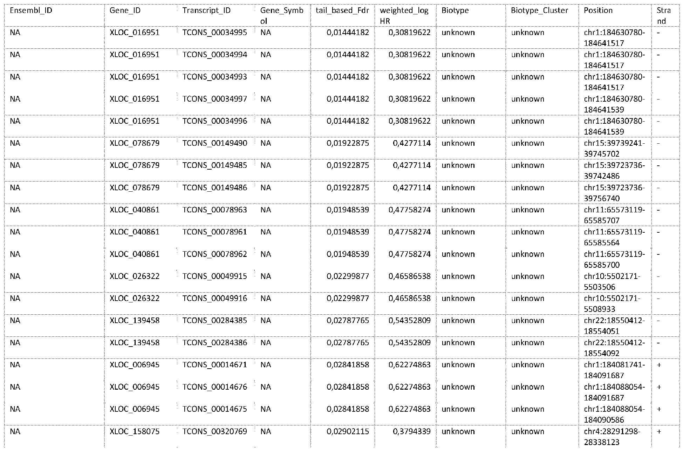

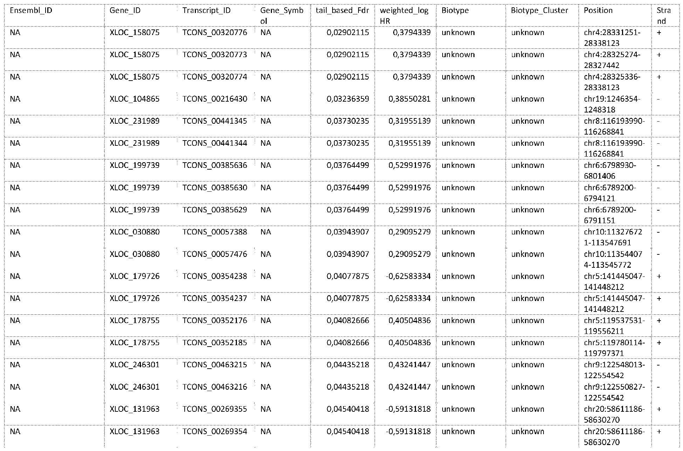

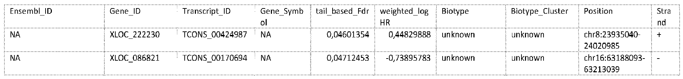

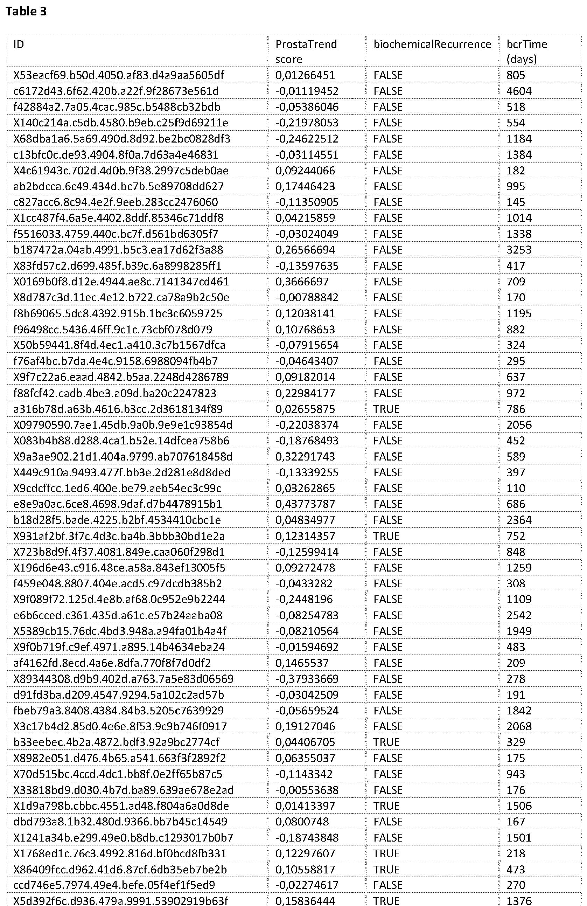

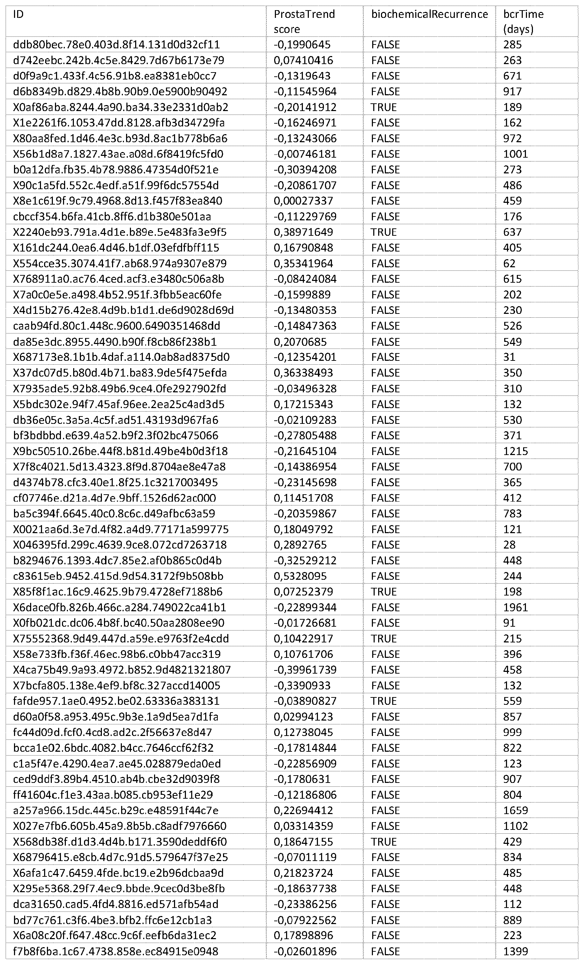

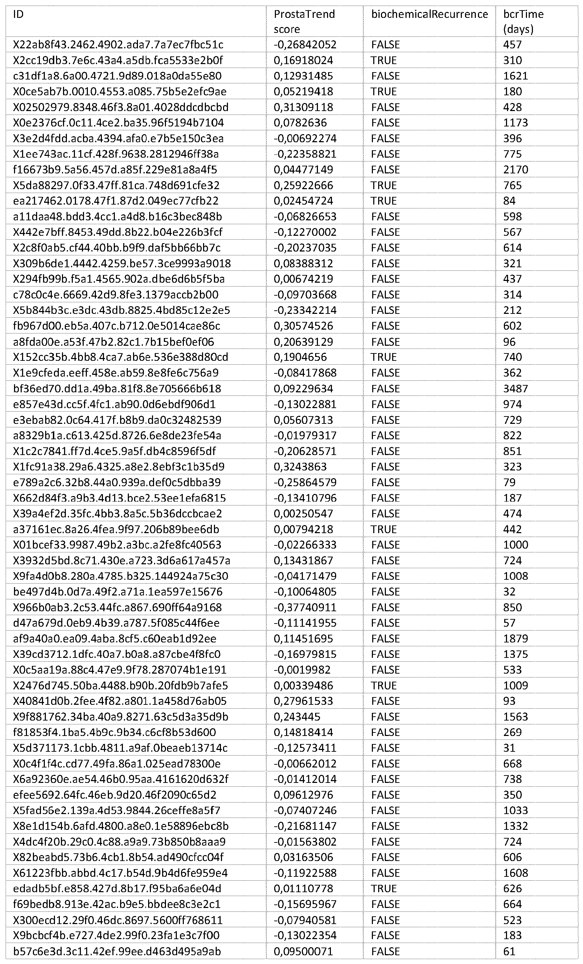

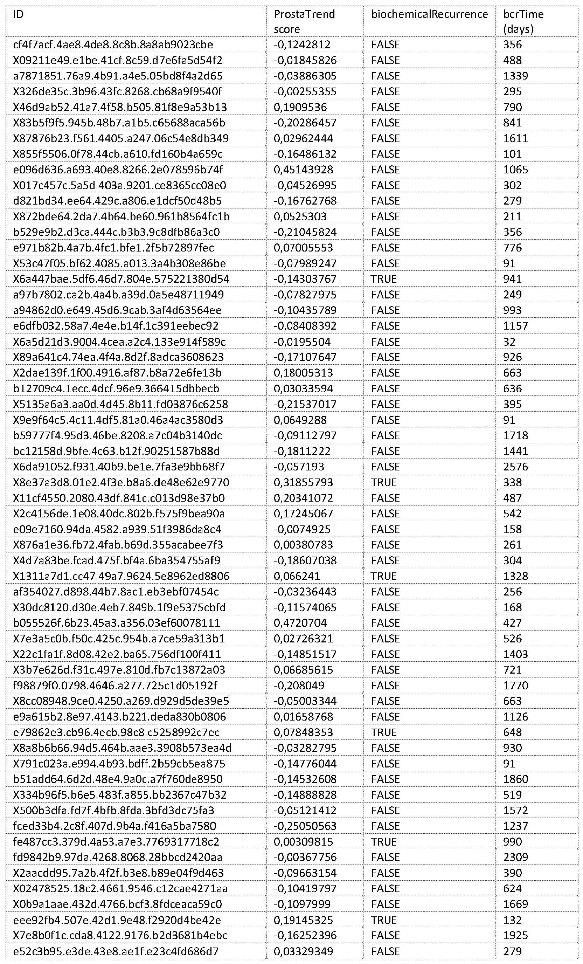

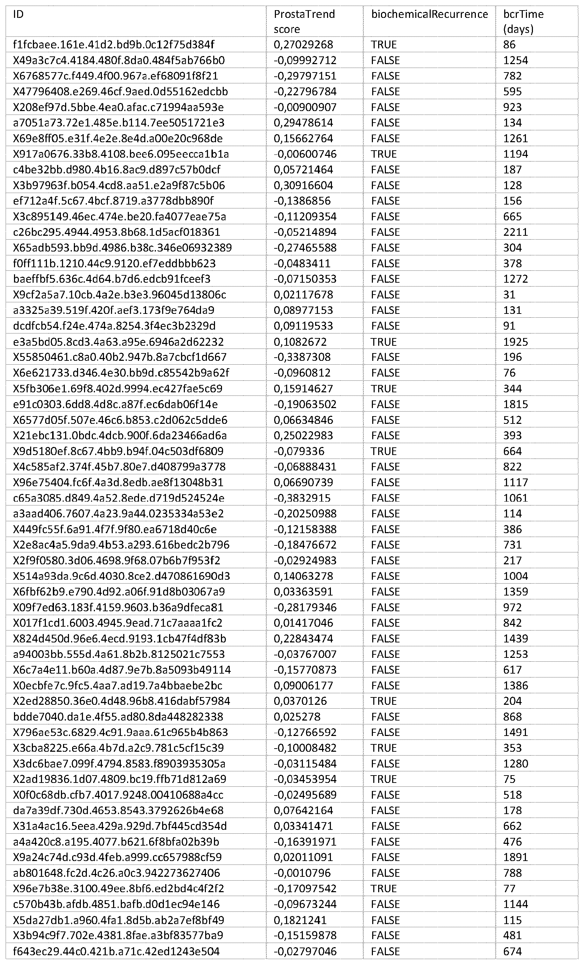

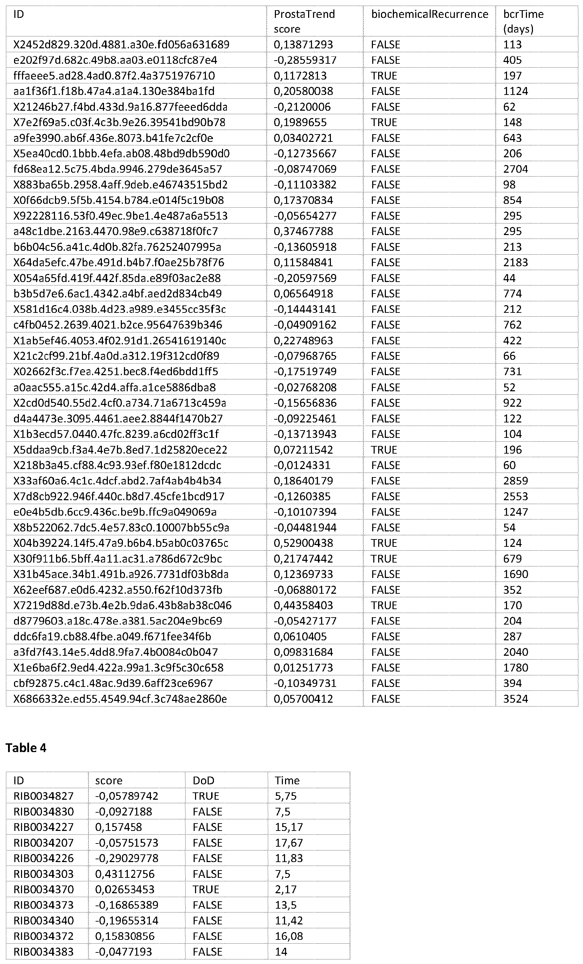

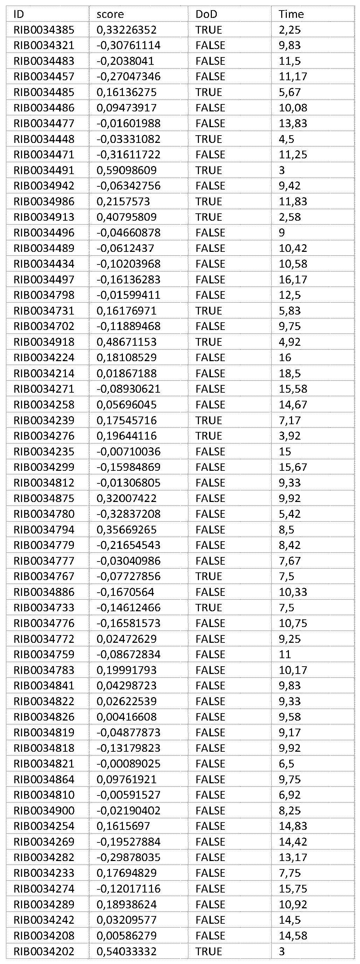

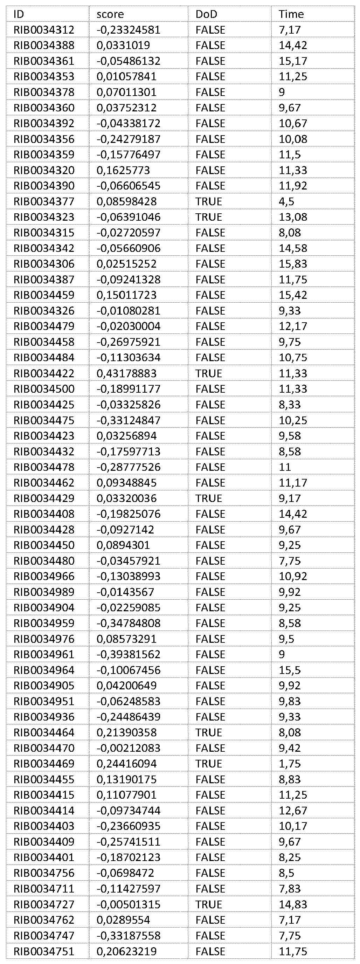

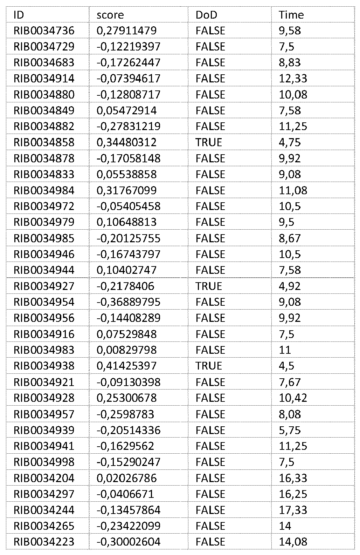

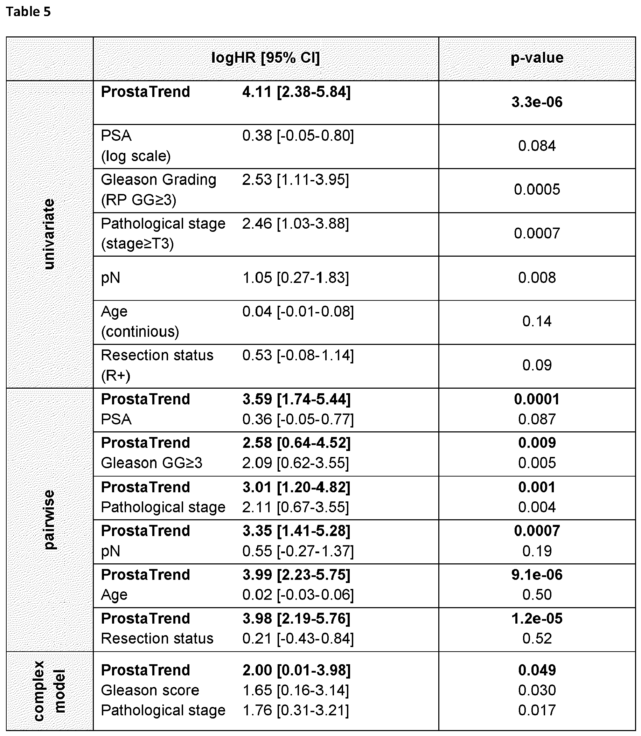

**Figure 9: Impact of number of reference genes selected from Table 2 for the classification in an independent validation cohort of biopsy specimen (UKDP cohort).**

AUC for classification of biochemical recurrence (yes/no) in this cohort is shown. AUC is estimated for n=1 to n=1396 genes from the complete set of the herein disclosed gene set (Table 2), which is used to calculate the ProstaTrend score. With increasing size of the gene set the AUC increases up to 0.68 at ˜n=400 genes (old FFPE specimen excluded).

**Figure 10: Results of the univariate fixed-effect meta-analysis of Cox proportional hazard models in FF RP tissue, FFPE RP tissue, and FFPE biopsies.**

For each reference gene (Table 2), a univariate fixed-effect meta-analysis of Cox-regression models in FF RP tissue (FF_array_RP and FF_seq_RP), FFPE RP tissue (FFPE_seq_RP) and FFPE biopsies (FFPE_seq_Bx) was conducted to identify genes with expression changes significantly associated with DoD. (A) An exemplary result of the conducted meta-analysis showing the output for the gene NUSAP1 (included in our prognostic score as well as in the Prolaris signature). (B) A histogram of p-values for the univariate fixed-effect meta-analysis of all genes included in the analysis. The histogram shows a strong enrichment for p-values near zero indicating an association with prognosis for a substantial fraction of genes. The estimated proportions of true and false null hypotheses by fitting a mixture model using the R-package fdrtool are 67.4% (null component: grey dotted line) and 32.6% (alternative component: solid black line), respectively. (C) Forest plot of the overall logHRs and corresponding 95% confidence intervals (95%-CI) for all genes included in the ProstaTrend score, i.e., all genes with tail-based false discovery rate (Fdr) <0.05 (n=1396). LogHRs are colored according to the particular gene biotype. For protein-coding genes, the y-axis is condensed to ensure readability for the smaller non-protein coding gene groups.

**Table 1: Prognostic value of dichotomized ProstaTrend score compared to clinical features in an independent cohort ("TCGA PRAD", n=332 samples) with the primary endpoint BCR.**

The prognostic value of dichotomized ProstaTrend and clinical risk factors that are available for the "TCGA PRAD" patient cohort was assessed by Cox proportional hazard regression. We analyzed dichotomized ProstaTrend score values (>0 vs ≤0), i.e. assigned value 0 to a patient if the patients' ProstaTrend score ≤0 and assigned value 1 to a patient if the patients' ProstaTrend score > 0. , We analysed PSA on the log-scale and age as a continuous variable. The GS was assessed based on RP tissue and Gleason grading groups (GG) ≥3 was compared to GG <3. For resection status we compared R+ vs R0. The upper part of the table shows univariate results, the lower part contains pairwise analysis of the dichotomized ProstaTrend score (see definition above) and each clinical feature. The bottom line shows the result of the cox regression including ProstaTrend, GG and pathological stage, i.e. all clinical features that remained significant in a pairwise model with the dichotomized ProstaTrend score.

**Table 2: List of reference genes, herein also referred to as prognostic genes, used to calculate the ProstaTrend score according to the present invention.**

To identify genes associated with DoD we applied weighted univariate Cox regression analysis on a set of 20,821 genes that passed the expression quality filter for all included training cohorts separately and used the estimated log hazard-ratios (logHR) and the respective standard errors (SEs) in a fixed-effect-model meta-analysis. For a conservative correction for multiple testing, we selected the 1,396 genes with a tail-based Fdr <0.05 as a marker set including 1,376 known genes and 20 novel genes. The logHR values in the present table are used during the calculation of the ProstaTrend score as a weighting factor for the respective gene. After this step these weighted genes are combined by the median. Genomic position of genes is w.r.t. human genome assembly hg19.

**Table 3: Samples of the validation patient cohort "TCGA PRAD".**

Table providing for each sample of the validation patient cohort ("TCGA PRAD") the ID, the calculated ProstaTrend score, occurrence of biochemical recurrence within follow-up, and the follow-up time in days.

**Table 4: Samples of the training patient cohort "FF_array_RP".**

Table providing for each sample of a training patient cohort ("FF_array_RP") the ID, the calculated ProstaTrend score (with leave-one out cross validation), occurrence of DoD within follow-up, and follow-up time in years.

**Table 5: Prognostic value of continuous ProstaTrend score compared to clinical features in an independent cohort ("TCGA PRAD", n=332 samples) with the primary endpoint BCR.**

We assessed the prognostic value of the continuous ProstaTrend score and clinical risk factors that are available for the "TCGA PRAD" cohort by Cox proportional hazard regression. We analyzed continuous ProstaTrend score, PSA on the log-scale and age as a continuous variable. The GS was assessed based on RP tissue and Gleason Grading Groups (GG) ≥3 was compared to GG <3. For resection status we compared R+ vs R0. The upper part of the table shows univariate results, the lower part contains pairwise analysis of ProstaTrend and each clinical feature. The bottom line shows the result of the cox regression including ProstaTrend, GG and pathological stage, i.e. all clinical variables that remained significant in the pairwise analysis with ProstaTrend.

## DETAILED DESCRIPTION OF THE INVENTION

The present invention relates to a method (ProstaTrend) for predicting the outcome and/or classifying the disease of prostate carcinoma patients, wherein the method comprises calculating and applying a prognostic multi-gene expression score (ProstaTrend score) based on the expression of a set of genes that are significantly associated with prostate cancer prognosis (Table 2) in a patient sample. The herein disclosed method allows for molecular risk stratification and improved prediction of long-term patient prognosis in the treatment of prostate cancer. The invention further relates to a method, comprising the prediction of death of disease (DoD) and biochemical recurrence (BCR), as well as the prediction of a long-term prognosis and the disease outcome of prostate cancer patients. The method provided by the invention (ProstaTrend) is independent from clinical and histological parameters after diagnosis and facilitates the identification of high-risk patients among patients with low or intermediate histological-risk profiles prior and after surgery.

One of the surprising technical effects of the present invention is the gain of patient outcome prediction accuracy through the application of the ProstaTrend score method, which is shown herein by a concordance analysis (C-Index) for a clinical model based on grading groups, pathological stage and resection status with and without inclusion of the herein disclosed ProstaTrend score method for samples of the patient "TCGA PRAD" cohort (see also Example 3). With the inclusion of the ProstaTrend score method the concordance index increased by 4.9% [95%-CI: -0.3-8.6%] from 71.4% to 76.3%. In greater detail, the gain of prediction accuracy by using the herein disclosed ProstaTrend score, is shown by the comparison of the concordance index between two multivariable cox models, one featuring clinical parameters grading groups, pathological staging, and resection status only, while the second model also includes the herein disclosed ProstaTrend score method. 1000 bootstrap samples were generated using the R-Package boot v1.3-2418 while keeping the number of events constant to estimate the 95% confidence for the difference of the concordance index (C-index) between both models.

In one embodiment the present invention relates to a method for predicting the outcome or classifying the disease of a prostate carcinoma patient with the method comprising the steps of obtaining a biological sample, wherein the sample comprises a plurality of nucleic acids, a plurality of cells, a biopsy and/or human or animal tissue and obtaining a plurality of nucleic acids from the sample, followed by subsequent analysis of the plurality of nucleic acids, thereby determining the sequences of the nucleic acids from the sample and/or the abundancies of target nucleic acid sequences from the sample, followed by comparing the sequences of the nucleic acids and/or the abundancies of the target nucleic acid sequences derived from the sample to a set of known reference nucleic acid sequences and finally calculating a score (ProstaTrend score) value depending on the comparison of the target nucleic acid sequences and/or of the target nucleic acid sequence abundancies in relation to the set of known reference gene nucleic acid sequences, wherein a difference or similarity in at least one of the nucleic acid sequences and/or nucleic acid sequence abundancies between the known reference gene nucleic acid sequences and the target nucleic acid sequences from said sample is used for calculating a score value that is predictive of a prognosis and/or classification of the patient from whom the sample was derived.

In a further embodiment the present invention relates to a method for predicting the outcome or classifying the disease of a prostate carcinoma patient, the method comprising the steps of: a) obtaining a sample from said patient, wherein the sample comprises a plurality of nucleic acids, b) analyzing the plurality of nucleic acids, thereby determining the abundancy of at least one or more different target nucleic acid sequences, c) normalizing of the target nucleic acid sequence abundancies against a reference dataset and standardization, d) weighting the abundancy of the at least one or more different target nucleic acid using a corresponding reference value and e) calculating a score value, and wherein, if the score value is above a threshold value of 0, the patient is classified as having a high risk of death of disease and/or biochemical recurrence and/or if the score value is below a threshold value of 0, the patient is classified as having a low risk of death of disease and/or biochemical recurrence.

In another embodiment the invention relates to a method for predicting the outcome or classifying the disease of a prostate carcinoma patient with the method comprising the steps of: a) obtaining a biological sample from said patient, wherein the sample comprises a plurality of nucleic acids, a plurality of cells, a biopsy and/or human or animal tissue and obtaining a plurality of nucleic acids from the sample, b) analyzing the plurality of nucleic acids, thereby determining the sequences of the nucleic acids from the sample and/or the abundancy of at least one or more different target nucleic acid sequences from the sample, c) comparing the sequences of the nucleic acids and/or the abundancy of at least one or more different target nucleic acid sequences derived from the sample to a reference abundancy, thereby calculating a score value, wherein, if the score value is above a threshold value of 0, the patient is classified as having a high risk of death of disease and/or biochemical recurrence and/or if the score value is below a threshold value of 0, the patient is classified as having a low risk of death of disease and/or biochemical recurrence.

Herein, the patient from whom said sample was derived may be classified as having a high risk of death of disease and/or biochemical recurrence, if the calculated score value is above a threshold value of 0 (score value >0) and if the score value is below a threshold value of 0 (score value <0), the sample is classified as having a low risk of death of disease and/or biochemical recurrence.

In one embodiment of the invention, each target nucleic acid sequence derived from the analysis of the plurality of nucleic acids from said patient sample is identical to the nucleic acid sequence of a corresponding specific transcript and /or isoform of a reference gene selected from the group of genes associated with cancer progression or cancer malignancy. In another embodiment, said group of reference genes are associated with the progression or malignancy of prostate cancer. In a preferred embodiment, each target nucleic acid sequence derived from the analysis of the plurality of nucleic acids from said patient sample is identical to the nucleic acid sequence of a corresponding specific transcript and /or isoform of a reference gene selected from the group of genes listed in Table 2.

In other words, herein, said target nucleic acids or target nucleic acid sequences may be those nucleic acids or nucleic acid sequences derived from or present in said sample, that have an identical nucleic acid sequence to at least one or more reference genes, or at least one or more isoforms or transcripts thereof, selected from the group of genes listed in Table 2.

In one embodiment, said reference abundancy refers to the reference abundancy of the reference gene or reference gene nucleic acid sequence, wherein said abundancy of the target nucleic acids is weighted_by the logHR value of the corresponding reference gene listed in Table 2, wherein the nucleic acid sequence of said reference gene, or of a transcript or isoform thereof, is identical to the nucleic acid sequence of one or more of said target nucleic acid sequences derived from or present in said sample.

In one embodiment of the present invention a reference dataset may be, without being limited to, a dataset comprising the gene expression values and/or logHR values and/or further characterizing values of all or a group of genes comprising all or a fraction of said reference genes listed in Table 2, the dataset disclosed in Table 2 or a fraction of said dataset, a dataset comprising the gene expression values of genes associated with cancer or prostate cancer or another dataset comprising gene expression values.

In one embodiment of the invention, said corresponding reference value for weighting abundancies is a logHR value selected from the group of logHR values listed in Table 2 corresponding to one or more reference genes listed in Table 2, wherein the nucleic acid sequence of said one or more reference gene, or of a transcript or isoform thereof, is identical to the nucleic acid sequence of one or more of said target nucleic acid sequences from said sample.

In another embodiment, said logHR values refer to one or more logHR values from the group of logHR values listed in Table 2, wherein said one or more logHR values in Table 2 are used during the calculation of the ProstaTrend score as a weighting factor for the respective reference gene listed in Table 2 and/or the expression value or abundancy of said respective reference gene in said sample, wherein subsequently during the calculation these weighted reference genes or weighted reference gene abundancies or expression values are combined by the median, and wherein the nucleic acid sequence of said reference gene, or of a transcript or isoform thereof, is identical to the nucleic acid sequence of one or more of said target nucleic acid sequences derived from or present in said sample.

Herein, said genes disclosed in Table 2, are also referred to as reference genes, marker genes or prognostic genes.

In one embodiment of the invention, each logHR value from said logHR values listed in Table 2 corresponds to one or more reference genes from the group of reference genes listed in Table 2, wherein the nucleic acid sequence of said reference gene, or of a transcripts or isoforms thereof, is identical to the nucleic acid sequence of one or more of said target nucleic acid sequences from said sample, if said reference gene is expressed in said sample. In one embodiment of the invention said logHRs are weighting factors from the training of the prognosticator, that are fixed for every new sample, wherein the comparison to a reference comes due to add-on normalization (or combined normalization) with said established ProstaTrend reference gene set.

In another embodiment said target abundancy is weighted_by the logHR value of a corresponding reference gene listed in Table 2, wherein the nucleic acid sequence of said reference gene, or of a transcripts or isoforms thereof, is identical to the nucleic acid sequence of one or more of said target nucleic acid sequences from said sample.

Herein, each gene from the group of reference genes listed in Table 2, or one or more isoforms or transcripts thereof, that have a nucleic acid sequence identical to one or more target nucleic acid sequences from said sample, may herein be referred to, without being limited to, as corresponding reference gene to said target nucleic acid sequence. In other words, if a reference gene, listed in Table 2, is expressed in said patient sample, a target nucleic acid corresponding to said reference gene may be detected during the nucleic acid analysis of said sample and it's abundancy, herein also referred to as gene expression, may be determined. Therefore, the expression value and/or abundancy of each gene, listed in Table 2 and/or each transcript or isoform thereof, in said patient sample, may be determined, without being limited to, by analyzing the nucleic acid sequences and their abundancies in said sample. Said gene expression values of the selected reference genes in said sample may subsequently be used to, without being limited to, calculate a ProstaTrend score for the patient said sample was derived from, according to the method described herein.

Herein, a biological sample may be derived from, without being limited to, a human patient, a cell culture, an "organ-on-a-chip" model, a microphysiological system or an animal. Herein, a biological sample may be an organ, a tissue, a biopsy, a liquid biopsy, a blood sample, a sample from a Patient Derived Xenograft, a sample from an "organ-on-a-chip" model, a sample from a microphysiological system, a cell line or cell culture of a patient-derived sample of a human patient, an animal or an animal model, of a cell line or of an organoid. In a more preferred embodiment of the invention the sample is an organ, a tissue, a biopsy, a liquid biopsy, a blood sample, a sample from an "organ-on-a-chip" model or a patient-derived cell culture of a human patient. In the most preferred embodiment, the sample is a tissue, a biopsy or a liquid biopsy sample of a human patient. In one embodiment the biopsy sample is a fresh sample, a frozen sample or a FFPE preserved sample.

Examples of methods for nucleic acid sequence analysis are known to those of ordinary skill in the art and may herein comprise, without being limited to, optional lysis of the tissue and/or the cells and optional subsequent purification of respective nucleic acids followed by optional fragmentation, enzymatic digestion or ligation of respective nucleic acids to anchors, solid phases or barcodes, reverse transcription, microarray analysis, Next Generation Sequencing analysis and any other Sequencing method such as, but not limited to, nanopore sequencing, third-generation sequencing, Sanger sequencing analysis, NanoString Hyb & Seq NGS analysis, nCounter-analysis, digital or droplet PCR analysis, quantitative Real-time PCR analysis and/or PCR amplification of the nucleic acids derived from respective sample and/or a sequential or parallel combination of two or more of the previously listed steps and techniques.

Herein, Next Generation Sequencing relates to, without being limited to, RNA sequencing, DNA sequencing, Whole Genome Sequencing, Whole Exome Sequencing, Single Cell Sequencing, Targeted Sequencing, NanoString Hyb & Seq NGS analysis, nCounter-analysis, sequencing methods allowing two-dimensional resolution of transcriptome or epigenome patterns in tissues such as, but not limited to, spatial sequencing or sequencing methods allowing the characterization of chromatin status such as, but not limited to, ChIP-Seq or ATAC-Seq.

In one embodiment of the invention the term patient can be equivalent, but is not limited, to a human patient, a Patient Derived Xenograft, an "organ-on-a-chip" model, a microphysiological system, an animal, an animal model, a cell line or a cell culture of a patient-derived sample of a human patient, of an animal or of an animal model, of an "organ-on-a-chip" model, of a microphysiological system, of a cell line or of an organoid. In a preferred embodiment the patient is a human patient, a Patient Derived Xenograft or an animal. In an even more preferred embodiment of the invention the patient is a human patient.

As used herein, ProstaTrend refers to a method that can be used in, without being limited to, the treatment of prostate cancer for molecular risk stratification and improved prediction of long-term prognosis for prostate cancer patients with the method comprising calculating and applying a prognostic multi-gene expression score, which is also referred to herein as ProstaTrend score, based on the expression values of a set of genes that are significantly associated with prostate cancer prognosis (Table 2), herein also referred to as reference genes. The ProstaTrend method disclosed herein can also be used in the prediction of death of disease (DoD) and biochemical recurrence (BCR), or a probability of one or both, or of a long-term prognosis and/or in the prediction of outcome for prostate cancer patients.

In one embodiment of the invention the score (ProstaTrend score) obtained by the method described herein can be used in the treatment of prostate cancer for, without being limited to, calculating a prognosis and/or a classification and/or stratification of prostate carcinoma patients from whom respective biological sample was derived. In another embodiment of the invention the score obtained by the method described herein can be used for the classification and/or grading of respective analyzed sample.

In another embodiment of the invention the score obtained by the method described herein can be used in the treatment of prostate cancer to, without being limited to, classify the aggressiveness of a prostate carcinoma during initial diagnosis, before, during or after treatment of respective cancer. In a further embodiment of the invention the score obtained by the method described herein can be used to calculate the stage of and/or to classify the aggressiveness of metastases of a prostate carcinoma during initial diagnosis, before, during or after cancer treatment. The present invention further relates to a method for classifying patients according to their gene expression profile into a numeric score. Herein, this score classifies patients associated with adverse prognosis and reduced DoD-free survival or BCR-free survival with a score value of >0. A score value of ≤0 further classifies patients with an extended DoD-free or biochemical recurrence-free survival (Figures 2 and 5; high probability of long DoD-free or long BCR-free survival (black curve); low probability of DoD-free and BCR-free survival (grey curve)). Therefore, the cutoff is a score value of 0 to distinguish between an increased or a reduced risk of an adverse prognosis and/or DoD and an increased or a reduced chance of extended BCR-free and/or DoD-free survival (see also Figure 2 and Figure 3; Table 1, Table 3, Table 4 and Table 5).

In one embodiment the score obtained by the herein described method can be used in the treatment of cancer, such as, but not limited to, prostate cancer, prostate carcinomas or metastases of prostate carcinomas, to support and improve the decision whether adjuvant therapy after surgery, such as RP, should be scheduled, as well as to support and improve the choice of therapy for patients after initial diagnosis, during active surveillance and after focal, organ-preserving therapies. Herein "therapy" includes, but is not limited to, surgery, radical prostatectomy (RP), "active surveillance" or "watchful waiting", radiotherapy, focal therapies, and wherein "focal therapy" includes, but is not limited to, vascular targeted photodynamic therapy, such as, but without being limited to, TOOKAD, High Intensity Focused Ultrasound (HiFu) and irreversible Electroporation (IRE), such as, but without being limited to, NanoKnife and Focal Cryoablation.

In one embodiment of the present invention, when correlating the score, obtained by the method described herein, to the proportion of prostate carcinoma patients developing BCR, said score of a value of -0.2 relates to an estimated conditional probability of BCR of respective prostate carcinoma patient of about 5%, whereas said score of a value of 0 relates to an estimated conditional probability of BCR of about 11% and said score of a value of 0.2 relates to an estimated conditional probability of BCR of about 23% (Table 3; Figure 3;).

In another embodiment the herein disclosed method and ProstaTrend score relate to a method for predicting the outcome or classifying and/or stratifying the disease of prostate carcinoma patients and is calculated by combining the expression values of all selected genes in a patient-wise prognostic score.

In one embodiment of the invention the method comprises the detection of nucleic acids that have a target nucleic acid sequence identical to one or more of said reference genes, or one or more transcripts or isoforms thereof, listed in Table 2, followed by the determination or measurement of the abundance of each target nucleic acid sequence in said sample, wherein said abundance is used to calculate the gene expression value of said corresponding reference gene from the group of reference genes listed in Table 2 in said sample.

In one embodiment of the invention, said score can be calculated by the herein disclosed method, wherein the abundances in said sample of all known isoforms or transcripts of each of said reference genes listed in Table 2 are summarized for each reference gene during the calculation.

In another embodiment the invention relates to a method for predicting the outcome or classifying the disease of prostate carcinoma patients, the method comprising the steps of: a) obtaining a sample from said patient, wherein the sample comprises a plurality of nucleic acids, b) analyzing the plurality of nucleic acids, thereby determining the abundance of at least two one or more different target nucleic acid sequences, c) normalizing said target nucleic acid sequences against a reference dataset and standardization, d) weighting the abundance of the at least one or more different target nucleic acid using the respective log hazard ratios presented in Table 2, and e) calculating a score value by summarizing the abundancies of all known isoforms or transcripts of each of said genes listed in Table 2, wherein, if the score value is above a threshold value of 0, the patient is classified as having a high risk of death of disease and/or biochemical recurrence and/or if the score value is below a threshold value of 0, the patient is classified as having a low risk of death of disease and/or biochemical recurrence.

In detail, in one embodiment of the invention the disclosed method of calculating a predictive score value (ProstaTrend score) for use in predicting the outcome or in classifying the disease of a prostate carcinoma patient comprises the calculation of expression values for at least one reference gene that were detected to be expressed in the sample, based on the abundancies of corresponding target nucleic acid sequences in said sample, wherein the abundancies of all target nucleic acid sequences, which are identical to the sequence of one or more isoforms and/or transcripts of a reference gene are summarized for each reference gene respectively, and wherein for each patient k the median over the weighted standardized gene expression values of all genes selected for said calculation $g_i$ is calculated applying:

$$ProstaTrend_k = Median(logHR_i * g_i),$$

wherein the weight for each significant gene i is the estimated univariate log hazard ratio (logHR) from the Cox regression meta-analysis to account for the direction of the prognostic effect of gene i as well as for the effect size, wherein a negative ProstaTrend-score (score value <0) characterizes patients with lower risk of an adverse prognosis and an increased chance of extended BCR-free and/or DoD-free survival, while a positive score (score value >0) characterizes patients with an increased risk of an adverse prognosis and a lower chance of extended BCR-free and/or DoD-free survival.

Briefly, in one embodiment of the invention the score (ProstaTrend score) is calculated herein by using the expression abundancies of each one or more target nucleic acid sequences from a sample that have an identical sequence to the sequence of one of the genes listed in Table 2 or one of the isoforms or transcripts of said genes, and wherein the score is calculated by summarizing the abundancies of all known isoforms or transcripts of each of said genes listed in Table 2.

In one embodiment of the invention said score value is calculated by summarizing the abundancies of all known isoforms or transcripts of each of said reference genes listed in Table 2.

In one embodiment of the invention, the ProstaTrend score for a patient is calculated as the median of the weighted, normalized and standardized gene expression values according to the before mentioned method using a set of at least 50 or more prognostic genes, herein also referred to as reference genes, which are disclosed herein in Table 2.

In one embodiment of the present invention said set of reference genes used to calculate the ProstaTrend score comprises at least 10, 50, 100, 150, 200, 250, 300, 350, 400, 450, 500, 750, 1000, 1250 or 1396 of the genes disclosed in Table 2. In another embodiment said set of reference genes comprises a number within the range of 50-1396 of the genes disclosed in Table 2. In another embodiment of this invention said set of reference genes used to calculate the ProstaTrend score comprises a number within the range of 50-500 of the genes disclosed in Table 2. In a preferred embodiment of this invention said set of reference genes used to calculate the ProstaTrend score comprises a number within the range of 100-500 (Figure 8 and Figure 9) of the genes disclosed in Table 2. In an even more preferred embodiment of this invention said set of reference genes used to calculate the ProstaTrend score comprises a number within the range of 100-300 of the genes disclosed in Table 2.

In one embodiment of the invention the normalization of gene expression values is done cohort-wise, if a set of samples from the same patient cohort is provided, or, in case of a single sample, an add-on normalization with respect to the normalized gene expression values of the training cohort is conducted. Further, herein the expression value of each gene, herein also referred to as reference gene, is weighted by the log hazard ratio (logHR) from the Cox regression meta-analysis of the training cohort. Herein, the weights for each gene are provided in Table 2.

In one embodiment, the present invention relates to a method for use in the treatment of prostate cancer, the method comprising the steps of: a) obtaining a sample from a patient, wherein the sample comprises a plurality of nucleic acids, b) analyzing the plurality of nucleic acids, thereby determining the abundancy of at least one or more different target nucleic acid sequences, c) normalizing of target nucleic acid sequences or target nucleic acid sequence abundancies against a reference dataset and standardization, d) weighting the abundancy of the at least one or more different target nucleic acid using the respective log hazard ratios presented in Table 2 and e) calculating a score value, and wherein, if the score value is above a threshold value of 0, the patient is classified as having a high risk of death of disease and/or biochemical recurrence and/or if the score value is below a threshold value of 0, the patient is classified as having a low risk of death of disease and/or biochemical recurrence.

In another embodiment the herein disclosed method relates to a method for use in the treatment of prostate cancer, the method comprising the steps of: a) obtaining a sample from a patient, wherein the sample comprises a plurality of nucleic acids, b) analyzing the plurality of nucleic acids, thereby determining the abundancy of at least one or more different target nucleic acid sequences, c) normalizing of the target nucleic acid sequence abundancies against a reference dataset and standardization, d) weighting the abundancy of the at least one or more different target nucleic acid using a corresponding reference value and e) calculating a score value, and wherein the outcome or classification of the disease of a prostate carcinoma patient from whom said sample was derived is predicted based on said score value, and wherein, if the score value is above a threshold value of 0, the patient is classified as having a high risk of death of disease and/or biochemical recurrence and/or if the score value is below a threshold value of 0, the patient is classified as having a low risk of death of disease and/or biochemical recurrence.

In another embodiment of said method for use in the treatment of prostate cancer, the outcome or classification of the disease of a prostate carcinoma patient from whom said sample was derived is predicted based on said score value.

The embodiments discussed herein with reference to method for predicting the outcome or classifying the disease of a prostate carcinoma patient apply also to the method for use in the treatment of prostate cancer.

EXAMPLES

**Example 1: Selection of marker genes for the calculation of a score for prostate cancer patient classification and outcome prediction.**

To identify genes associated with DoD four clinical cohorts (Figure 1) were incorporated in the "ProstaTrend" dataset, relating to the present invention. Weighted univariate Cox regression analysis was applied on all 20,821 genes that passed the expression quality filter for all included cohorts separately and the estimated logHRs and the respective SEs were used in a fixed-effect-model meta-analysis. The results of the meta-analysis are exemplarily presented in Figure 10A for the gene NUSAP1, one of the genes significantly associated with DoD in the cohort and included in other signatures for risk stratification in prostate cancer. The histogram of the p-values of the meta-analysis for all genes shows strong enrichment of p-values near zero (Figure 10B). Fitting a mixture model to the p-value distribution using the R-package fdrtool (Strimmer at al., 2008; Bioinformatics) a fraction of 32.6% of all analyzed genes was estimated to be prognostic. For a conservative correction for multiple testing, 1,396 genes with a tail-based FDR < 0.05 were selected as a marker set including 1,214 known protein-coding genes (Figure 10C).

The herein disclosed list of reference genes (Table 2), which can be used to calculate the herein disclosed prognostic ProstaTrend score, is consistent with already established prognostic gene signatures. In total, 24 genes of the Decipher, Oncotype, and Prolaris signatures were included in the list of genes disclosed by the present invention, which can be used to calculate the herein disclosed prognostic ProstaTrend score. Removing these genes did not affect the accuracy of the calculated ProstaTrend score (Figure 7). A direct comparison of the list of genes disclosed by the present invention (Table 2) with the Prolaris gene set, which was initially similarly developed to predict long-term prognosis after RP, showed a stronger effect for the list of genes disclosed by the present invention (Table 2), underling the benefit of evaluating all tumor-relevant pathways for molecular risk stratification, as disclosed by the present invention (Figure 7A and 7C).

**Example 2: Validation of the prognostic relevance of the ProstaTrend score.**

The ProstaTrend score, described by the current invention, admitted high discrimination accuracy in the "FF_array_RP" patient cohort (Figure 1), validating the inference technique by leave-one-out cross-validation in a training patient cohort, and therefore the final ProstaTrend score to predict time to DoD for a patient inferred from the complete patient set (Figure 5A and Table 4).

Patients with score values > 0 had significantly reduced prostate cancer-specific survival as compared to patients with score values ≤ 0 (5-year survival: 83% vs. 98%; log-rank test: p = 0.0002). The ProstataTrend score remained an independent predictor for the time to DoD in a multivariate Cox regression after adjusting for Gleason grading group (GG) ≥ 3 and positive resection status (Figure 5B, p=0.001). Adjusting the model for pathological stage was not reasonable in the training cohort, because no DoD cases were observed for low risk pathological stage (pT2).

To confirm the prognostic relevance of the continuous ProstaTrend score, we assessed the concordance with the order of DoD events. A univariable analysis showed a C-index of 78.5% [95%-CI: 68.5-85.9%] for the cross validated ProstaTrend score.

**Example 3: Validation of effectiveness of the ProstaTrend score method for the prediction of outcome and patient prognosis.**

In order to supply a strong validation study, the effectiveness of the ProstaTrend score, disclosed by the present application, in an external patient cohort (TCGA PRAD) was demonstrated. The ProstaTrend score allowed for significant discrimination of different risk groups (Figure 2 and Table 3). Patients with a score value > 0 showed significantly reduced BCR-free survival compared to patients with a score value ≤ 0 (2-year BCR-free survival: 78% vs. 95%; log-rank test: $p=7.2e^{-7}$). Similarly, the ProstaTrend score admitted persistent relevance in a multivariate Cox regression model with GG ≥ 3, pathological stage ≥T3, and positive resection status as additional factors (Figure 2, p=0.037). A concordance analysis of the ProstaTrend score showed 71% [95%-CI: 62.6-77.2%] concordance with the order of BCR events.

In univariate Cox regression analysis, the dichotomized ProstaTrend score (dichotomized, for example dividing the ProstaTrend score in two separate classes: value 1 in case of a ProstaTrend score value > 0 and value 0 in case of a ProstaTrend score value ≤ 0) was the most significant predictor for BCR compared to log(PSA), GG ≥ 3, pathological stage ≥ T3, lymph node status, age and resection status. A pairwise analysis with the ProstaTrend score and each clinical feature resulted in the statistical significance of the ProstaTrend score in all models. In each paired model, the ProstaTrend score was more significant than the clinical feature and the ProstaTrend score remained significant when adjusted for GG and stage, for example all clinical variables that remained significant in the pairwise analysis (see Table 1).

Similarly, in univariate Cox regression analysis the continuous ProstaTrend score was the most significant predictor for BCR compared to log(PSA), GG≥3, pathological stage≥T3, age and resections status. A pairwise analysis with ProstaTrend and each clinical feature resulted in the statistical significance of ProstaTrend in all models. ProstaTrend remained significant when adjusted for GG and stage, i.e. all clinical variables that remained significant in the pairwise analysis (see Table 5).

To assess the prognostic potential of the gene expression signature in a low and intermediate risk group the ProstaTrend score was applied to patients with Gleason score (GS) ≤ 7 (Figure 2A). The gene expression-based classification indicated poor prognosis (ProstaTrend score > 0) for 20% (38/186) of these patients. BCR-free survival of these patients was significantly shorter compared to patients with a ProstaTrend score ≤ 0 (Figure 2A; log-rank test p = 0.0004; 2-year BCR-free survival: 85% vs. 98%). In patients with GS >7, 2-year BCR-free survival was 75% for patients with score >0 and 85% for patients with score≤0 (Figure 2A; log-rank test p=0.076). An increase of both, the mean ProstaTrend score and the overall frequency of BCR for higher GS groups could be observed (Figure 2). Furthermore, an elevated ProstaTrend score was associated with a higher rate of BCR events within GS subgroups.

To investigate the gain of prediction accuracy through the application of the ProstaTrend score, we applied the concordance analysis for a clinical model based on GG, pathological stage and resection status with and without inclusion of the ProstaTrend score. With the inclusion of the ProstaTrend score the concordance index increased by 4.9% [95%-CI: -0.3-8.6%] from 71.4% to 76.3%.

**Example 4: Unsupervised clustering analysis of overexpressed genes in prostate cancer patients reveals functional context of genes used herein to calculate ProstaTrend score.**

To investigate the functional context of genes associated with prognosis in prostate cancer unsupervised clustering with self-organizing maps (SOM) was performed. OposSOM v.1.1619 was applied to the quality-filtered and between-array normalized genes of the FF_array_RP patient cohort. 23 correlated overexpression spots were identified (Figure 6A). For six spots, a significant enrichment of genes included in the list of genes disclosed by the present application (Table 2), which are used to calculate the ProstaTrend score was observed. Spot B and A were associated with adverse prognosis (Figure 6B and E) and linked to proliferation (Spot B GO-enrichment: cell cycle $p = 8*10^{-46}$) and immune response (Spot A GO-enrichment: immune system process $p=1*10^{-99}$). Spot I is linked to negative regulation of growth and androgen signaling (GO-enrichment: negative regulation of growth $2*10^{-10}$ and GSEA: hallmark_androgen_response $p=1*10^{-14}$) and associated with advantageous prognosis. In contrast, a clear biological process linked to spots S, J and V could not be identified.

**Example 5: Comparison of the genes disclosed herein for the calculation of the ProstaTrend score with known prostate cancer related gene signatures.**

The list of genes disclosed by the present application (Table 2) that is used for the calculation of a ProstaTrend score comprises genes of all gene sets representing the biological processes of the "hallmarks of cancer" (Hanahan et al., 2011; Cell and Loeffler-Wirth et al, 2019; Genome Med.). In contrast, Decipher, Oncotype, and Prolaris had no overlap with at least three hallmark gene sets indicating that the spectrum of biological characteristics covered by these assays is limited compared to the ProstaTrend score. The "hallmarks of cancer" have been defined as a set of functional capabilities that a tumor acquires during tumor development (Hanahan et al., 2011; Cell). We compared previously defined gene sets referring to these "hallmarks" (Loeffler-Wirth et al, 2019; Genome Med.) to determine the overlap with the herein disclosed gene set (Table 2) used to calculate the ProstaTrend score and thus the potential of the ProstaTrend score to represent these biological processes. All "hallmarks" were represented within the herein disclosed gene set (Table 2), which is used herein to calculate the ProstaTrend score, for multiple of genes of said gene set: angiogenesis (n=17), controlling genomic instability (n=50), glucose energetics (n=3), inflammation (n=18), invasion and metastasis (n=71), proliferation (n=156), replicative immortality (n=57) and resisting death (n=45) suggesting that the herein disclosed ProstaTrend score covers the full spectrum of biological characteristics involved in tumorigenesis.

**Claims**

1. A method for predicting the outcome or classifying the disease of a prostate carcinoma patient, the method comprising the steps of:

   a) obtaining a sample from said patient, wherein the sample comprises a plurality of nucleic acids,
   b) analyzing the plurality of nucleic acids, thereby determining the abundance of at least one or more different target nucleic acid sequences,
   c) normalizing of the target nucleic acid sequence abundancies against a reference dataset and standardization,
   d) weighting the abundance of the at least one or more different target nucleic acid using a corresponding reference value and
   e) calculating a score value,
   wherein, if the score value is above a threshold value of 0, the patient is classified as having a high risk of death of disease and/or biochemical recurrence and/or if the score value is below a threshold value of 0, the patient is classified as having a low risk of death of disease and/or biochemical recurrence.

2. The method according to claim 1, wherein each of the one or more different target nucleic acid sequences is identical to the nucleic acid sequence of a corresponding specific transcript or isoform of a reference gene selected from the group of reference genes listed in Table 2.

3. The method according to one of the claims 1 or 2, wherein said abundancy of the target nucleic acids is weighted in step d) by the logHR value of a corresponding reference gene listed in Table 2, wherein the nucleic acid sequence of said reference gene, or of a transcript or isoform thereof, is identical to the nucleic acid sequence of one or more of said target nucleic acid sequences from said sample.

4. The method according to any of the claims 1 to 3, wherein the score value for said patient is calculated by using the abundancies of said target nucleic acid sequences in said sample to calculate expression values for all corresponding reference genes in said sample, and wherein for each reference gene the abundancies of all target nucleic sequences that are identical to the nucleic acid sequence of at least one or more transcripts and/or isoforms of said reference gene are summarized for each said reference gene, and wherein for each patient k the median over the weighted standardized gene expression values of all reference genes selected for said calculation $g_i$ is calculated applying:

$$score_k = Median(logHR_i * g_i),$$

and

wherein the weight for each significant gene i is the estimated univariate log hazard ratio *(logHR)* from the Cox regression meta-analysis of a training cohort to account for the direction of the prognostic effect of said gene *i* as well as for the effect size, and

wherein a normalization of said gene expression values is done cohort-wise, if a set of samples from the same cohort is provided, or, in case of a single sample, an add-on normalization with respect to the normalized gene expression values of the training cohort is conducted, and wherein a negative value of said score characterizes said patient with lower risk of an adverse prognosis and/or death of disease and an increased chance of extended biochemical recurrence-free and/or death of disease-free survival, and a positive value of said score characterizes said patient with a higher risk of an adverse prognosis and/or death of disease and an decreased chance of extended biochemical recurrence-free and/or death of disease-free survival.

5. The method according to any of the claims 1-4, wherein the number of said specific reference genes used to calculate said score and selected from the genes listed in Table 2 with an identical nucleic acid sequence to at least one of said target nucleic acid sequences from said sample is from the range of 50-1396.

6. The method according to any of the claims 1-5, wherein said nucleic acid analysis comprises the sequential use of one or more techniques selected from the group of reverse transcription, microarray analysis, DNA sequencing, Next Generation Sequencing, NanoString Hyb & Seq NGS analysis, nCounter-analysis, nanopore sequencing, third-generation sequencing, Sanger sequencing, digital or droplet PCR analysis, quantitative real-time PCR analysis, PCR amplification, RNA sequencing, DNA sequencing, Whole Genome Sequencing, Whole Exome Sequencing, Single Cell Sequencing, Targeted Sequencing, spatial sequencing, ATAC-Seq and ChIP-Seq.

7. The method according to any of the claims 1-6, wherein said patient is a human patient, an animal or a cell culture of a patient-derived sample of a human patient, of an animal or an animal model, of a sample from an "organ-on-a-chip" model, a sample from a microphysiological system, of a cell line or an organoid.

8. The method according to any of the claims 1-7, wherein the sample is selected from the group consisting of an organ, a tissue, a biopsy, a liquid biopsy, a blood sample, a patient derived xenograft sample, of a sample from an "organ-on-a-chip" model, of a sample from a microphysiological system, of a cell line or a cell culture of a patient-derived sample of a human patient, an animal or an animal model, a cell line or an organoid.

9. A method for use in the treatment of prostate cancer, the method comprising the steps of:

a) obtaining a sample from a patient, wherein the sample comprises a plurality of nucleic acids,
b) analyzing the plurality of nucleic acids, thereby determining the abundance of at least one or more different target nucleic acid sequences,
c) normalizing of the target nucleic acid sequence abundancies against a reference dataset and standardization,
d) weighting the abundance of the at least one or more different target nucleic acid using a corresponding reference value and calculating a score value, and

wherein the outcome or classification of the disease of a prostate carcinoma patient from whom said sample was derived is predicted based on said score value, and wherein, if the score value is above a threshold value of 0, the patient is classified as having a high risk of death of disease and/or biochemical recurrence and/or if the score value is below a threshold value of 0, the patient is classified as having a low risk of death of disease and/or biochemical recurrence.

**Figure 1**

Figure 2

Figure 3

**Figure 4**

**Figure 5**

**Figure 6**

**Figure 7**

**Figure 8**

**Figure 9**

AUC vs number of genes in UKDP

Number of included genes [sorted by significance]

**Figure 10**

A

NUSAP1

| Study | logHR | logHR | 95%-CI | Weight |
|---|---|---|---|---|
| FF_array_RP (n=164) | | 0.59 | [ 0.18; 1.01] | 79.5% |
| FF_seq_RP (n=40) | | 1.46 | [ 0.21; 2.71] | 8.8% |
| FFPE_seq_RP (n=13) | | 0.86 | [−1.11; 2.83] | 3.5% |
| FFPE_seq_Bx1 (n=16) | | 1.17 | [−0.12; 2.46] | 8.2% |
| **Fixed effect model** | | **0.73** | **[ 0.36; 1.10]** | **100.0%** |

z = 3.85 (p < 0.01)

B

Type of Statistic: p−Value $(\eta_0 = 67.4\%)$

— Mixture
- - - Null Component
— Alternative Component

p−values

C

- protein coding (n=1214)
- lncRNA (n=111)
- pseudogene (n=34)
- unknown (n=20)
- Ig / TcR (n=16)
- small ncRNA (n=1)

log hazard ratio

20

**Table 1**

| | | logHR [95% CI] | p-value |
|---|---|---|---|
| **Univariate** | **ProstaTrend (score>0)** | **1.73 [0.96-2.50]** | **1.1e-05** |
| | PSA (log scale) | 0.38 [-0.05-0.80] | 0.084 |
| | Gleason Grading (RP GG≥3) | 2.53 [1.11-3.95] | 0.0005 |
| | Pathological stage (stage≥T3) | 2.46 [1.03-3.88] | 0.0007 |
| | pN | 1.05 [0.27-1.83] | 0.008 |
| | Age (continious) | 0.04 [-0.01-0.08] | 0.14 |
| | Resection status (R+) | 0.53 [-0.08-1.14] | 0.09 |
| **pairwise** | **ProstaTrend** PSA | **1.52 [0.75-2.30]** 0.30 [-0.12-0.73] | **0.0001** 0.16 |
| | **ProstaTrend** Gleason GG≥3 | **1.16 [0.36-1.96]** 1.96 [0.49-3.43] | **0.005** 0.009 |
| | **ProstaTrend** Pathological stage | **1.35 [0.57-2.13]** 2.03 [0.59-3.47] | **0.0007** 0.006 |
| | **ProstaTrend** pN | **1.41 [0.61-2.21]** 0.53 [-0.27-1.34] | **0.0006** 0.19 |
| | **ProstaTrend** Age | **1.69 [0.91-2.47]** 0.02 [-0.03-0.06] | **2.0e-05** 0.51 |
| | **ProstaTrend** Resection status | **1.68 [0.908-2.46]** 0.23 [-0.39-0.85] | **2.6e-05** 0.47 |
| **complex model** | **ProstaTrend** Gleason score Pathological stage | **0.96 [0.16-1.77]** 1.52 [0.02-3.02] 1.70 [0.25-3.15] | **0.019** 0.047 0.022 |

Table 2

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000101057.15_2 | NA | NA | MYBL2 | 3,99E-05 | 1,12112061 | protein_coding | protein coding | NA | NA |
| ENSG00000142731.10_2 | NA | NA | PLK4 | 3,99E-05 | 1,11016944 | protein_coding | protein coding | NA | NA |
| ENSG00000168476.11_3 | NA | NA | REEP4 | 3,99E-05 | 0,98136061 | protein_coding | protein coding | NA | NA |
| ENSG00000184445.11_3 | NA | NA | KNTC1 | 3,99E-05 | 1,04829639 | protein_coding | protein coding | NA | NA |
| ENSG00000187741.14_3 | NA | NA | FANCA | 3,99E-05 | 1,02193747 | protein_coding | protein coding | NA | NA |
| ENSG00000106290.14_3 | NA | NA | TAF6 | 3,99E-05 | 0,93097892 | protein_coding | protein coding | NA | NA |
| ENSG00000080839.11_3 | NA | NA | RBL1 | 5,00E-05 | 1,02535537 | protein_coding | protein coding | NA | NA |
| ENSG00000185507.19_2 | NA | NA | IRF7 | 7,67E-05 | 0,83973088 | protein_coding | protein coding | NA | NA |
| ENSG00000249471.7_2 | NA | NA | ZNF324B | 0,00010272 | 0,85070836 | protein_coding | protein coding | NA | NA |
| ENSG00000169221.13_2 | NA | NA | TBC1D10B | 0,00010485 | 1,01975238 | protein_coding | protein coding | NA | NA |
| ENSG00000226419.7_3 | NA | NA | SLC16A1-AS1 | 0,0001067 | 0,89249536 | antisense_RNA | lncRNA | NA | NA |
| ENSG00000172780.16_3 | NA | NA | RAB43 | 0,00014032 | 0,92235211 | protein_coding | protein coding | NA | NA |
| ENSG00000117472.9_2 | NA | NA | TSPAN1 | 0,00016107 | -0,89722275 | protein_coding | protein coding | NA | NA |
| ENSG00000137648.17_3 | NA | NA | TMPRSS4 | 0,00016905 | 0,6666111 | protein_coding | protein coding | NA | NA |
| ENSG00000175643.8_2 | NA | NA | RMI2 | 0,00017709 | 0,9186168 | protein_coding | protein coding | NA | NA |
| ENSG00000167767.13_2 | NA | NA | KRT80 | 0,00017883 | 0,67195441 | protein_coding | protein coding | NA | NA |
| ENSG00000173137.11_2 | NA | NA | ADCK5 | 0,00018625 | 0,81258643 | protein_coding | protein coding | NA | NA |
| ENSG00000274641.1_2 | NA | NA | HIST1H2BO | 0,00021106 | 0,8363546 | protein_coding | protein coding | NA | NA |
| ENSG00000099953.9_3 | NA | NA | MMP11 | 0,00021511 | 0,80488689 | protein_coding | protein coding | NA | NA |
| ENSG00000157778.8_2 | NA | NA | PSMG3 | 0,00022257 | 0,87597451 | protein_coding | protein coding | NA | NA |
| ENSG00000072864.14_3 | NA | NA | NDE1 | 0,00023965 | 0,71093973 | protein_coding | protein coding | NA | NA |
| ENSG00000168298.6_2 | NA | NA | HIST1H1E | 0,00024986 | 1,04632655 | protein_coding | protein coding | NA | NA |
| ENSG00000140534.13_3 | NA | NA | TICRR | 0,00026482 | 0,83169107 | protein_coding | protein coding | NA | NA |
| ENSG00000073111.13_3 | NA | NA | MCM2 | 0,00026928 | 0,9025888 | protein_coding | protein coding | NA | NA |
| ENSG00000112118.18_2 | NA | NA | MCM3 | 0,00028066 | 0,90436567 | protein_coding | protein coding | NA | NA |
| ENSG00000211673.2_2 | NA | NA | IGLV3-1 | 0,00031418 | 0,60812262 | IG_V_gene | Ig / TcR | NA | NA |
| ENSG00000275713.2_2 | NA | NA | HIST1H2BH | 0,00033763 | 0,76923866 | protein_coding | protein coding | NA | NA |
| ENSG00000064655.18_4 | NA | NA | EYA2 | 0,0003427 | 0,84225416 | protein_coding | protein coding | NA | NA |
| ENSG00000129810.14_4 | NA | NA | SGO1 | 0,00036892 | 0,81496776 | protein_coding | protein coding | NA | NA |
| ENSG00000109674.3_2 | NA | NA | NEIL3 | 0,00037469 | 0,94230842 | protein_coding | protein coding | NA | NA |
| ENSG00000136295.14_2 | NA | NA | TTYH3 | 0,0003942 | 0,76534283 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000280734.2_2 | NA | NA | LINC01232 | 0,00039989 | 0,78707865 | processed_transcript | lncRNA | NA | NA |
| ENSG00000172818.9_2 | NA | NA | OVOL1 | 0,00040539 | 0,7750343 | protein_coding | protein coding | NA | NA |
| ENSG00000273703.1_2 | NA | NA | HIST1H2BM | 0,00041562 | 0,83829991 | protein_coding | protein coding | NA | NA |
| ENSG00000105705.15_4 | NA | NA | SUGP1 | 0,00042631 | 0,83669835 | protein_coding | protein coding | NA | NA |
| ENSG00000137807.13_2 | NA | NA | KIF23 | 0,00043155 | 0,96513916 | protein_coding | protein coding | NA | NA |
| ENSG00000175550.7_2 | NA | NA | DRAP1 | 0,00043536 | 0,74006943 | protein_coding | protein coding | NA | NA |
| ENSG00000013588.7_3 | NA | NA | GPRC5A | 0,00044284 | 0,6971623 | protein_coding | protein coding | NA | NA |
| ENSG00000014138.8_3 | NA | NA | POLA2 | 0,0004551 | 0,94327885 | protein_coding | protein coding | NA | NA |
| ENSG00000167977.8_3 | NA | NA | KCTD5 | 0,00045975 | 0,81674266 | protein_coding | protein coding | NA | NA |
| ENSG00000186340.14_3 | NA | NA | THBS2 | 0,00046043 | 0,90951411 | protein_coding | protein coding | NA | NA |
| ENSG00000187990.4 | NA | NA | HIST1H2BG | 0,00048896 | 0,78909527 | protein_coding | protein coding | NA | NA |
| ENSG00000106012.17_3 | NA | NA | IQCE | 0,00048944 | 0,84262332 | protein_coding | protein coding | NA | NA |
| ENSG00000197993.7_3 | NA | NA | KEL | 0,00050943 | 0,54971757 | protein_coding | protein coding | NA | NA |
| ENSG00000120075.5_3 | NA | NA | HOXB5 | 0,00051519 | 0,53437145 | protein_coding | protein coding | NA | NA |
| ENSG00000260027.4_3 | NA | NA | HOXB7 | 0,00051698 | 0,49386273 | protein_coding | protein coding | NA | NA |
| ENSG00000124635.8_2 | NA | NA | HIST1H2BJ | 0,00052555 | 0,7775271 | protein_coding | protein coding | NA | NA |
| ENSG00000161904.11_3 | NA | NA | LEMD2 | 0,00056188 | 0,80903005 | protein_coding | protein coding | NA | NA |
| ENSG00000108511.9_3 | NA | NA | HOXB6 | 0,00058618 | 0,39612369 | protein_coding | protein coding | NA | NA |
| ENSG00000144648.15_3 | NA | NA | ACKR2 | 0,00060561 | 0,63111263 | protein_coding | protein coding | NA | NA |
| ENSG00000102384.13_2 | NA | NA | CENPI | 0,00061292 | 0,76898174 | protein_coding | protein coding | NA | NA |
| ENSG00000198026.7_2 | NA | NA | ZNF335 | 0,00062574 | 0,79016098 | protein_coding | protein coding | NA | NA |
| ENSG00000167535.7_2 | NA | NA | CACNB3 | 0,00062609 | 0,86669818 | protein_coding | protein coding | NA | NA |
| ENSG00000185130.5_2 | NA | NA | HIST1H2BL | 0,00063319 | 0,7914076 | protein_coding | protein coding | NA | NA |
| ENSG00000120875.8_2 | NA | NA | DUSP4 | 0,00065677 | 0,56172222 | protein_coding | protein coding | NA | NA |
| ENSG00000124201.14_2 | NA | NA | ZNFX1 | 0,0007558 | 0,78878357 | protein_coding | protein coding | NA | NA |
| ENSG00000184270.4_2 | NA | NA | HIST2H2AB | 0,00080913 | 0,94823142 | protein_coding | protein coding | NA | NA |
| ENSG00000149923.13_2 | NA | NA | PPP4C | 0,00081998 | 0,93073969 | protein_coding | protein coding | NA | NA |
| ENSG00000198554.11_3 | NA | NA | WDHD1 | 0,0008281 | 0,78217644 | protein_coding | protein coding | NA | NA |
| ENSG00000165949.12_3 | NA | NA | IFI27 | 0,00084062 | 0,49536814 | protein_coding | protein coding | NA | NA |
| ENSG00000162949.16_3 | NA | NA | CAPN13 | 0,00088396 | 0,51781688 | protein_coding | protein coding | NA | NA |
| ENSG00000275379.1_2 | NA | NA | HIST1H3I | 0,00092393 | 0,94715831 | protein_coding | protein coding | NA | NA |
| ENSG00000196177.12_2 | NA | NA | ACADSB | 0,00093435 | -1,08245838 | protein_coding | protein coding | NA | NA |
| ENSG00000270550.1_2 | NA | NA | IGHV3-30 | 0,00097275 | 0,59395239 | IG_V_gene | Ig / TcR | NA | NA |
| ENSG00000167670.15_2 | NA | NA | CHAF1A | 0,0009759 | 0,76222794 | protein_coding | protein coding | NA | NA |
| ENSG00000144645.13_3 | NA | NA | OSBPL10 | 0,00099492 | 0,86009928 | protein_coding | protein coding | NA | NA |
| ENSG00000128563.13_3 | NA | NA | PRKRIP1 | 0,00099937 | 0,8390338 | protein_coding | protein coding | NA | NA |

23

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000168539.3_3 | NA | NA | CHRM1 | 0,00101083 | -0,96731702 | protein_coding | protein coding | NA | NA |
| ENSG00000182809.10_2 | NA | NA | CRIP2 | 0,00103717 | 0,67176066 | protein_coding | protein coding | NA | NA |
| ENSG00000122966.15_4 | NA | NA | CIT | 0,00106683 | 0,83919404 | protein_coding | protein coding | NA | NA |
| ENSG00000147883.10_2 | NA | NA | CDKN2B | 0,00106693 | 0,81021358 | protein_coding | protein coding | NA | NA |
| ENSG00000196652.11_2 | NA | NA | ZKSCAN5 | 0,00107916 | 0,44009581 | protein_coding | protein coding | NA | NA |
| ENSG00000111331.12_2 | NA | NA | OAS3 | 0,00109651 | 0,53603873 | protein_coding | protein coding | NA | NA |
| ENSG00000137200.12_2 | NA | NA | CMTR1 | 0,00113692 | 0,76120358 | protein_coding | protein coding | NA | NA |
| ENSG00000066279.17_3 | NA | NA | ASPM | 0,00116377 | 0,73287134 | protein_coding | protein coding | NA | NA |
| ENSG00000163932.13_3 | NA | NA | PRKCD | 0,00125449 | 0,77025578 | protein_coding | protein coding | NA | NA |
| ENSG00000174371.16_3 | NA | NA | EXO1 | 0,00132529 | 0,73358191 | protein_coding | protein coding | NA | NA |
| ENSG00000275221.1_2 | NA | NA | HIST1H2AK | 0,00133225 | 0,7162784 | protein_coding | protein coding | NA | NA |
| ENSG00000276410.3_3 | NA | NA | HIST1H2BB | 0,00135372 | 0,68007407 | protein_coding | protein coding | NA | NA |
| ENSG00000161533.11_3 | NA | NA | ACOX1 | 0,00136576 | -0,88404191 | protein_coding | protein coding | NA | NA |
| ENSG00000166689.15_4 | NA | NA | PLEKHA7 | 0,00141519 | 0,73680527 | protein_coding | protein coding | NA | NA |
| ENSG00000172732.11_2 | NA | NA | MUS81 | 0,00144316 | 0,68308384 | protein_coding | protein coding | NA | NA |
| ENSG00000119242.8_3 | NA | NA | CCDC92 | 0,00148072 | 0,88543429 | protein_coding | protein coding | NA | NA |
| ENSG00000119969.14_3 | NA | NA | HELLS | 0,00151227 | 0,8199534 | protein_coding | protein coding | NA | NA |
| ENSG00000118193.11_2 | NA | NA | KIF14 | 0,00152505 | 0,81338812 | protein_coding | protein coding | NA | NA |
| ENSG00000149636.15_4 | NA | NA | DSN1 | 0,00154147 | 0,69800473 | protein_coding | protein coding | NA | NA |
| ENSG00000093009.9_2 | NA | NA | CDC45 | 0,00155368 | 0,82008206 | protein_coding | protein coding | NA | NA |
| ENSG00000142515.14_3 | NA | NA | KLK3 | 0,00155903 | -0,73448852 | protein_coding | protein coding | NA | NA |
| ENSG00000007541.16_1 | NA | NA | PIGQ | 0,00157708 | 0,71669391 | protein_coding | protein coding | NA | NA |
| ENSG00000115884.10_2 | NA | NA | SDC1 | 0,00161103 | 0,73126032 | protein_coding | protein coding | NA | NA |
| ENSG00000273321.1_3 | NA | NA | AC023983.2 | 0,00161824 | 0,56092315 | lincRNA | lncRNA | NA | NA |
| ENSG00000144560.14_3 | NA | NA | VGLL4 | 0,00164177 | 0,91029338 | protein_coding | protein coding | NA | NA |
| ENSG00000105717.13_3 | NA | NA | PBX4 | 0,00166971 | 0,80561333 | protein_coding | protein coding | NA | NA |
| ENSG00000163082.9_2 | NA | NA | SGPP2 | 0,00173449 | 0,71515321 | protein_coding | protein coding | NA | NA |
| ENSG00000274290.2_2 | NA | NA | HIST1H2BE | 0,00174242 | 0,92712974 | protein_coding | protein coding | NA | NA |
| ENSG00000142330.19_3 | NA | NA | CAPN10 | 0,00174962 | 0,86647769 | protein_coding | protein coding | NA | NA |
| ENSG00000168026.18_3 | NA | NA | TTC21A | 0,00175981 | 0,82613898 | protein_coding | protein coding | NA | NA |
| ENSG00000117115.12_2 | NA | NA | PADI2 | 0,00182629 | 0,65351242 | protein_coding | protein coding | NA | NA |
| ENSG00000271086.5_2 | NA | NA | NAMA | 0,00186368 | 0,46993585 | lincRNA | lncRNA | NA | NA |
| ENSG00000257923.9_3 | NA | NA | CUX1 | 0,00186738 | 0,80480283 | protein_coding | protein coding | NA | NA |
| ENSG00000246790.2_3 | NA | NA | AP000977.1 | 0,0018887 | 0,52544477 | antisense_RNA | lncRNA | NA | NA |
| ENSG00000101412.12_2 | NA | NA | E2F1 | 0,00203055 | 0,75803236 | protein_coding | protein coding | NA | NA |
| ENSG00000105486.13_3 | NA | NA | LIG1 | 0,0020378 | 0,83635109 | protein_coding | protein coding | NA | NA |
| ENSG00000172478.17_4 | NA | NA | C2orf54 | 0,00203982 | 0,80670219 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000226696.5_3 | NA | NA | LENG8-AS1 | 0,00214869 | 0,74082995 | antisense_RNA | lncRNA | NA | NA |
| ENSG00000234432.4_3 | NA | NA | AC092171.3 | 0,00215451 | 0,73903378 | lincRNA | lncRNA | NA | NA |
| ENSG00000111206.12_3 | NA | NA | FOXM1 | 0,00217054 | 0,82811806 | protein_coding | protein coding | NA | NA |
| ENSG00000164828.17_4 | NA | NA | SUN1 | 0,00223712 | 0,86991379 | protein_coding | protein coding | NA | NA |
| ENSG00000106268.15_3 | NA | NA | NUDT1 | 0,00225399 | 0,74037568 | protein_coding | protein coding | NA | NA |
| ENSG00000184357.4_2 | NA | NA | HIST1H1B | 0,00225807 | 0,93062934 | protein_coding | protein coding | NA | NA |
| ENSG00000177409.11_3 | NA | NA | SAMD9L | 0,00225944 | 0,46889715 | protein_coding | protein coding | NA | NA |
| ENSG00000233237.6_3 | NA | NA | LINC00472 | 0,0022842 | -0,85568874 | lincRNA | lncRNA | NA | NA |
| ENSG00000169891.17_2 | NA | NA | REPS2 | 0,00228636 | -0,80989935 | protein_coding | protein coding | NA | NA |
| ENSG00000075218.18_2 | NA | NA | GTSE1 | 0,0023269 | 0,75220022 | protein_coding | protein coding | NA | NA |
| ENSG00000144481.16_3 | NA | NA | TRPM8 | 0,00236153 | -0,76509338 | protein_coding | protein coding | NA | NA |
| ENSG00000112062.10_3 | NA | NA | MAPK14 | 0,00238039 | 0,99129061 | protein_coding | protein coding | NA | NA |
| ENSG00000013810.18_3 | NA | NA | TACC3 | 0,00238876 | 0,77248911 | protein_coding | protein coding | NA | NA |
| ENSG00000161800.12_2 | NA | NA | RACGAP1 | 0,00243528 | 0,76063456 | protein_coding | protein coding | NA | NA |
| ENSG00000103199.13_3 | NA | NA | ZNF500 | 0,00252264 | 0,6780742 | protein_coding | protein coding | NA | NA |
| ENSG00000114805.16_3 | NA | NA | PLCH1 | 0,00257001 | 0,93002346 | protein_coding | protein coding | NA | NA |
| ENSG00000218350.1_2 | NA | NA | LYPLA1P3 | 0,00257392 | -0,89667001 | processed_pseudogene | pseudogene | NA | NA |
| ENSG00000157259.6_3 | NA | NA | GATAD1 | 0,00258351 | 0,58393491 | protein_coding | protein coding | NA | NA |
| ENSG00000141985.9_3 | NA | NA | SH3GL1 | 0,00258722 | 0,96655941 | protein_coding | protein coding | NA | NA |
| ENSG00000124181.14_3 | NA | NA | PLCG1 | 0,0026669 | 0,99340282 | protein_coding | protein coding | NA | NA |
| ENSG00000167674.14_4 | NA | NA | HDGFL2 | 0,00267598 | 1,00296584 | protein_coding | protein coding | NA | NA |
| ENSG00000197846.3 | NA | NA | HIST1H2BF | 0,002676 | 0,63077538 | protein_coding | protein coding | NA | NA |
| ENSG00000040933.15_2 | NA | NA | INPP4A | 0,00268405 | 0,84551491 | protein_coding | protein coding | NA | NA |
| ENSG00000260063.1_3 | NA | NA | AL512408.1 | 0,00271602 | 0,65919998 | antisense_RNA | lncRNA | NA | NA |
| ENSG00000196208.13_2 | NA | NA | GREB1 | 0,00273972 | -0,88487342 | protein_coding | protein coding | NA | NA |
| ENSG00000104866.10_3 | NA | NA | PPP1R37 | 0,00274498 | 0,78231645 | protein_coding | protein coding | NA | NA |
| ENSG00000196787.3_2 | NA | NA | HIST1H2AG | 0,0027487 | 0,74448212 | protein_coding | protein coding | NA | NA |
| ENSG00000205913.6_3 | NA | NA | SRRM2-AS1 | 0,0027664 | 0,67390072 | antisense_RNA | lncRNA | NA | NA |
| ENSG00000105063.18_3 | NA | NA | PPP6R1 | 0,00278666 | 0,7627532 | protein_coding | protein coding | NA | NA |
| ENSG00000118900.14_3 | NA | NA | UBN1 | 0,0027897 | 0,83920001 | protein_coding | protein coding | NA | NA |
| ENSG00000165304.7_3 | NA | NA | MELK | 0,00279158 | 0,64869867 | protein_coding | protein coding | NA | NA |
| ENSG00000188157.14_3 | NA | NA | AGRN | 0,00280832 | 0,77877008 | protein_coding | protein coding | NA | NA |
| ENSG00000142552.7_2 | NA | NA | RCN3 | 0,00280909 | 0,80439587 | protein_coding | protein coding | NA | NA |
| ENSG00000159259.7_3 | NA | NA | CHAF1B | 0,00282941 | 0,77035335 | protein_coding | protein coding | NA | NA |
| ENSG00000140521.12_3 | NA | NA | POLG | 0,00283372 | 0,86334322 | protein_coding | protein coding | NA | NA |
| ENSG00000197635.9_3 | NA | NA | DPP4 | 0,0028393 | -0,79216333 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000143630.9_2 | NA | NA | HCN3 | 0,00284387 | 0,69944661 | protein_coding | protein coding | NA | NA |
| ENSG00000211658.2_2 | NA | NA | IGLV3-27 | 0,0028872 | 0,55975869 | IG_V_gene | Ig / TcR | NA | NA |
| ENSG00000158715.5_2 | NA | NA | SLC45A3 | 0,00290734 | -0,75306206 | protein_coding | protein coding | NA | NA |
| ENSG00000111424.10_2 | NA | NA | VDR | 0,00295641 | 0,55986983 | protein_coding | protein coding | NA | NA |
| ENSG00000247373.3_4 | NA | NA | AC055713.1 | 0,00295849 | 0,52153374 | lincRNA | lncRNA | NA | NA |
| ENSG00000073050.11_3 | NA | NA | XRCC1 | 0,00304888 | 0,85025846 | protein_coding | protein coding | NA | NA |
| ENSG00000123983.13_2 | NA | NA | ACSL3 | 0,00306362 | -0,90590893 | protein_coding | protein coding | NA | NA |
| ENSG00000166508.17_3 | NA | NA | MCM7 | 0,00308765 | 0,56722409 | protein_coding | protein coding | NA | NA |
| ENSG00000211936.2 | NA | NA | IGHV4-4 | 0,00316336 | 0,58619269 | IG_V_gene | Ig / TcR | NA | NA |
| ENSG00000178814.16_3 | NA | NA | OPLAH | 0,00316683 | 0,63035297 | protein_coding | protein coding | NA | NA |
| ENSG00000047936.10_3 | NA | NA | ROS1 | 0,00322585 | 0,71348267 | protein_coding | protein coding | NA | NA |
| ENSG00000076003.4_2 | NA | NA | MCM6 | 0,00327736 | 0,77742183 | protein_coding | protein coding | NA | NA |
| ENSG00000178685.13_3 | NA | NA | PARP10 | 0,00327765 | 0,76925757 | protein_coding | protein coding | NA | NA |
| ENSG00000148541.12_4 | NA | NA | FAM13C | 0,00328755 | -0,81042242 | protein_coding | protein coding | NA | NA |
| ENSG00000164181.13_3 | NA | NA | ELOVL7 | 0,00328767 | -1,05023111 | protein_coding | protein coding | NA | NA |
| ENSG00000158062.20_4 | NA | NA | UBXN11 | 0,00331054 | 0,80882628 | protein_coding | protein coding | NA | NA |
| ENSG00000085276.17_4 | NA | NA | MECOM | 0,00331135 | 0,72517565 | protein_coding | protein coding | NA | NA |
| ENSG00000184924.5_2 | NA | NA | PTRHD1 | 0,00332736 | -0,72477559 | protein_coding | protein coding | NA | NA |
| ENSG00000178623.11_3 | NA | NA | GPR35 | 0,00338803 | 0,73267236 | protein_coding | protein coding | NA | NA |
| ENSG00000005075.15_2 | NA | NA | POLR2J | 0,00339209 | 0,71548941 | protein_coding | protein coding | NA | NA |
| ENSG00000088325.15_2 | NA | NA | TPX2 | 0,00339332 | 0,7229801 | protein_coding | protein coding | NA | NA |
| ENSG00000278828.1_2 | NA | NA | HIST1H3H | 0,00339698 | 0,67913863 | protein_coding | protein coding | NA | NA |
| ENSG00000135540.11_4 | NA | NA | NHSL1 | 0,0034317 | 0,64383779 | protein_coding | protein coding | NA | NA |
| ENSG00000105298.13_3 | NA | NA | CACTIN | 0,0034533 | 0,7559548 | protein_coding | protein coding | NA | NA |
| ENSG00000101945.16_2 | NA | NA | SUV39H1 | 0,00347753 | 0,74975996 | protein_coding | protein coding | NA | NA |
| ENSG00000167751.12_2 | NA | NA | KLK2 | 0,00350158 | -0,56570249 | protein_coding | protein coding | NA | NA |
| ENSG00000169957.9_3 | NA | NA | ZNF768 | 0,00351819 | 0,8347382 | protein_coding | protein coding | NA | NA |
| ENSG00000010292.12_3 | NA | NA | NCAPD2 | 0,0035408 | 0,90441206 | protein_coding | protein coding | NA | NA |
| ENSG00000049192.14_3 | NA | NA | ADAMTS6 | 0,00354585 | 0,99764198 | protein_coding | protein coding | NA | NA |
| ENSG00000271447.5_3 | NA | NA | MMP28 | 0,003547 | -0,81558867 | protein_coding | protein coding | NA | NA |
| ENSG00000165475.13_2 | NA | NA | CRYL1 | 0,00355085 | -0,77218189 | protein_coding | protein coding | NA | NA |
| ENSG00000185880.12_3 | NA | NA | TRIM69 | 0,00359666 | 0,66025404 | protein_coding | protein coding | NA | NA |
| ENSG00000135637.13_3 | NA | NA | CCDC142 | 0,00363855 | 0,69554959 | protein_coding | protein coding | NA | NA |
| ENSG00000076382.16_3 | NA | NA | SPAG5 | 0,00364559 | 0,75671388 | protein_coding | protein coding | NA | NA |
| ENSG00000117139.16_3 | NA | NA | KDM5B | 0,00368381 | 0,76432926 | protein_coding | protein coding | NA | NA |
| ENSG00000132017.10_3 | NA | NA | DCAF15 | 0,00369278 | 0,85182192 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000235859.5_3 | NA | NA | AC006978.1 | 0,0036999 | 0,67870661 | processed_pseudogene | pseudogene | NA | NA |
| ENSG00000130402.11_3 | NA | NA | ACTN4 | 0,00371214 | 0,70146979 | protein_coding | protein coding | NA | NA |
| ENSG00000166535.19_2 | NA | NA | A2ML1 | 0,00375182 | 0,43580681 | protein_coding | protein coding | NA | NA |
| ENSG00000101199.12_3 | NA | NA | ARFGAP1 | 0,00384535 | 0,73894061 | protein_coding | protein coding | NA | NA |
| ENSG00000183323.12_3 | NA | NA | CCDC125 | 0,00388612 | -0,60323092 | protein_coding | protein coding | NA | NA |
| ENSG00000100297.15_3 | NA | NA | MCM5 | 0,00392481 | 0,74707102 | protein_coding | protein coding | NA | NA |
| ENSG00000114737.15_2 | NA | NA | CISH | 0,0040372 | 0,78948805 | protein_coding | protein coding | NA | NA |
| ENSG00000172731.13_2 | NA | NA | LRRC20 | 0,0040402 | 0,70389285 | protein_coding | protein coding | NA | NA |
| ENSG00000276903.1_2 | NA | NA | HIST1H2AL | 0,00404428 | 0,71422909 | protein_coding | protein coding | NA | NA |
| ENSG00000101216.10_2 | NA | NA | GMEB2 | 0,00405146 | 0,81408409 | protein_coding | protein coding | NA | NA |
| ENSG00000085999.11_3 | NA | NA | RAD54L | 0,00405409 | 0,78604182 | protein_coding | protein coding | NA | NA |
| ENSG00000277075.2_2 | NA | NA | HIST1H2AE | 0,00406417 | 0,680059 | protein_coding | protein coding | NA | NA |
| ENSG00000014257.15_2 | NA | NA | ACPP | 0,00410777 | -0,89027658 | protein_coding | protein coding | NA | NA |
| ENSG00000148331.11_2 | NA | NA | ASB6 | 0,00421237 | 0,70011635 | protein_coding | protein coding | NA | NA |
| ENSG00000184897.5_2 | NA | NA | H1FX | 0,00423155 | 0,8524089 | protein_coding | protein coding | NA | NA |
| ENSG00000169738.7_3 | NA | NA | DCXR | 0,00428115 | -0,88510233 | protein_coding | protein coding | NA | NA |
| ENSG00000140525.17_4 | NA | NA | FANCI | 0,00428652 | 0,73710838 | protein_coding | protein coding | NA | NA |
| ENSG00000147536.11_2 | NA | NA | GINS4 | 0,00433453 | 0,65667641 | protein_coding | protein coding | NA | NA |
| ENSG00000106624.10_3 | NA | NA | AEBP1 | 0,00443102 | 0,68865096 | protein_coding | protein coding | NA | NA |
| ENSG00000163507.13_2 | NA | NA | KIAA1524 | 0,00444602 | 0,71069806 | protein_coding | protein coding | NA | NA |
| ENSG00000257790.1_2 | NA | NA | EIF4A1P4 | 0,00444862 | -0,71518443 | processed_pseudogene | pseudogene | NA | NA |
| ENSG00000101146.12_3 | NA | NA | RAE1 | 0,00445027 | 0,69720189 | protein_coding | protein coding | NA | NA |
| ENSG00000101160.13_2 | NA | NA | CTSZ | 0,00445247 | 0,7308364 | protein_coding | protein coding | NA | NA |
| ENSG00000163689.19_4 | NA | NA | C3orf67 | 0,00453579 | 0,73258058 | protein_coding | protein coding | NA | NA |
| ENSG00000141526.16_3 | NA | NA | SLC16A3 | 0,00455179 | 0,66516167 | protein_coding | protein coding | NA | NA |
| ENSG00000174013.7_2 | NA | NA | FBXO45 | 0,00456355 | 0,70551579 | protein_coding | protein coding | NA | NA |
| ENSG00000106346.11_2 | NA | NA | USP42 | 0,00462923 | 0,72798401 | protein_coding | protein coding | NA | NA |
| ENSG00000139714.12_2 | NA | NA | MORN3 | 0,00465701 | 0,48552337 | protein_coding | protein coding | NA | NA |
| ENSG00000109265.13_4 | NA | NA | KIAA1211 | 0,00468442 | 0,72467981 | protein_coding | protein coding | NA | NA |
| ENSG00000091527.15_2 | NA | NA | CDV3 | 0,00470761 | 0,71573728 | protein_coding | protein coding | NA | NA |
| ENSG00000173653.7_3 | NA | NA | RCE1 | 0,00471878 | 0,76994999 | protein_coding | protein coding | NA | NA |
| ENSG00000214244.4_2 | NA | NA | SETP21 | 0,00473604 | -0,8735651 | processed_pseudogene | pseudogene | NA | NA |
| ENSG00000102858.12_3 | NA | NA | MGRN1 | 0,00476473 | 0,76153499 | protein_coding | protein coding | NA | NA |
| ENSG00000268516.2_3 | NA | NA | AC020915.4 | 0,00477603 | 0,70241283 | antisense_RNA | lncRNA | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000104435.13_2 | NA | NA | STMN2 | 0,00478809 | 0,63622159 | protein_coding | protein coding | NA | NA |
| ENSG00000087301.8_3 | NA | NA | TXNDC16 | 0,00483845 | -0,89819401 | protein_coding | protein coding | NA | NA |
| ENSG00000135114.12_2 | NA | NA | OASL | 0,00485224 | 0,39203459 | protein_coding | protein coding | NA | NA |
| ENSG00000112936.18_2 | NA | NA | C7 | 0,00489963 | -0,7766094 | protein_coding | protein coding | NA | NA |
| ENSG00000196876.9 | NA | NA | SCN8A | 0,00490275 | 0,67708279 | protein_coding | protein coding | NA | NA |
| ENSG00000130816.14_3 | NA | NA | DNMT1 | 0,00492676 | 0,89277443 | protein_coding | protein coding | NA | NA |
| ENSG00000145386.9_2 | NA | NA | CCNA2 | 0,00498895 | 0,70839403 | protein_coding | protein coding | NA | NA |
| ENSG00000171451.13_3 | NA | NA | DSEL | 0,00499001 | -0,81233196 | protein_coding | protein coding | NA | NA |
| ENSG00000254470.2_2 | NA | NA | AP5B1 | 0,00501053 | 0,69111081 | protein_coding | protein coding | NA | NA |
| ENSG00000148481.13_2 | NA | NA | MINDY3 | 0,00501475 | -0,69027678 | protein_coding | protein coding | NA | NA |
| ENSG00000189139.5_2 | NA | NA | FSCB | 0,00506922 | 0,37613202 | protein_coding | protein coding | NA | NA |
| ENSG00000146540.14_3 | NA | NA | C7orf50 | 0,00507892 | 0,75107047 | protein_coding | protein coding | NA | NA |
| ENSG00000006704.10_3 | NA | NA | GTF2IRD1 | 0,00508258 | 0,72521992 | protein_coding | protein coding | NA | NA |
| ENSG00000133619.17_2 | NA | NA | KRBA1 | 0,0050919 | 0,86087003 | protein_coding | protein coding | NA | NA |
| ENSG00000168675.18_3 | NA | NA | LDLRAD4 | 0,00511991 | 0,74931856 | protein_coding | protein coding | NA | NA |
| ENSG00000143614.9_3 | NA | NA | GATAD2B | 0,00514371 | 0,74954074 | protein_coding | protein coding | NA | NA |
| ENSG00000076513.16_3 | NA | NA | ANKRD13A | 0,00514761 | 0,75128151 | protein_coding | protein coding | NA | NA |
| ENSG00000170265.11_2 | NA | NA | ZNF282 | 0,00515188 | 0,72740791 | protein_coding | protein coding | NA | NA |
| ENSG00000072694.20_3 | NA | NA | FCGR2B | 0,00517631 | 0,67018784 | protein_coding | protein coding | NA | NA |
| ENSG00000126870.15_3 | NA | NA | WDR60 | 0,0051776 | 0,73551612 | protein_coding | protein coding | NA | NA |
| ENSG00000104341.16_3 | NA | NA | LAPTM4B | 0,00519344 | 0,84630458 | protein_coding | protein coding | NA | NA |
| ENSG00000087116.14_3 | NA | NA | ADAMTS2 | 0,00526981 | 0,83690607 | protein_coding | protein coding | NA | NA |
| ENSG00000280424.1_3 | NA | NA | BX537318.2 | 0,00528505 | 0,65921023 | TEC | lncRNA | NA | NA |
| ENSG00000164631.18_2 | NA | NA | ZNF12 | 0,00529428 | 0,60182718 | protein_coding | protein coding | NA | NA |
| ENSG00000048052.21_3 | NA | NA | HDAC9 | 0,00529471 | 0,64540562 | protein_coding | protein coding | NA | NA |
| ENSG00000238058.1_3 | NA | NA | AL355574.1 | 0,00531146 | 0,5482525 | antisense_RNA | lncRNA | NA | NA |
| ENSG00000163156.11_3 | NA | NA | SCNM1 | 0,00535505 | 0,65347484 | protein_coding | protein coding | NA | NA |
| ENSG00000003400.14_4 | NA | NA | CASP10 | 0,00537187 | 0,64014164 | protein_coding | protein coding | NA | NA |
| ENSG00000180596.7_2 | NA | NA | HIST1H2BC | 0,00541121 | 0,64129282 | protein_coding | protein coding | NA | NA |
| ENSG00000142945.12_2 | NA | NA | KIF2C | 0,00542797 | 0,6988548 | protein_coding | protein coding | NA | NA |
| ENSG00000143153.12_2 | NA | NA | ATP1B1 | 0,00543261 | 0,65917142 | protein_coding | protein coding | NA | NA |
| ENSG00000239407.5_4 | NA | NA | Z68871.1 | 0,00543821 | 0,45682082 | lincRNA | lncRNA | NA | NA |
| ENSG00000167332.8_4 | NA | NA | OR51E2 | 0,00546096 | -0,7184431 | protein_coding | protein coding | NA | NA |
| ENSG00000085644.13_2 | NA | NA | ZNF213 | 0,00546453 | 0,59178905 | protein_coding | protein coding | NA | NA |
| ENSG00000260747.1_3 | NA | NA | AC022968.1 | 0,00555887 | -0,59108129 | processed_pseudogene | pseudogene | NA | NA |
| ENSG00000130309.10_4 | NA | NA | COLGALT1 | 0,00559387 | 0,74514842 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000226650.5_3 | NA | NA | KIF4B | 0,00560633 | 0,59691667 | protein_coding | protein coding | NA | NA |
| ENSG00000162775.14_2 | NA | NA | RBM15 | 0,00560703 | 0,81826466 | protein_coding | protein coding | NA | NA |
| ENSG00000096063.15_3 | NA | NA | SRPK1 | 0,00570192 | 0,76400612 | protein_coding | protein coding | NA | NA |
| ENSG00000180667.10_2 | NA | NA | YOD1 | 0,00572804 | 0,72003062 | protein_coding | protein coding | NA | NA |
| ENSG00000276368.1_2 | NA | NA | HIST1H2AJ | 0,00576016 | 0,61170053 | protein_coding | protein coding | NA | NA |
| ENSG00000103549.21_3 | NA | NA | RNF40 | 0,00578789 | 0,79978602 | protein_coding | protein coding | NA | NA |
| ENSG00000183831.6_2 | NA | NA | ANKRD45 | 0,00580703 | 0,52223889 | protein_coding | protein coding | NA | NA |
| ENSG00000143569.18_3 | NA | NA | UBAP2L | 0,0058566 | 0,70166801 | protein_coding | protein coding | NA | NA |
| ENSG00000114650.18_2 | NA | NA | SCAP | 0,00590903 | 0,54982067 | protein_coding | protein coding | NA | NA |
| ENSG00000007968.6_2 | NA | NA | E2F2 | 0,00594556 | 0,67515025 | protein_coding | protein coding | NA | NA |
| ENSG00000105612.8_2 | NA | NA | DNASE2 | 0,00597706 | 0,77311509 | protein_coding | protein coding | NA | NA |
| ENSG00000164197.11_3 | NA | NA | RNF180 | 0,00598155 | -0,6949414 | protein_coding | protein coding | NA | NA |
| ENSG00000174243.9_3 | NA | NA | DDX23 | 0,00603068 | 0,72913875 | protein_coding | protein coding | NA | NA |
| ENSG00000213918.10_3 | NA | NA | DNASE1 | 0,00603161 | 0,60671995 | protein_coding | protein coding | NA | NA |
| ENSG00000170775.2_2 | NA | NA | GPR37 | 0,00606961 | 0,72646143 | protein_coding | protein coding | NA | NA |
| ENSG00000182492.15_2 | NA | NA | BGN | 0,00607225 | 0,8004589 | protein_coding | protein coding | NA | NA |
| ENSG00000170260.8_3 | NA | NA | ZNF212 | 0,00607409 | 0,75708098 | protein_coding | protein coding | NA | NA |
| ENSG00000226174.6_4 | NA | NA | TEX22 | 0,00611113 | 0,68543966 | protein_coding | protein coding | NA | NA |
| ENSG00000072042.12_3 | NA | NA | RDH11 | 0,00611223 | -0,66202193 | protein_coding | protein coding | NA | NA |
| ENSG00000156304.14_3 | NA | NA | SCAF4 | 0,00611529 | 0,76224715 | protein_coding | protein coding | NA | NA |
| ENSG00000137804.12_3 | NA | NA | NUSAP1 | 0,00616148 | 0,72624819 | protein_coding | protein coding | NA | NA |
| ENSG00000196453.7_2 | NA | NA | ZNF777 | 0,00616386 | 0,78551016 | protein_coding | protein coding | NA | NA |
| ENSG00000133863.7_2 | NA | NA | TEX15 | 0,00620609 | 0,73452474 | protein_coding | protein coding | NA | NA |
| ENSG00000166405.14_3 | NA | NA | RIC3 | 0,00620666 | -0,69351192 | protein_coding | protein coding | NA | NA |
| ENSG00000239857.6_4 | NA | NA | GET4 | 0,00621227 | 0,68781717 | protein_coding | protein coding | NA | NA |
| ENSG00000162909.17_2 | NA | NA | CAPN2 | 0,00623359 | 0,73299248 | protein_coding | protein coding | NA | NA |
| ENSG00000104957.13_2 | NA | NA | CCDC130 | 0,00624837 | 0,74569688 | protein_coding | protein coding | NA | NA |
| ENSG00000177602.5_3 | NA | NA | HASPIN | 0,00625129 | 0,68733891 | protein_coding | protein coding | NA | NA |
| ENSG00000165943.4_2 | NA | NA | MOAP1 | 0,00629881 | -0,72436847 | protein_coding | protein coding | NA | NA |
| ENSG00000273983.1_2 | NA | NA | HIST1H3G | 0,00639926 | 0,6423454 | protein_coding | protein coding | NA | NA |
| ENSG00000120802.13_3 | NA | NA | TMPO | 0,0064147 | 0,84431684 | protein_coding | protein coding | NA | NA |
| ENSG00000225975.6_2 | NA | NA | LINC01534 | 0,00644967 | 0,60031662 | lincRNA | lncRNA | NA | NA |
| ENSG00000166987.14_3 | NA | NA | MBD6 | 0,00645421 | 0,84424502 | protein_coding | protein coding | NA | NA |
| ENSG00000124145.6_3 | NA | NA | SDC4 | 0,00647337 | 0,56801367 | protein_coding | protein coding | NA | NA |
| ENSG00000166923.10_2 | NA | NA | GREM1 | 0,00649653 | 0,68134893 | protein_coding | protein coding | NA | NA |
| ENSG00000175727.9 | NA | NA | MLXIP | 0,00650384 | 0,78381537 | protein_coding | protein coding | NA | NA |
| ENSG00000067141.16_2 | NA | NA | NEO1 | 0,00650663 | -0,79363322 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000163864.15_3 | NA | NA | NMNAT3 | 0,00651052 | 0,89353374 | protein_coding | protein coding | NA | NA |
| ENSG00000176208.8_2 | NA | NA | ATAD5 | 0,00652327 | 0,80070317 | protein_coding | protein coding | NA | NA |
| ENSG00000048471.13_3 | NA | NA | SNX29 | 0,00654557 | 0,77962508 | protein_coding | protein coding | NA | NA |
| ENSG00000131747.14_2 | NA | NA | TOP2A | 0,00655663 | 0,70397256 | protein_coding | protein coding | NA | NA |
| ENSG00000116171.17_3 | NA | NA | SCP2 | 0,00656278 | -0,78453246 | protein_coding | protein coding | NA | NA |
| ENSG00000104889.5_2 | NA | NA | RNASEH2A | 0,00667083 | 0,630166 | protein_coding | protein coding | NA | NA |
| ENSG00000228801.5_3 | NA | NA | AC064807.1 | 0,00668321 | 0,57872584 | antisense_RNA | lncRNA | NA | NA |
| ENSG00000002822.15_3 | NA | NA | MAD1L1 | 0,00671091 | 0,68337838 | protein_coding | protein coding | NA | NA |
| ENSG00000105388.15_3 | NA | NA | CEACAM5 | 0,00672776 | 0,40459311 | protein_coding | protein coding | NA | NA |
| ENSG00000182831.11_3 | NA | NA | C16orf72 | 0,00678147 | 0,72321914 | protein_coding | protein coding | NA | NA |
| ENSG00000188322.4_3 | NA | NA | SBK1 | 0,00681165 | 0,64949601 | protein_coding | protein coding | NA | NA |
| ENSG00000099364.16_3 | NA | NA | FBXL19 | 0,00683494 | 0,80143342 | protein_coding | protein coding | NA | NA |
| ENSG00000187764.11_3 | NA | NA | SEMA4D | 0,00684157 | 0,67162226 | protein_coding | protein coding | NA | NA |
| ENSG00000180900.18_3 | NA | NA | SCRIB | 0,00684167 | 0,80240127 | protein_coding | protein coding | NA | NA |
| ENSG00000254726.2_2 | NA | NA | MEX3A | 0,00685968 | 0,7129495 | protein_coding | protein coding | NA | NA |
| ENSG00000137812.19_3 | NA | NA | KNL1 | 0,00696387 | 0,67532459 | protein_coding | protein coding | NA | NA |
| ENSG00000244682.7_2 | NA | NA | FCGR2C | 0,00698828 | 0,64814663 | polymorphic_pseudogene | pseudogene | NA | NA |
| ENSG00000205413.7_3 | NA | NA | SAMD9 | 0,00705403 | 0,40946327 | protein_coding | protein coding | NA | NA |
| ENSG00000279369.1_3 | NA | NA | AC046185.3 | 0,00706012 | 0,66941823 | TEC | lncRNA | NA | NA |
| ENSG00000163755.8_2 | NA | NA | HPS3 | 0,00707463 | 0,54516145 | protein_coding | protein coding | NA | NA |
| ENSG00000149179.13_2 | NA | NA | C11orf49 | 0,00713141 | 0,68423837 | protein_coding | protein coding | NA | NA |
| ENSG00000172757.12_3 | NA | NA | CFL1 | 0,00714868 | 0,63377006 | protein_coding | protein coding | NA | NA |
| ENSG00000152527.13_3 | NA | NA | PLEKHH2 | 0,00719757 | 0,61973525 | protein_coding | protein coding | NA | NA |
| ENSG00000278463.1_2 | NA | NA | HIST1H2AB | 0,00720641 | 0,69424341 | protein_coding | protein coding | NA | NA |
| ENSG00000142409.5_2 | NA | NA | ZNF787 | 0,00726444 | 0,69051338 | protein_coding | protein coding | NA | NA |
| ENSG00000079112.9_3 | NA | NA | CDH17 | 0,00728394 | 0,5845861 | protein_coding | protein coding | NA | NA |
| ENSG00000188895.11_4 | NA | NA | MSL1 | 0,00734789 | 0,70124282 | protein_coding | protein coding | NA | NA |
| ENSG00000124151.18_3 | NA | NA | NCOA3 | 0,00734845 | 0,80282693 | protein_coding | protein coding | NA | NA |
| ENSG00000181904.8_3 | NA | NA | C5orf24 | 0,00747963 | -0,65026949 | protein_coding | protein coding | NA | NA |
| ENSG00000147364.16_2 | NA | NA | FBXO25 | 0,00748744 | -0,82087403 | protein_coding | protein coding | NA | NA |
| ENSG00000156787.16_3 | NA | NA | TBC1D31 | 0,00751176 | 0,60009549 | protein_coding | protein coding | NA | NA |
| ENSG00000141756.18_4 | NA | NA | FKBP10 | 0,00751767 | 0,55301623 | protein_coding | protein coding | NA | NA |
| ENSG00000068137.14_2 | NA | NA | PLEKHH3 | 0,00752711 | 0,67137194 | protein_coding | protein coding | NA | NA |
| ENSG00000138756.17_3 | NA | NA | BMP2K | 0,00759169 | 0,65704721 | protein_coding | protein coding | NA | NA |
| ENSG00000183778.17_3 | NA | NA | B3GALT5 | 0,00762355 | 0,52865349 | protein_coding | protein coding | NA | NA |
| ENSG00000165244.6_2 | NA | NA | ZNF367 | 0,00765759 | 0,61425894 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000071054.16_3 | NA | NA | MAP4K4 | 0,00766812 | 0,78029519 | protein_coding | protein coding | NA | NA |
| ENSG00000109472.13_2 | NA | NA | CPE | 0,00767132 | -0,68653717 | protein_coding | protein coding | NA | NA |
| ENSG00000183856.10_2 | NA | NA | IQGAP3 | 0,00768715 | 0,75469277 | protein_coding | protein coding | NA | NA |
| ENSG00000105698.15_2 | NA | NA | USF2 | 0,0077034 | 0,72651665 | protein_coding | protein coding | NA | NA |
| ENSG00000213024.11_3 | NA | NA | NUP62 | 0,00770865 | 0,75860995 | protein_coding | protein coding | NA | NA |
| ENSG00000181135.15_4 | NA | NA | ZNF707 | 0,00775207 | 0,60464366 | protein_coding | protein coding | NA | NA |
| ENSG00000111642.14_2 | NA | NA | CHD4 | 0,00775751 | 0,81565598 | protein_coding | protein coding | NA | NA |
| ENSG00000143226.13_4 | NA | NA | FCGR2A | 0,0077635 | 0,73860165 | protein_coding | protein coding | NA | NA |
| ENSG00000083812.11_3 | NA | NA | ZNF324 | 0,00776394 | 0,65425054 | protein_coding | protein coding | NA | NA |
| ENSG00000003249.13_2 | NA | NA | DBNDD1 | 0,00781023 | 0,7210908 | protein_coding | protein coding | NA | NA |
| ENSG00000071462.11_4 | NA | NA | BUD23 | 0,00783166 | 0,50239505 | protein_coding | protein coding | NA | NA |
| ENSG00000106144.19_2 | NA | NA | CASP2 | 0,00795894 | 0,90694493 | protein_coding | protein coding | NA | NA |
| ENSG00000223350.2_2 | NA | NA | IGLV9-49 | 0,00801309 | 0,50552759 | IG_V_gene | Ig / TcR | NA | NA |
| ENSG00000188064.9_3 | NA | NA | WNT7B | 0,0080259 | 0,68935319 | protein_coding | protein coding | NA | NA |
| ENSG00000214293.8_2 | NA | NA | APTR | 0,00805006 | 0,55756262 | lincRNA | lncRNA | NA | NA |
| ENSG00000262772.1_2 | NA | NA | LINC01977 | 0,00807308 | 0,48461167 | lincRNA | lncRNA | NA | NA |
| ENSG00000131381.12_3 | NA | NA | RBSN | 0,00808121 | 0,76164983 | protein_coding | protein coding | NA | NA |
| ENSG00000234814.8_3 | NA | NA | SVILP1 | 0,0081088 | 0,44580833 | transcribed_un processed_pseu dogene | pseudogene | NA | NA |
| ENSG00000161996.18_3 | NA | NA | WDR90 | 0,00816203 | 0,5621422 | protein_coding | protein coding | NA | NA |
| ENSG00000182872.15_3 | NA | NA | RBM10 | 0,00816917 | 0,74206953 | protein_coding | protein coding | NA | NA |
| ENSG00000113810.15_2 | NA | NA | SMC4 | 0,00820813 | 0,66305211 | protein_coding | protein coding | NA | NA |
| ENSG00000168014.16_4 | NA | NA | C2CD3 | 0,00821438 | 0,64244803 | protein_coding | protein coding | NA | NA |
| ENSG00000127586.16_2 | NA | NA | CHTF18 | 0,00823568 | 0,605582 | protein_coding | protein coding | NA | NA |
| ENSG00000133884.9_2 | NA | NA | DPF2 | 0,0082556 | 0,81302861 | protein_coding | protein coding | NA | NA |
| ENSG00000116954.7_2 | NA | NA | RRAGC | 0,00828588 | 0,71890827 | protein_coding | protein coding | NA | NA |
| ENSG00000136828.18_3 | NA | NA | RALGPS1 | 0,00828852 | 0,56719854 | protein_coding | protein coding | NA | NA |
| ENSG00000272168.6_3 | NA | NA | CASC15 | 0,00829912 | 0,61703248 | lincRNA | lncRNA | NA | NA |
| ENSG00000197114.11_3 | NA | NA | ZGPAT | 0,00833339 | 0,77127548 | protein_coding | protein coding | NA | NA |
| ENSG00000241058.3_3 | NA | NA | NSUN6 | 0,00834246 | -0,68431576 | protein_coding | protein coding | NA | NA |
| ENSG00000081181.7_2 | NA | NA | ARG2 | 0,00837362 | -0,70396194 | protein_coding | protein coding | NA | NA |
| ENSG00000160207.8_3 | NA | NA | HSF2BP | 0,00839222 | 0,5559067 | protein_coding | protein coding | NA | NA |
| ENSG00000138495.6_3 | NA | NA | COX17 | 0,00839522 | 0,65803731 | protein_coding | protein coding | NA | NA |
| ENSG00000179588.8_2 | NA | NA | ZFPM1 | 0,00841476 | 0,74516522 | protein_coding | protein coding | NA | NA |
| ENSG00000234616.8_3 | NA | NA | JRK | 0,00851223 | 0,64880311 | protein_coding | protein coding | NA | NA |
| ENSG00000167491.17_3 | NA | NA | GATAD2A | 0,00851649 | 0,64266606 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000173465.7_3 | NA | NA | SSSCA1 | 0,00852553 | 0,90865985 | protein_coding | protein coding | NA | NA |
| ENSG00000146063.19_2 | NA | NA | TRIM41 | 0,00856263 | 0,67204679 | protein_coding | protein coding | NA | NA |
| ENSG00000230102.7_3 | NA | NA | LINC02028 | 0,00865465 | 0,53176327 | processed_transcript | lncRNA | NA | NA |
| ENSG00000131781.12_3 | NA | NA | FMO5 | 0,00866251 | -0,91412305 | protein_coding | protein coding | NA | NA |
| ENSG0000144810.15_2 | NA | NA | COL8A1 | 0,00868143 | 0,73441907 | protein_coding | protein coding | NA | NA |
| ENSG00000215895.4_3 | NA | NA | AL354702.1 | 0,00869648 | -0,70177898 | processed_pseudogene | pseudogene | NA | NA |
| ENSG00000103550.13_4 | NA | NA | KNOP1 | 0,00870132 | 0,63047696 | protein_coding | protein coding | NA | NA |
| ENSG00000143793.12_2 | NA | NA | C1orf35 | 0,00873092 | 0,61397902 | protein_coding | protein coding | NA | NA |
| ENSG00000122641.10_3 | NA | NA | INHBA | 0,00875931 | 0,76428744 | protein_coding | protein coding | NA | NA |
| ENSG00000169604.19_3 | NA | NA | ANTXR1 | 0,00880644 | 0,70968961 | protein_coding | protein coding | NA | NA |
| ENSG00000146830.9_2 | NA | NA | GIGYF1 | 0,00882685 | 0,69918713 | protein_coding | protein coding | NA | NA |
| ENSG00000110583.12_3 | NA | NA | NAA40 | 0,0089188 | 0,67658987 | protein_coding | protein coding | NA | NA |
| ENSG00000278588.1_2 | NA | NA | HIST1H2BI | 0,0089328 | 0,58065532 | protein_coding | protein coding | NA | NA |
| ENSG00000088340.15_3 | NA | NA | FER1L4 | 0,00902712 | 0,47494442 | transcribed_unitary_pseudogene | pseudogene | NA | NA |
| ENSG00000239911.2_3 | NA | NA | PRKAG2-AS1 | 0,00912075 | 0,58960453 | antisense_RNA | lncRNA | NA | NA |
| ENSG00000250427.1_2 | NA | NA | LINC02148 | 0,00912239 | 0,63765114 | lincRNA | lncRNA | NA | NA |
| ENSG00000215447.7_3 | NA | NA | BX322557.1 | 0,00913075 | 0,68715328 | processed_transcript | lncRNA | NA | NA |
| ENSG00000107581.12_2 | NA | NA | EIF3A | 0,00913164 | -0,6226504 | protein_coding | protein coding | NA | NA |
| ENSG00000111671.9_3 | NA | NA | SPSB2 | 0,00916413 | 0,66416412 | protein_coding | protein coding | NA | NA |
| ENSG00000070814.17_3 | NA | NA | TCOF1 | 0,00918699 | 0,77794612 | protein_coding | protein coding | NA | NA |
| ENSG00000095397.13_3 | NA | NA | WHRN | 0,00928508 | 0,67652589 | protein_coding | protein coding | NA | NA |
| ENSG00000143228.12_3 | NA | NA | NUF2 | 0,00929714 | 0,65087105 | protein_coding | protein coding | NA | NA |
| ENSG00000158014.14_2 | NA | NA | SLC30A2 | 0,00930471 | 0,44985271 | protein_coding | protein coding | NA | NA |
| ENSG00000164684.13_3 | NA | NA | ZNF704 | 0,00930541 | 0,67518345 | protein_coding | protein coding | NA | NA |
| ENSG00000103540.16_3 | NA | NA | CCP110 | 0,00936624 | 0,72277398 | protein_coding | protein coding | NA | NA |
| ENSG00000006118.14_4 | NA | NA | TMEM132A | 0,0093766 | 0,73786933 | protein_coding | protein coding | NA | NA |
| ENSG00000211956.2_2 | NA | NA | IGHV4-34 | 0,0094039 | 0,53521645 | IG_V_gene | Ig / TcR | NA | NA |
| ENSG00000126001.15_2 | NA | NA | CEP250 | 0,00941588 | 0,67765673 | protein_coding | protein coding | NA | NA |
| ENSG00000198700.9_2 | NA | NA | IPO9 | 0,00942796 | 0,62750844 | protein_coding | protein coding | NA | NA |
| ENSG00000171345.13_2 | NA | NA | KRT19 | 0,00948521 | 0,6901451 | protein_coding | protein coding | NA | NA |
| ENSG00000221838.9_2 | NA | NA | AP4M1 | 0,00954467 | 0,58919679 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000077152.9_2 | NA | NA | UBE2T | 0,00955502 | 0,71956205 | protein_coding | protein coding | NA | NA |
| ENSG00000274997.1_2 | NA | NA | HIST1H2AH | 0,00957247 | 0,54346487 | protein_coding | protein coding | NA | NA |
| ENSG00000169562.9_2 | NA | NA | GJB1 | 0,00958892 | -0,66644036 | protein_coding | protein coding | NA | NA |
| ENSG00000116096.5_2 | NA | NA | SPR | 0,00960341 | -0,67577061 | protein_coding | protein coding | NA | NA |
| ENSG00000064601.16_2 | NA | NA | CTSA | 0,00961796 | 0,6153457 | protein_coding | protein coding | NA | NA |
| ENSG00000124429.17_2 | NA | NA | POF1B | 0,00966114 | 0,56155802 | protein_coding | protein coding | NA | NA |
| ENSG00000064419.13_2 | NA | NA | TNPO3 | 0,00977203 | 0,58908967 | protein_coding | protein coding | NA | NA |
| ENSG00000166479.9_3 | NA | NA | TMX3 | 0,00979454 | -0,66025697 | protein_coding | protein coding | NA | NA |
| ENSG00000011243.17_3 | NA | NA | AKAP8L | 0,0098506 | 0,69274866 | protein_coding | protein coding | NA | NA |
| ENSG00000145604.15_2 | NA | NA | SKP2 | 0,00991531 | 0,84486826 | protein_coding | protein coding | NA | NA |
| ENSG00000106993.11_2 | NA | NA | CDC37L1 | 0,00994843 | -0,69887518 | protein_coding | protein coding | NA | NA |
| ENSG00000102302.7_2 | NA | NA | FGD1 | 0,00995554 | 0,63520011 | protein_coding | protein coding | NA | NA |
| ENSG00000101189.6_2 | NA | NA | MRGBP | 0,00996969 | 0,67802406 | protein_coding | protein coding | NA | NA |
| ENSG00000261189.1_3 | NA | NA | AL031058.1 | 0,01000006 | 0,56679582 | antisense_RNA | lncRNA | NA | NA |
| ENSG00000011426.10_3 | NA | NA | ANLN | 0,01005453 | 0,66920662 | protein_coding | protein coding | NA | NA |
| ENSG00000117266.15_4 | NA | NA | CDK18 | 0,01008693 | 0,62485057 | protein_coding | protein coding | NA | NA |
| ENSG00000087460.24_3 | NA | NA | GNAS | 0,01013446 | 0,63257625 | protein_coding | protein coding | NA | NA |
| ENSG00000185347.17_4 | NA | NA | C14orf80 | 0,01014507 | 0,62369031 | protein_coding | protein coding | NA | NA |
| ENSG00000166889.13_2 | NA | NA | PATL1 | 0,0101625 | 0,70793651 | protein_coding | protein coding | NA | NA |
| ENSG00000113140.10_3 | NA | NA | SPARC | 0,01017746 | 0,7552262 | protein_coding | protein coding | NA | NA |
| ENSG00000009335.17_3 | NA | NA | UBE3C | 0,01023207 | 0,63323831 | protein_coding | protein coding | NA | NA |
| ENSG00000148308.17_2 | NA | NA | GTF3C5 | 0,0102819 | 0,65467649 | protein_coding | protein coding | NA | NA |
| ENSG00000268686.1_3 | NA | NA | AC010643.1 | 0,01029908 | 0,56627655 | antisense_RNA | lncRNA | NA | NA |
| ENSG00000179526.16_2 | NA | NA | SHARPIN | 0,01030367 | 0,5890813 | protein_coding | protein coding | NA | NA |
| ENSG00000162931.11_3 | NA | NA | TRIM17 | 0,01034691 | 0,75639709 | protein_coding | protein coding | NA | NA |
| ENSG00000047457.13_2 | NA | NA | CP | 0,01034802 | 0,62050095 | protein_coding | protein coding | NA | NA |
| ENSG00000162063.12_3 | NA | NA | CCNF | 0,01036566 | 0,56665491 | protein_coding | protein coding | NA | NA |
| ENSG00000182551.13_2 | NA | NA | ADI1 | 0,01043435 | -0,75942697 | protein_coding | protein coding | NA | NA |
| ENSG00000077514.8_3 | NA | NA | POLD3 | 0,01044054 | 0,72367416 | protein_coding | protein coding | NA | NA |
| ENSG00000127191.17_2 | NA | NA | TRAF2 | 0,01046014 | 0,68283364 | protein_coding | protein coding | NA | NA |
| ENSG00000176410.7_2 | NA | NA | DNAJC30 | 0,01053929 | 0,54003076 | protein_coding | protein coding | NA | NA |
| ENSG00000157020.17_3 | NA | NA | SEC13 | 0,01060194 | 0,64626238 | protein_coding | protein coding | NA | NA |
| ENSG00000185499.16_3 | NA | NA | MUC1 | 0,01063878 | 0,51838622 | protein_coding | protein coding | NA | MUC1 |
| ENSG00000165724.5_2 | NA | NA | ZMYND19 | 0,01065956 | 0,7221455 | protein_coding | protein coding | NA | NA |
| ENSG00000119711.12_3 | NA | NA | ALDH6A1 | 0,01070678 | -0,81730283 | protein_coding | protein coding | NA | NA |
| ENSG00000197093.10_2 | NA | NA | GAL3ST4 | 0,0107387 | 0,63781249 | protein_coding | protein coding | NA | NA |
| ENSG00000236212.1_3 | NA | NA | AC009262.1 | 0,01079282 | 0,47061883 | lincRNA | lncRNA | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000196943.12_3 | NA | NA | NOP9 | 0,01080689 | 0,66546892 | protein_coding | protein coding | NA | NA |
| ENSG00000068028.17_2 | NA | NA | RASSF1 | 0,01081678 | 0,69637936 | protein_coding | protein coding | NA | NA |
| ENSG00000100889.11_2 | NA | NA | PCK2 | 0,01084259 | 0,55737596 | protein_coding | protein coding | NA | NA |
| ENSG00000244300.2_3 | NA | NA | GATA2-AS1 | 0,01089866 | 0,58659972 | antisense_RNA | lncRNA | NA | NA |
| ENSG00000166578.9_2 | NA | NA | IQCD | 0,01092876 | 0,46688559 | protein_coding | protein coding | NA | NA |
| ENSG00000167470.12_3 | NA | NA | MIDN | 0,01093228 | 0,71568692 | protein_coding | protein coding | NA | NA |
| ENSG00000168887.10_3 | NA | NA | C2orf68 | 0,01095765 | 0,81407149 | protein_coding | protein coding | NA | NA |
| ENSG00000138642.14_3 | NA | NA | HERC6 | 0,01095836 | 0,55384442 | protein_coding | protein coding | NA | NA |
| ENSG00000103257.8_2 | NA | NA | SLC7A5 | 0,01097362 | 0,64342187 | protein_coding | protein coding | NA | NA |
| ENSG00000041353.9_3 | NA | NA | RAB27B | 0,01101355 | -0,69973537 | protein_coding | protein coding | NA | NA |
| ENSG00000168496.3_3 | NA | NA | FEN1 | 0,0111089 | 0,71882095 | protein_coding | protein coding | NA | NA |
| ENSG00000198901.13_2 | NA | NA | PRC1 | 0,01113266 | 0,68883778 | protein_coding | protein coding | NA | NA |
| ENSG00000172530.20_4 | NA | NA | BANP | 0,01115984 | 0,68266007 | protein_coding | protein coding | NA | NA |
| ENSG00000160917.14_2 | NA | NA | CPSF4 | 0,01120835 | 0,61324443 | protein_coding | protein coding | NA | NA |
| ENSG00000211952.3_2 | NA | NA | IGHV4-28 | 0,01126027 | 0,5351291 | IG_V_gene | Ig / TcR | NA | NA |
| ENSG00000005339.14_3 | NA | NA | CREBBP | 0,01128237 | 0,69778573 | protein_coding | protein coding | NA | NA |
| ENSG00000173193.13_3 | NA | NA | PARP14 | 0,01130854 | 0,57970849 | protein_coding | protein coding | NA | NA |
| ENSG00000130787.13_2 | NA | NA | HIP1R | 0,01132878 | 0,69867691 | protein_coding | protein coding | NA | NA |
| ENSG00000132680.10_3 | NA | NA | KIAA0907 | 0,01139508 | 0,68325812 | protein_coding | protein coding | NA | NA |
| ENSG00000149782.11_2 | NA | NA | PLCB3 | 0,01149484 | 0,72756235 | protein_coding | protein coding | NA | NA |
| ENSG00000134321.11_2 | NA | NA | RSAD2 | 0,01151294 | 0,38388396 | protein_coding | protein coding | NA | NA |
| ENSG00000087365.15_3 | NA | NA | SF3B2 | 0,01151631 | 0,60118645 | protein_coding | protein coding | NA | NA |
| ENSG00000143294.14_2 | NA | NA | PRCC | 0,01155804 | 0,73552376 | protein_coding | protein coding | NA | NA |
| ENSG00000123500.9_3 | NA | NA | COL10A1 | 0,01159673 | 0,66307459 | protein_coding | protein coding | NA | NA |
| ENSG00000101150.17_3 | NA | NA | TPD52L2 | 0,01160378 | 0,65247133 | protein_coding | protein coding | NA | NA |
| ENSG00000028310.17_3 | NA | NA | BRD9 | 0,01161754 | 0,66095199 | protein_coding | protein coding | NA | NA |
| ENSG00000169231.13_2 | NA | NA | THBS3 | 0,01163756 | 0,66825918 | protein_coding | protein coding | NA | NA |
| ENSG00000158710.14_2 | NA | NA | TAGLN2 | 0,01164128 | 0,64873798 | protein_coding | protein coding | NA | NA |
| ENSG00000117724.12_2 | NA | NA | CENPF | 0,011664 | 0,63709503 | protein_coding | protein coding | NA | NA |
| ENSG00000113448.18_3 | NA | NA | PDE4D | 0,01167126 | -0,73959138 | protein_coding | protein coding | NA | NA |
| ENSG00000242622.1_3 | NA | NA | AC092910.3 | 0,01167797 | 0,54247427 | lincRNA | lncRNA | NA | NA |
| ENSG00000173702.7_4 | NA | NA | MUC13 | 0,01169595 | 0,46280486 | protein_coding | protein coding | NA | NA |
| ENSG00000066422.4_2 | NA | NA | ZBTB11 | 0,01171617 | 0,68817094 | protein_coding | protein coding | NA | NA |
| ENSG00000215807.4_2 | NA | NA | KRT18P65 | 0,01171888 | -0,7445129 | processed_pseudogene | pseudogene | NA | NA |
| ENSG00000149269.9_3 | NA | NA | PAK1 | 0,01172178 | 0,63067102 | protein_coding | protein coding | NA | NA |
| ENSG00000134343.12_3 | NA | NA | ANO3 | 0,01172846 | 0,480181 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000167394.12_2 | NA | NA | ZNF668 | 0,01179446 | 0,74849117 | protein_coding | protein coding | NA | NA |
| ENSG00000110492.15_4 | NA | NA | MDK | 0,01179897 | 0,63255907 | protein_coding | protein coding | NA | NA |
| ENSG00000146039.10_2 | NA | NA | SLC17A4 | 0,01182543 | 0,56669039 | protein_coding | protein coding | NA | NA |
| ENSG00000136490.8_2 | NA | NA | LIMD2 | 0,01184049 | 0,74166873 | protein_coding | protein coding | NA | NA |
| ENSG00000175305.17_3 | NA | NA | CCNE2 | 0,0118812 | 0,7131421 | protein_coding | protein coding | NA | NA |
| ENSG00000184661.13_2 | NA | NA | CDCA2 | 0,01188388 | 0,54526357 | protein_coding | protein coding | NA | NA |
| ENSG00000196670.13_3 | NA | NA | ZFP62 | 0,01191116 | 0,73769526 | protein_coding | protein coding | NA | NA |
| ENSG00000185024.16_3 | NA | NA | BRF1 | 0,01192608 | 0,63043027 | protein_coding | protein coding | NA | NA |
| ENSG00000225937.1_3 | NA | NA | PCA3 | 0,01193377 | -0,70818105 | antisense_RNA | lncRNA | NA | NA |
| ENSG00000133466.13_2 | NA | NA | C1QTNF6 | 0,01194804 | 0,50413918 | protein_coding | protein coding | NA | NA |
| ENSG00000181036.13_2 | NA | NA | FCRL6 | 0,01197963 | 0,65023644 | protein_coding | protein coding | NA | NA |
| ENSG00000105948.13_2 | NA | NA | TTC26 | 0,01198665 | 0,56897941 | protein_coding | protein coding | NA | NA |
| ENSG00000183935.5_2 | NA | NA | HTR7P1 | 0,01205782 | 0,50558102 | transcribed_processed_pseudogene | pseudogene | NA | NA |
| ENSG00000159216.18_3 | NA | NA | RUNX1 | 0,01222787 | 0,70739425 | protein_coding | protein coding | NA | NA |
| ENSG00000180233.10_3 | NA | NA | ZNRF2 | 0,01225014 | 0,6499055 | protein_coding | protein coding | NA | NA |
| ENSG00000109685.17_3 | NA | NA | NSD2 | 0,01228054 | 0,51541239 | protein_coding | protein coding | NA | NA |
| ENSG00000120903.12_3 | NA | NA | CHRNA2 | 0,01228097 | -0,71050224 | protein_coding | protein coding | NA | NA |
| ENSG00000117399.13_2 | NA | NA | CDC20 | 0,01238595 | 0,70222918 | protein_coding | protein coding | NA | NA |
| ENSG00000173120.14_2 | NA | NA | KDM2A | 0,01239209 | 0,54360668 | protein_coding | protein coding | NA | NA |
| ENSG00000248367.1_3 | NA | NA | AC008610.1 | 0,01239337 | 0,50077911 | antisense_RNA | lncRNA | NA | NA |
| ENSG00000168795.4_2 | NA | NA | ZBTB5 | 0,0124072 | 0,47460516 | protein_coding | protein coding | NA | NA |
| ENSG00000160991.15_2 | NA | NA | ORAI2 | 0,01242361 | 0,70237283 | protein_coding | protein coding | NA | NA |
| ENSG00000130684.13_2 | NA | NA | ZNF337 | 0,01242444 | 0,7076635 | protein_coding | protein coding | NA | NA |
| ENSG00000137103.17_3 | NA | NA | TMEM8B | 0,01243271 | 0,39954848 | protein_coding | protein coding | NA | NA |
| ENSG00000142149.8_3 | NA | NA | HUNK | 0,01244213 | 0,6072494 | protein_coding | protein coding | NA | NA |
| ENSG00000070476.14_2 | NA | NA | ZXDC | 0,01245345 | 0,68964659 | protein_coding | protein coding | NA | NA |
| ENSG00000104825.16_2 | NA | NA | NFKBIB | 0,01247454 | 0,57723646 | protein_coding | protein coding | NA | NA |
| ENSG00000176182.5_2 | NA | NA | MYPOP | 0,01254589 | 0,71732828 | protein_coding | protein coding | NA | NA |
| ENSG00000139211.6_2 | NA | NA | AMIGO2 | 0,01259465 | 0,64197396 | protein_coding | protein coding | NA | NA |
| ENSG00000143398.19_3 | NA | NA | PIP5K1A | 0,01265509 | 0,65964107 | protein_coding | protein coding | NA | NA |
| ENSG00000166529.14_2 | NA | NA | ZSCAN21 | 0,01267692 | 0,51954108 | protein_coding | protein coding | NA | NA |
| ENSG00000137185.11_2 | NA | NA | ZSCAN9 | 0,01269085 | 0,64177788 | protein_coding | protein coding | NA | NA |
| ENSG00000167996.15_3 | NA | NA | FTH1 | 0,01272067 | 0,69759787 | protein_coding | protein coding | NA | NA |
| ENSG00000172987.12_3 | NA | NA | HPSE2 | 0,01277461 | -0,91081138 | protein_coding | protein coding | NA | NA |
| ENSG00000138180.15_2 | NA | NA | CEP55 | 0,01278527 | 0,5947136 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000230918.1_3 | NA | NA | AC008063.1 | 0,01279083 | -0,73897286 | antisense_RNA | lncRNA | NA | NA |
| ENSG00000124508.16_3 | NA | NA | BTN2A2 | 0,01282083 | 0,64191387 | protein_coding | protein coding | NA | NA |
| ENSG00000173950.15_3 | NA | NA | XXYLT1 | 0,01284998 | 0,69347156 | protein_coding | protein coding | NA | NA |
| ENSG00000197136.4_3 | NA | NA | PCNX3 | 0,01290462 | 0,69434658 | protein_coding | protein coding | NA | NA |
| ENSG00000149554.12_2 | NA | NA | CHEK1 | 0,01293473 | 0,7003543 | protein_coding | protein coding | NA | NA |
| ENSG00000101986.11_2 | NA | NA | ABCD1 | 0,0130551 | 0,60212655 | protein_coding | protein coding | NA | NA |
| ENSG00000095906.16_3 | NA | NA | NUBP2 | 0,01306309 | 0,63786821 | protein_coding | protein coding | NA | NA |
| ENSG00000213693.4_2 | NA | NA | SEC14L1P1 | 0,01316821 | 0,60571772 | processed_pseudogene | pseudogene | NA | NA |
| ENSG00000274267.1_2 | NA | NA | HIST1H3B | 0,01317207 | 0,59413022 | protein_coding | protein coding | NA | NA |
| ENSG00000011132.11_3 | NA | NA | APBA3 | 0,01318899 | 0,76179282 | protein_coding | protein coding | NA | NA |
| ENSG00000158301.18_3 | NA | NA | GPRASP2 | 0,01319599 | -0,68469677 | protein_coding | protein coding | NA | NA |
| ENSG00000111145.7_3 | NA | NA | ELK3 | 0,01325256 | 0,68991384 | protein_coding | protein coding | NA | NA |
| ENSG00000139044.10_3 | NA | NA | B4GALNT3 | 0,01325973 | 0,74113091 | protein_coding | protein coding | NA | NA |
| ENSG00000100911.15_3 | NA | NA | PSME2 | 0,01329833 | 0,57838754 | protein_coding | protein coding | NA | NA |
| ENSG00000175376.8_2 | NA | NA | EIF1AD | 0,01330766 | 0,49580301 | protein_coding | protein coding | NA | NA |
| ENSG00000049541.10_3 | NA | NA | RFC2 | 0,01332577 | 0,6471473 | protein_coding | protein coding | NA | NA |
| ENSG00000106462.10_3 | NA | NA | EZH2 | 0,01333789 | 0,57824031 | protein_coding | protein coding | NA | NA |
| ENSG00000147799.11_2 | NA | NA | ARHGAP39 | 0,01337987 | 0,73668279 | protein_coding | protein coding | NA | NA |
| ENSG00000178951.8_3 | NA | NA | ZBTB7A | 0,01338062 | 0,668084 | protein_coding | protein coding | NA | NA |
| ENSG00000172167.7_3 | NA | NA | MTBP | 0,01341891 | 0,57517385 | protein_coding | protein coding | NA | NA |
| ENSG00000102547.18_2 | NA | NA | CAB39L | 0,01343524 | -0,71490795 | protein_coding | protein coding | NA | NA |
| ENSG00000167395.10_3 | NA | NA | ZNF646 | 0,01346027 | 0,73398281 | protein_coding | protein coding | NA | NA |
| ENSG00000102882.11_3 | NA | NA | MAPK3 | 0,01354995 | 0,64648292 | protein_coding | protein coding | NA | NA |
| ENSG00000240216.7_3 | NA | NA | CPHL1P | 0,01356482 | 0,46742062 | transcribed_unitary_pseudogene | pseudogene | NA | NA |
| ENSG00000162032.15_3 | NA | NA | SPSB3 | 0,01357995 | 0,60153706 | protein_coding | protein coding | NA | NA |
| ENSG00000253320.5_4 | NA | NA | AZIN1-AS1 | 0,01362734 | 0,57110489 | antisense_RNA | lncRNA | NA | NA |
| ENSG00000111319.12_3 | NA | NA | SCNN1A | 0,01364232 | 0,66019146 | protein_coding | protein coding | NA | NA |
| ENSG00000152669.8_2 | NA | NA | CCNO | 0,01366595 | 0,55174333 | protein_coding | protein coding | NA | NA |
| ENSG00000135899.17_3 | NA | NA | SP110 | 0,01367048 | 0,48820189 | protein_coding | protein coding | NA | NA |
| ENSG00000127947.15_2 | NA | NA | PTPN12 | 0,0137175 | 0,53167614 | protein_coding | protein coding | NA | NA |
| ENSG00000120697.8_3 | NA | NA | ALG5 | 0,01372201 | -0,66205881 | protein_coding | protein coding | NA | NA |
| ENSG00000211637.2_2 | NA | NA | IGLV4-69 | 0,01376761 | 0,31646192 | IG_V_gene | Ig / TcR | NA | NA |
| ENSG00000161202.17_2 | NA | NA | DVL3 | 0,01379383 | 0,6404853 | protein_coding | protein coding | NA | NA |
| ENSG00000161888.11_3 | NA | NA | SPC24 | 0,01384808 | 0,62223368 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000198369.9_3 | NA | NA | SPRED2 | 0,01388639 | 0,62159764 | protein_coding | protein coding | NA | NA |
| ENSG00000080189.14_2 | NA | NA | SLC35C2 | 0,01390008 | 0,6427984 | protein_coding | protein coding | NA | NA |
| ENSG00000135956.8_2 | NA | NA | TMEM127 | 0,01392866 | 0,63862878 | protein_coding | protein coding | NA | NA |
| ENSG00000139436.20_3 | NA | NA | GIT2 | 0,01397872 | 0,68336111 | protein_coding | protein coding | NA | NA |
| ENSG00000148773.13_3 | NA | NA | MKI67 | 0,01399042 | 0,73520329 | protein_coding | protein coding | NA | NA |
| ENSG00000099954.18_3 | NA | NA | CECR2 | 0,01399187 | -0,62376177 | protein_coding | protein coding | NA | NA |
| ENSG00000167461.11_3 | NA | NA | RAB8A | 0,0141098 | 0,70913543 | protein_coding | protein coding | NA | NA |
| ENSG00000013583.8_3 | NA | NA | HEBP1 | 0,01412902 | 0,48559596 | protein_coding | protein coding | NA | NA |
| ENSG00000135250.16_3 | NA | NA | SRPK2 | 0,01414753 | 0,6419536 | protein_coding | protein coding | NA | NA |
| ENSG00000142252.10_3 | NA | NA | GEMIN7 | 0,01419199 | 0,75122519 | protein_coding | protein coding | NA | NA |
| ENSG00000135063.17_3 | NA | NA | FAM189A2 | 0,01419388 | -0,74239538 | protein_coding | protein coding | NA | NA |
| ENSG00000082701.14_4 | NA | NA | GSK3B | 0,01419883 | 0,68851244 | protein_coding | protein coding | NA | NA |
| ENSG00000215915.9_3 | NA | NA | ATAD3C | 0,01422577 | 0,54540741 | protein_coding | protein coding | NA | NA |
| ENSG00000163590.13_2 | NA | NA | PPM1L | 0,01424799 | 0,73540866 | protein_coding | protein coding | NA | NA |
| ENSG00000142453.11_3 | NA | NA | CARM1 | 0,01427986 | 0,72793331 | protein_coding | protein coding | NA | NA |
| ENSG00000171877.20_3 | NA | NA | FRMD5 | 0,01428514 | 0,58156692 | protein_coding | protein coding | NA | NA |
| ENSG00000183763.8_2 | NA | NA | TRAIP | 0,01431678 | 0,71043837 | protein_coding | protein coding | NA | NA |
| ENSG00000166851.14_3 | NA | NA | PLK1 | 0,01435128 | 0,63295463 | protein_coding | protein coding | NA | NA |
| ENSG00000151458.11_2 | NA | NA | ANKRD50 | 0,01438888 | 0,708942 | protein_coding | protein coding | NA | NA |
| ENSG00000211959.2_3 | NA | NA | IGHV4-39 | 0,01443497 | 0,56417841 | IG_V_gene | Ig / TcR | NA | NA |
| ENSG00000104147.8_2 | NA | NA | OIP5 | 0,01448275 | 0,57358028 | protein_coding | protein coding | NA | NA |
| ENSG00000213901.10_4 | NA | NA | SLC23A3 | 0,01449048 | 0,48909727 | protein_coding | protein coding | NA | NA |
| ENSG00000113328.18_2 | NA | NA | CCNG1 | 0,01450744 | -0,57761383 | protein_coding | protein coding | NA | NA |
| ENSG00000132792.18_2 | NA | NA | CTNNBL1 | 0,01458523 | 0,50864222 | protein_coding | protein coding | NA | NA |
| ENSG00000112877.7_2 | NA | NA | CEP72 | 0,01459067 | 0,65118806 | protein_coding | protein coding | NA | NA |
| ENSG00000211965.4_3 | NA | NA | IGHV3-49 | 0,01463299 | 0,52848671 | IG_V_gene | Ig / TcR | NA | NA |
| ENSG00000149600.11_2 | NA | NA | COMMD7 | 0,01470687 | 0,62086764 | protein_coding | protein coding | NA | NA |
| ENSG00000197024.8_2 | NA | NA | ZNF398 | 0,01472845 | 0,60538102 | protein_coding | protein coding | NA | NA |
| ENSG00000185684.13_3 | NA | NA | EP400NL | 0,01473991 | 0,63811709 | transcribed_unprocessed_pseudogene | pseudogene | NA | NA |
| ENSG00000185650.9_3 | NA | NA | ZFP36L1 | 0,01476669 | 0,73591473 | protein_coding | protein coding | NA | NA |
| ENSG00000102225.15_3 | NA | NA | CDK16 | 0,01477876 | 0,66399207 | protein_coding | protein coding | NA | NA |
| ENSG00000148297.15_3 | NA | NA | MED22 | 0,01493233 | 0,66695237 | protein_coding | protein coding | NA | NA |
| ENSG00000145916.18_2 | NA | NA | RMND5B | 0,01496082 | 0,7115739 | protein_coding | protein coding | NA | NA |
| ENSG00000240567.1_3 | NA | NA | LINC02067 | 0,01501619 | 0,55817512 | lincRNA | lncRNA | NA | NA |
| ENSG00000239569.3_4 | NA | NA | KMT2E-AS1 | 0,0150566 | 0,51254403 | antisense_RNA | lncRNA | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000068654.15_2 | NA | NA | POLR1A | 0,01519863 | 0,65652913 | protein_coding | protein coding | NA | NA |
| ENSG00000168005.8_2 | NA | NA | C11orf84 | 0,01521699 | 0,65169571 | protein_coding | protein coding | NA | NA |
| ENSG00000187257.15_4 | NA | NA | RSBN1L | 0,01524284 | 0,53842883 | protein_coding | protein coding | NA | NA |
| ENSG00000233930.3_3 | NA | NA | KRTAP5-AS1 | 0,015269 | 0,53499038 | antisense_RNA | lncRNA | NA | NA |
| ENSG00000173110.7_3 | NA | NA | HSPA6 | 0,01527018 | 0,42732121 | protein_coding | protein coding | NA | NA |
| ENSG00000154263.17_3 | NA | NA | ABCA10 | 0,01530531 | -0,71297119 | protein_coding | protein coding | NA | NA |
| ENSG00000114346.13_2 | NA | NA | ECT2 | 0,01535994 | 0,59005818 | protein_coding | protein coding | NA | NA |
| ENSG00000140577.15_3 | NA | NA | CRTC3 | 0,01537657 | 0,75671304 | protein_coding | protein coding | NA | NA |
| ENSG00000137965.10_2 | NA | NA | IFI44 | 0,0154033 | 0,42632785 | protein_coding | protein coding | NA | NA |
| ENSG00000130758.7_3 | NA | NA | MAP3K10 | 0,01547218 | 0,66038062 | protein_coding | protein coding | NA | NA |
| ENSG00000272391.5_4 | NA | NA | POM121C | 0,01550812 | 0,7118826 | protein_coding | protein coding | NA | NA |
| ENSG00000105576.15_4 | NA | NA | TNPO2 | 0,01551373 | 0,71239131 | protein_coding | protein coding | NA | NA |
| ENSG00000155903.11_3 | NA | NA | RASA2 | 0,01553513 | 0,62071264 | protein_coding | protein coding | NA | NA |
| ENSG00000119185.12_2 | NA | NA | ITGB1BP1 | 0,01555738 | 0,69840544 | protein_coding | protein coding | NA | NA |
| ENSG00000239213.5_3 | NA | NA | NCK1-AS1 | 0,01561078 | 0,65705909 | antisense_RNA | lncRNA | NA | NA |
| ENSG00000227531.1_3 | NA | NA | AL162414.1 | 0,01574098 | 0,60974602 | lincRNA | lncRNA | NA | NA |
| ENSG00000137809.16_3 | NA | NA | ITGA11 | 0,01578766 | 0,62615385 | protein_coding | protein coding | NA | NA |
| ENSG00000073910.20_4 | NA | NA | FRY | 0,01582214 | 0,59739537 | protein_coding | protein coding | NA | NA |
| ENSG00000263072.6_3 | NA | NA | ZNF213-AS1 | 0,01590049 | 0,46363085 | antisense_RNA | lncRNA | NA | NA |
| ENSG00000071051.13_3 | NA | NA | NCK2 | 0,01595329 | 0,56582742 | protein_coding | protein coding | NA | NA |
| ENSG00000174586.10_3 | NA | NA | ZNF497 | 0,01595349 | 0,58868159 | protein_coding | protein coding | NA | NA |
| ENSG00000134086.7_2 | NA | NA | VHL | 0,01602522 | 0,71349626 | protein_coding | protein coding | NA | NA |
| ENSG00000184675.9_2 | NA | NA | AMER1 | 0,01605564 | 0,63016009 | protein_coding | protein coding | NA | NA |
| ENSG00000106809.10_2 | NA | NA | OGN | 0,01613622 | -0,62153059 | protein_coding | protein coding | NA | NA |
| ENSG00000148219.16_3 | NA | NA | ASTN2 | 0,01614251 | -0,66782705 | protein_coding | protein coding | NA | NA |
| ENSG00000188986.6_2 | NA | NA | NELFB | 0,01621449 | 0,6112958 | protein_coding | protein coding | NA | NA |
| ENSG00000116213.15_2 | NA | NA | WRAP73 | 0,01622024 | 0,53980626 | protein_coding | protein coding | NA | NA |
| ENSG00000141696.12_4 | NA | NA | P3H4 | 0,01625328 | 0,5240871 | protein_coding | protein coding | NA | NA |
| ENSG00000088038.17_3 | NA | NA | CNOT3 | 0,01626705 | 0,66098601 | protein_coding | protein coding | NA | NA |
| ENSG00000162849.15_3 | NA | NA | KIF26B | 0,01626862 | 0,53050732 | protein_coding | protein coding | NA | NA |
| ENSG00000146233.7_2 | NA | NA | CYP39A1 | 0,01627021 | -0,65246877 | protein_coding | protein coding | NA | NA |
| ENSG00000179583.18_3 | NA | NA | CIITA | 0,01627761 | 0,68187903 | protein_coding | protein coding | NA | NA |
| ENSG00000078246.16_3 | NA | NA | TULP3 | 0,01630731 | 0,65277163 | protein_coding | protein coding | NA | NA |
| ENSG00000139154.14_4 | NA | NA | AEBP2 | 0,01633792 | 0,4775039 | protein_coding | protein coding | NA | NA |
| ENSG00000026950.16_3 | NA | NA | BTN3A1 | 0,01634493 | 0,58617767 | protein_coding | protein coding | NA | NA |
| ENSG00000248932.5_4 | NA | NA | AC097103.2 | 0,01638687 | 0,54230575 | antisense_RNA | lncRNA | NA | NA |
| ENSG00000164983.7_2 | NA | NA | TMEM65 | 0,01641511 | 0,62221999 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000225643.1_3 | NA | NA | AL606491.1 | 0,01643844 | 0,60572027 | lincRNA | lncRNA | NA | NA |
| ENSG00000133216.16_3 | NA | NA | EPHB2 | 0,01653517 | 0,62021031 | protein_coding | protein coding | NA | NA |
| ENSG00000174669.11_2 | NA | NA | SLC29A2 | 0,0165943 | 0,59334997 | protein_coding | protein coding | NA | NA |
| ENSG00000033100.16_3 | NA | NA | CHPF2 | 0,01669807 | 0,62670021 | protein_coding | protein coding | NA | NA |
| ENSG00000143476.17_2 | NA | NA | DTL | 0,0167152 | 0,61643718 | protein_coding | protein coding | NA | NA |
| ENSG00000033050.7_2 | NA | NA | ABCF2 | 0,01671957 | 0,61196851 | protein_coding | protein coding | NA | NA |
| ENSG00000148680.15_2 | NA | NA | HTR7 | 0,01673816 | 0,50303051 | protein_coding | protein coding | NA | NA |
| ENSG00000103056.11_3 | NA | NA | SMPD3 | 0,01675748 | 0,47535063 | protein_coding | protein coding | NA | NA |
| ENSG00000232445.1_3 | NA | NA | AC006329.1 | 0,01677901 | 0,57593159 | antisense_RNA | lncRNA | NA | NA |
| ENSG00000134490.13_3 | NA | NA | TMEM241 | 0,01682557 | 0,71391686 | protein_coding | protein coding | NA | NA |
| ENSG00000139055.6_2 | NA | NA | ERP27 | 0,01688473 | 0,37239848 | protein_coding | protein coding | NA | NA |
| ENSG00000166016.5_3 | NA | NA | ABTB2 | 0,01689563 | 0,58074385 | protein_coding | protein coding | NA | NA |
| ENSG00000137747.15_3 | NA | NA | TMPRSS13 | 0,01690811 | 0,60602193 | protein_coding | protein coding | NA | NA |
| ENSG00000178163.7_2 | NA | NA | ZNF518B | 0,01692119 | 0,56809907 | protein_coding | protein coding | NA | NA |
| ENSG00000104081.13_3 | NA | NA | BMF | 0,01696346 | 0,68882382 | protein_coding | protein coding | NA | NA |
| ENSG00000172594.12_2 | NA | NA | SMPDL3A | 0,01696967 | -0,8029396 | protein_coding | protein coding | NA | NA |
| ENSG00000170632.13_3 | NA | NA | ARMC10 | 0,01711504 | 0,59342996 | protein_coding | protein coding | NA | NA |
| ENSG00000183258.11_3 | NA | NA | DDX41 | 0,01720142 | 0,61914509 | protein_coding | protein coding | NA | NA |
| ENSG00000158373.8_3 | NA | NA | HIST1H2BD | 0,01726047 | 0,56510998 | protein_coding | protein coding | NA | NA |
| ENSG00000127616.17_3 | NA | NA | SMARCA4 | 0,01728241 | 0,79794008 | protein_coding | protein coding | NA | NA |
| ENSG00000058799.14_2 | NA | NA | YIPF1 | 0,01728319 | -0,66020412 | protein_coding | protein coding | NA | NA |
| ENSG00000074803.17_3 | NA | NA | SLC12A1 | 0,01732309 | 0,40052453 | protein_coding | protein coding | NA | NA |
| ENSG00000175287.18_3 | NA | NA | PHYHD1 | 0,01732828 | -0,91741105 | protein_coding | protein coding | NA | NA |
| ENSG00000198513.11_2 | NA | NA | ATL1 | 0,01734502 | -0,76913088 | protein_coding | protein coding | NA | NA |
| ENSG00000165355.7_3 | NA | NA | FBXO33 | 0,01737692 | -0,5362502 | protein_coding | protein coding | NA | NA |
| ENSG00000138829.11_3 | NA | NA | FBN2 | 0,01744693 | 0,4162346 | protein_coding | protein coding | NA | NA |
| ENSG00000123154.11_3 | NA | NA | WDR83 | 0,01756642 | 0,47256543 | protein_coding | protein coding | NA | NA |
| ENSG00000134815.18_2 | NA | NA | DHX34 | 0,01761621 | 0,62623267 | protein_coding | protein coding | NA | NA |
| ENSG00000103335.21_3 | NA | NA | PIEZO1 | 0,01764608 | 0,68683533 | protein_coding | protein coding | NA | NA |
| ENSG00000160949.16_2 | NA | NA | TONSL | 0,01767192 | 0,70884373 | protein_coding | protein coding | NA | NA |
| ENSG00000164754.14_3 | NA | NA | RAD21 | 0,01768444 | 0,71664805 | protein_coding | protein coding | NA | NA |
| ENSG00000163249.11_3 | NA | NA | CCNYL1 | 0,01768513 | 0,57652038 | protein_coding | protein coding | NA | NA |
| ENSG00000072062.13_2 | NA | NA | PRKACA | 0,01771025 | 0,62544306 | protein_coding | protein coding | NA | NA |
| ENSG00000099814.15_2 | NA | NA | CEP170B | 0,0177797 | 0,58857007 | protein_coding | protein coding | NA | NA |
| ENSG00000146411.5_2 | NA | NA | SLC2A12 | 0,01788532 | -0,68012066 | protein_coding | protein coding | NA | NA |
| ENSG00000148655.14_4 | NA | NA | LRMDA | 0,0178906 | -0,65279444 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000197674.6_3 | NA | NA | OR51C1P | 0,01791642 | -0,70763418 | transcribed_unprocessed_pseudogene | pseudogene | NA | NA |
| ENSG00000064995.16_2 | NA | NA | TAF11 | 0,01797792 | 0,61239356 | protein_coding | protein coding | NA | NA |
| ENSG00000260233.3_2 | NA | NA | SSSCA1-AS1 | 0,01798711 | 0,5821517 | processed_transcript | lncRNA | NA | NA |
| ENSG00000166483.10_2 | NA | NA | WEE1 | 0,01802165 | 0,6479873 | protein_coding | protein coding | NA | NA |
| ENSG00000176619.12_3 | NA | NA | LMNB2 | 0,01808737 | 0,70905113 | protein_coding | protein coding | NA | NA |
| ENSG00000176855.11_2 | NA | NA | KRT18P28 | 0,01810069 | -0,66043777 | processed_pseudogene | pseudogene | NA | NA |
| ENSG00000135245.9_3 | NA | NA | HILPDA | 0,018376 | 0,52167531 | protein_coding | protein coding | NA | NA |
| ENSG00000206417.8_3 | NA | NA | H1FX-AS1 | 0,01841645 | 0,55583075 | antisense_RNA | lncRNA | NA | NA |
| ENSG00000235530.6_3 | NA | NA | AC087294.1 | 0,01846915 | 0,55208022 | antisense_RNA | lncRNA | NA | NA |
| ENSG00000108370.16_3 | NA | NA | RGS9 | 0,01848429 | 0,60129785 | protein_coding | protein coding | NA | NA |
| ENSG00000110427.14_3 | NA | NA | KIAA1549L | 0,01851653 | 0,6256169 | protein_coding | protein coding | NA | NA |
| ENSG00000132970.12_3 | NA | NA | WASF3 | 0,01854483 | -0,603406 | protein_coding | protein coding | NA | NA |
| ENSG00000122687.17_3 | NA | NA | MRM2 | 0,01861102 | 0,55978462 | protein_coding | protein coding | NA | NA |
| ENSG00000070756.15_3 | NA | NA | PABPC1 | 0,01862597 | 0,53175666 | protein_coding | protein coding | NA | NA |
| ENSG00000124357.12_2 | NA | NA | NAGK | 0,01871696 | 0,70244603 | protein_coding | protein coding | NA | NA |
| ENSG00000135622.12_2 | NA | NA | SEMA4F | 0,01879502 | 0,63432032 | protein_coding | protein coding | NA | NA |
| ENSG00000172432.18_3 | NA | NA | GTPBP2 | 0,01881736 | 0,66155335 | protein_coding | protein coding | NA | NA |
| ENSG00000084731.14_3 | NA | NA | KIF3C | 0,01883206 | 0,72204959 | protein_coding | protein coding | NA | NA |
| ENSG00000165629.19_2 | NA | NA | ATP5C1 | 0,01883533 | -0,58203119 | protein_coding | protein coding | NA | NA |
| ENSG00000134709.10_2 | NA | NA | HOOK1 | 0,01887826 | -0,60740585 | protein_coding | protein coding | NA | NA |
| ENSG00000227718.1_2 | NA | NA | AC016730.1 | 0,01887835 | -0,86913169 | lincRNA | lncRNA | NA | NA |
| ENSG00000133107.14_2 | NA | NA | TRPC4 | 0,01894506 | -0,67333053 | protein_coding | protein coding | NA | NA |
| ENSG00000110274.15_3 | NA | NA | CEP164 | 0,01896965 | 0,64813767 | protein_coding | protein coding | NA | NA |
| ENSG00000068489.12_3 | NA | NA | PRR11 | 0,01917399 | 0,71311068 | protein_coding | protein coding | NA | NA |
| ENSG00000171940.13_3 | NA | NA | ZNF217 | 0,01920501 | 0,56681164 | protein_coding | protein coding | NA | NA |
| ENSG00000170852.10_3 | NA | NA | KBTBD2 | 0,01920694 | 0,6623616 | protein_coding | protein coding | NA | NA |
| ENSG00000253405.1_3 | NA | NA | EVX1-AS | 0,01920939 | 0,58795712 | antisense_RNA | lncRNA | NA | NA |
| ENSG00000260658.5_4 | NA | NA | AC138305.1 | 0,01921106 | -0,73540899 | lincRNA | lncRNA | NA | NA |
| ENSG00000113594.9_2 | NA | NA | LIFR | 0,01924023 | -0,67191458 | protein_coding | protein coding | NA | NA |
| ENSG00000136986.9_2 | NA | NA | DERL1 | 0,01927098 | 0,63009058 | protein_coding | protein coding | NA | NA |
| ENSG00000089127.12_3 | NA | NA | OAS1 | 0,01931923 | 0,40972016 | protein_coding | protein coding | NA | NA |
| ENSG00000176953.12_4 | NA | NA | NFATC2IP | 0,01933296 | 0,71075546 | protein_coding | protein coding | NA | NA |
| ENSG00000197785.13_2 | NA | NA | ATAD3A | 0,01935699 | 0,6455055 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000084112.14_3 | NA | NA | SSH1 | 0,01936267 | 0,58468112 | protein_coding | protein coding | NA | NA |
| ENSG00000134480.13_2 | NA | NA | CCNH | 0,01938002 | -0,80182676 | protein_coding | protein coding | NA | NA |
| ENSG00000164880.15_2 | NA | NA | INTS1 | 0,01943477 | 0,74053209 | protein_coding | protein coding | NA | NA |
| ENSG00000146067.15_2 | NA | NA | FAM193B | 0,01945073 | 0,59673983 | protein_coding | protein coding | NA | NA |
| ENSG00000124743.5_2 | NA | NA | KLHL31 | 0,01948971 | 0,54620233 | protein_coding | protein coding | NA | NA |
| ENSG00000008323.15_2 | NA | NA | PLEKHG6 | 0,01951457 | 0,70621606 | protein_coding | protein coding | NA | NA |
| ENSG00000198792.12_3 | NA | NA | TMEM184B | 0,0195345 | 0,57335206 | protein_coding | protein coding | NA | NA |
| ENSG00000120915.13_4 | NA | NA | EPHX2 | 0,01955356 | -0,64689683 | protein_coding | protein coding | NA | NA |
| ENSG00000240204.2_2 | NA | NA | SMKR1 | 0,01959441 | 0,5393277 | protein_coding | protein coding | NA | NA |
| ENSG00000138193.15_3 | NA | NA | PLCE1 | 0,01963483 | 0,46142649 | protein_coding | protein coding | NA | NA |
| ENSG00000163930.9_2 | NA | NA | BAP1 | 0,01966501 | 0,64724082 | protein_coding | protein coding | NA | NA |
| ENSG00000154920.14_3 | NA | NA | EME1 | 0,0197897 | 0,64641797 | protein_coding | protein coding | NA | NA |
| ENSG00000165487.13_2 | NA | NA | MICU2 | 0,0198438 | -0,59025614 | protein_coding | protein coding | NA | NA |
| ENSG00000102032.12_3 | NA | NA | RENBP | 0,01990189 | 0,52699761 | protein_coding | protein coding | NA | NA |
| ENSG00000154917.10_2 | NA | NA | RAB6B | 0,01991814 | 0,60017718 | protein_coding | protein coding | NA | NA |
| ENSG00000127452.8_2 | NA | NA | FBXL12 | 0,02001566 | 0,60461026 | protein_coding | protein coding | NA | NA |
| ENSG00000259702.2_3 | NA | NA | AC024337.1 | 0,0200534 | 0,50664044 | lincRNA | lncRNA | NA | NA |
| ENSG00000255622.3_2 | NA | NA | PCDHB17P | 0,02009405 | 0,52474503 | transcribed_unprocessed_pseudogene | pseudogene | NA | NA |
| ENSG00000105323.16_2 | NA | NA | HNRNPUL1 | 0,02009796 | 0,73600279 | protein_coding | protein coding | NA | NA |
| ENSG00000130675.14_3 | NA | NA | MNX1 | 0,02018137 | 0,50289082 | protein_coding | protein coding | NA | NA |
| ENSG00000089351.14_3 | NA | NA | GRAMD1A | 0,02023653 | 0,66041636 | protein_coding | protein coding | NA | NA |
| ENSG00000143514.16_2 | NA | NA | TP53BP2 | 0,02032624 | 0,61484161 | protein_coding | protein coding | NA | NA |
| ENSG00000256916.1_3 | NA | NA | AP000851.1 | 0,02033027 | 0,38734436 | antisense_RNA | lncRNA | NA | NA |
| ENSG00000160051.11_3 | NA | NA | IQCC | 0,02033675 | 0,61599286 | protein_coding | protein coding | NA | NA |
| ENSG00000226824.6_4 | NA | NA | AC006001.2 | 0,02043644 | 0,56900865 | sense_intronic | lncRNA | NA | NA |
| ENSG00000122574.10_2 | NA | NA | WIPF3 | 0,02046716 | 0,47582868 | protein_coding | protein coding | NA | NA |
| ENSG00000211648.2_2 | NA | NA | IGLV1-47 | 0,02048106 | 0,52103474 | IG_V_gene | Ig / TcR | NA | NA |
| ENSG00000196642.18_3 | NA | NA | RABL6 | 0,02053113 | 0,64640381 | protein_coding | protein coding | NA | NA |
| ENSG00000253223.1_3 | NA | NA | AC110998.1 | 0,02053486 | 0,59009887 | processed_pseudogene | pseudogene | NA | NA |
| ENSG00000049323.15_2 | NA | NA | LTBP1 | 0,02054664 | 0,57245994 | protein_coding | protein coding | NA | NA |
| ENSG00000054690.13_2 | NA | NA | PLEKHH1 | 0,02059138 | -0,56895317 | protein_coding | protein coding | NA | NA |
| ENSG00000138744.14_4 | NA | NA | NAAA | 0,02060817 | -0,65329428 | protein_coding | protein coding | NA | NA |
| ENSG00000156983.15_3 | NA | NA | BRPF1 | 0,02063337 | 0,66454005 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000122566.20_3 | NA | NA | HNRNPA2B1 | 0,0207217 | 0,63443502 | protein_coding | protein coding | NA | NA |
| ENSG00000169609.13_2 | NA | NA | C15orf40 | 0,02073854 | 0,60740548 | protein_coding | protein coding | NA | NA |
| ENSG00000242802.6_2 | NA | NA | AP5Z1 | 0,02079863 | 0,56791969 | protein_coding | protein coding | NA | NA |
| ENSG00000261360.1_3 | NA | NA | AC010491.1 | 0,02088045 | 0,47548601 | antisense_RNA | lncRNA | NA | NA |
| ENSG00000165025.14_2 | NA | NA | SYK | 0,02089535 | 0,64350459 | protein_coding | protein coding | NA | NA |
| ENSG00000256128.5_2 | NA | NA | LINC00944 | 0,02092545 | 0,55394368 | lincRNA | lncRNA | NA | NA |
| ENSG00000162688.16_3 | NA | NA | AGL | 0,02093508 | -0,66460774 | protein_coding | protein coding | NA | NA |
| ENSG00000159884.11_3 | NA | NA | CCDC107 | 0,02094044 | 0,42144649 | protein_coding | protein coding | NA | NA |
| ENSG00000100836.10_3 | NA | NA | PABPN1 | 0,02094753 | 0,6054652 | protein_coding | protein coding | NA | NA |
| ENSG00000127831.10_3 | NA | NA | VIL1 | 0,02102921 | 0,50292607 | protein_coding | protein coding | NA | NA |
| ENSG00000167657.13_3 | NA | NA | DAPK3 | 0,02104658 | 0,61209093 | protein_coding | protein coding | NA | NA |
| ENSG00000232295.7_4 | NA | NA | AL589935.1 | 0,0210615 | -0,59780599 | processed_transcript | lncRNA | NA | NA |
| ENSG00000260418.1_3 | NA | NA | AL023284.4 | 0,02108279 | 0,45179396 | lincRNA | lncRNA | NA | NA |
| ENSG00000184060.10_3 | NA | NA | ADAP2 | 0,02108714 | 0,5534058 | protein_coding | protein coding | NA | NA |
| ENSG00000182979.17_4 | NA | NA | MTA1 | 0,02111266 | 0,64889895 | protein_coding | protein coding | NA | NA |
| ENSG00000116761.11_2 | NA | NA | CTH | 0,02114615 | -0,66053162 | protein_coding | protein coding | NA | NA |
| ENSG00000253276.2_4 | NA | NA | CCDC71L | 0,02115408 | 0,54264616 | protein_coding | protein coding | NA | NA |
| ENSG00000131470.14_2 | NA | NA | PSMC3IP | 0,02119766 | 0,61075889 | protein_coding | protein coding | NA | NA |
| ENSG00000233994.2_2 | NA | NA | GDI2P2 | 0,02122528 | -0,5805929 | processed_pseudogene | pseudogene | NA | NA |
| ENSG00000157456.7_3 | NA | NA | CCNB2 | 0,02123871 | 0,61304474 | protein_coding | protein coding | NA | NA |
| ENSG00000196584.2_2 | NA | NA | XRCC2 | 0,02127836 | 0,62152949 | protein_coding | protein coding | NA | NA |
| ENSG00000086205.16_2 | NA | NA | FOLH1 | 0,02130411 | -0,62188467 | protein_coding | protein coding | NA | NA |
| ENSG00000176915.14_2 | NA | NA | ANKLE2 | 0,02135547 | 0,87682099 | protein_coding | protein coding | NA | NA |
| ENSG00000106070.17_3 | NA | NA | GRB10 | 0,02146569 | 0,60359353 | protein_coding | protein coding | NA | NA |
| ENSG00000184990.12_2 | NA | NA | SIVA1 | 0,02148942 | 0,65272841 | protein_coding | protein coding | NA | NA |
| ENSG00000149657.19_3 | NA | NA | LSM14B | 0,02152567 | 0,56860974 | protein_coding | protein coding | NA | NA |
| ENSG00000085552.16_2 | NA | NA | IGSF9 | 0,02163028 | 0,62145497 | protein_coding | protein coding | NA | NA |
| ENSG00000211639.2_2 | NA | NA | IGLV4-60 | 0,02168535 | 0,3854758 | IG_V_gene | Ig / TcR | NA | NA |
| ENSG00000272304.1_4 | NA | NA | AC096721.1 | 0,02173921 | 0,36639248 | lincRNA | lncRNA | NA | NA |
| ENSG00000153179.12_3 | NA | NA | RASSF3 | 0,02179629 | -0,7280614 | protein_coding | protein coding | NA | NA |
| ENSG00000008256.15_2 | NA | NA | CYTH3 | 0,02181867 | 0,53304612 | protein_coding | protein coding | NA | NA |
| ENSG00000172543.7_3 | NA | NA | CTSW | 0,0218331 | 0,57711282 | protein_coding | protein coding | NA | NA |
| ENSG00000101191.16_3 | NA | NA | DIDO1 | 0,0218759 | 0,59277407 | protein_coding | protein coding | NA | NA |
| ENSG00000197063.10_3 | NA | NA | MAFG | 0,02192368 | 0,63350057 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000177045.7_2 | NA | NA | SIX5 | 0,02196427 | 0,57369499 | protein_coding | protein coding | NA | NA |
| ENSG00000128918.14_3 | NA | NA | ALDH1A2 | 0,02197779 | -0,68109013 | protein_coding | protein coding | NA | NA |
| ENSG00000134107.4_2 | NA | NA | BHLHE40 | 0,02198773 | 0,58709289 | protein_coding | protein coding | NA | NA |
| ENSG00000147010.17_3 | NA | NA | SH3KBP1 | 0,02218398 | 0,62172039 | protein_coding | protein coding | NA | NA |
| ENSG00000161036.12_3 | NA | NA | LRWD1 | 0,02223524 | 0,60723665 | protein_coding | protein coding | NA | NA |
| ENSG00000163558.12_2 | NA | NA | PRKCI | 0,02223625 | 0,65581222 | protein_coding | protein coding | NA | NA |
| ENSG00000116005.11_2 | NA | NA | PCYOX1 | 0,02230465 | -0,56821917 | protein_coding | protein coding | NA | NA |
| ENSG00000163617.10_2 | NA | NA | CCDC191 | 0,02234682 | 0,55866823 | protein_coding | protein coding | NA | NA |
| ENSG00000060491.16_3 | NA | NA | OGFR | 0,02256199 | 0,53462238 | protein_coding | protein coding | NA | NA |
| ENSG00000277893.1_2 | NA | NA | SRD5A2 | 0,02265 | -0,6338243 | protein_coding | protein coding | NA | NA |
| ENSG00000170445.12_3 | NA | NA | HARS | 0,02266825 | -0,56227872 | protein_coding | protein coding | NA | NA |
| ENSG00000168067.11_4 | NA | NA | MAP4K2 | 0,02270451 | 0,60059488 | protein_coding | protein coding | NA | NA |
| ENSG00000119333.11_2 | NA | NA | WDR34 | 0,02270638 | 0,62206526 | protein_coding | protein coding | NA | NA |
| ENSG00000163191.5_2 | NA | NA | S100A11 | 0,02271996 | 0,58059859 | protein_coding | protein coding | NA | NA |
| ENSG00000153485.5_3 | NA | NA | TMEM251 | 0,02272342 | -0,57725352 | protein_coding | protein coding | NA | NA |
| ENSG00000178188.14_3 | NA | NA | SH2B1 | 0,02285808 | 0,70094765 | protein_coding | protein coding | NA | NA |
| ENSG00000215193.12_4 | NA | NA | PEX26 | 0,0228652 | 0,68802979 | protein_coding | protein coding | NA | NA |
| ENSG00000138778.11_2 | NA | NA | CENPE | 0,02287176 | 0,61500329 | protein_coding | protein coding | NA | NA |
| ENSG00000229056.2_3 | NA | NA | AC020571.1 | 0,02290145 | -0,67088369 | antisense_RNA | lncRNA | NA | NA |
| ENSG00000137573.13_3 | NA | NA | SULF1 | 0,02303875 | 0,61219906 | protein_coding | protein coding | NA | NA |
| ENSG00000049540.16_3 | NA | NA | ELN | 0,02310706 | 0,58559614 | protein_coding | protein coding | NA | NA |
| ENSG00000117360.12_2 | NA | NA | PRPF3 | 0,02313045 | 0,60159285 | protein_coding | protein coding | NA | NA |
| ENSG00000106397.11_3 | NA | NA | PLOD3 | 0,0233416 | 0,56180073 | protein_coding | protein coding | NA | NA |
| ENSG00000122012.13_3 | NA | NA | SV2C | 0,02335666 | 0,4819521 | protein_coding | protein coding | NA | NA |
| ENSG00000153714.5_2 | NA | NA | LURAP1L | 0,02341609 | 0,57208732 | protein_coding | protein coding | NA | NA |
| ENSG00000165891.15_3 | NA | NA | E2F7 | 0,0234548 | 0,63673301 | protein_coding | protein coding | NA | NA |
| ENSG00000245571.6_4 | NA | NA | AP001258.1 | 0,02352404 | -0,82215442 | lincRNA | lncRNA | NA | NA |
| ENSG00000146535.13_2 | NA | NA | GNA12 | 0,02354537 | 0,58091748 | protein_coding | protein coding | NA | NA |
| ENSG00000189298.13_2 | NA | NA | ZKSCAN3 | 0,02354818 | 0,62160826 | protein_coding | protein coding | NA | NA |
| ENSG00000205339.9_2 | NA | NA | IPO7 | 0,02359277 | -0,52024272 | protein_coding | protein coding | NA | NA |
| ENSG00000141627.13_3 | NA | NA | DYM | 0,02359497 | -0,59853264 | protein_coding | protein coding | NA | NA |
| ENSG00000134056.11_2 | NA | NA | MRPS36 | 0,02360013 | -0,52085851 | protein_coding | protein coding | NA | NA |
| ENSG00000240370.6_2 | NA | NA | RPL13P5 | 0,02363854 | 0,62512069 | transcribed_processed_pseudogene | pseudogene | NA | NA |
| ENSG00000176444.18_2 | NA | NA | CLK2 | 0,02364842 | 0,62790142 | protein_coding | protein coding | NA | NA |
| ENSG00000211655.3_2 | NA | NA | IGLV1-36 | 0,02365083 | 0,52682515 | IG_V_gene | Ig / TcR | NA | NA |

EP 3 901 288 A1

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000172716.16_3 | NA | NA | SLFN11 | 0,02365649 | 0,6469471 | protein_coding | protein coding | NA | NA |
| ENSG00000075336.11_3 | NA | NA | TIMM21 | 0,02374734 | -0,58065317 | protein_coding | protein coding | NA | NA |
| ENSG00000112902.11_3 | NA | NA | SEMA5A | 0,02377221 | 0,50378513 | protein_coding | protein coding | NA | NA |
| ENSG00000160957.12_3 | NA | NA | RECQL4 | 0,02385091 | 0,55405706 | protein_coding | protein coding | NA | NA |
| ENSG00000163281.11_3 | NA | NA | GNPDA2 | 0,02385907 | -0,57291273 | protein_coding | protein coding | NA | NA |
| ENSG00000165490.12_3 | NA | NA | DDIAS | 0,02388603 | 0,62868176 | protein_coding | protein coding | NA | NA |
| ENSG00000166592.11_3 | NA | NA | RRAD | 0,0238894 | 0,5486542 | protein_coding | protein coding | NA | NA |
| ENSG00000117983.17_3 | NA | NA | MUC5B | 0,0239186 | 0,3799802 | protein_coding | protein coding | NA | NA |
| ENSG00000113761.11_3 | NA | NA | ZNF346 | 0,0239196 | 0,63197217 | protein_coding | protein coding | NA | NA |
| ENSG00000279020.1_3 | NA | NA | C18orf15 | 0,02392093 | 0,51252768 | TEC | lncRNA | NA | NA |
| ENSG00000234771.3_3 | NA | NA | SLC25A25-AS1 | 0,02394197 | 0,57253904 | antisense_RNA | lncRNA | NA | NA |
| ENSG00000214870.8_3 | NA | NA | AC004540.1 | 0,02395098 | 0,53857395 | lincRNA | lncRNA | NA | NA |
| ENSG00000111445.13_2 | NA | NA | RFC5 | 0,02397637 | 0,62924698 | protein_coding | protein coding | NA | NA |
| ENSG00000158079.14_3 | NA | NA | PTPDC1 | 0,02411599 | 0,54965149 | protein_coding | protein coding | NA | NA |
| ENSG00000239523.5_3 | NA | NA | MYLK-AS1 | 0,02418969 | 0,39121077 | antisense_RNA | lncRNA | NA | NA |
| ENSG00000138160.5_2 | NA | NA | KIF11 | 0,02423351 | 0,58774814 | protein_coding | protein coding | NA | NA |
| ENSG00000011105.12_3 | NA | NA | TSPAN9 | 0,02425697 | 0,63489449 | protein_coding | protein coding | NA | NA |
| ENSG00000250986.1_2 | NA | NA | AC141928.1 | 0,02427073 | -0,75237855 | lincRNA | lncRNA | NA | NA |
| ENSG00000196092.12_2 | NA | NA | PAX5 | 0,02432529 | 0,44812598 | protein_coding | protein coding | NA | NA |
| ENSG00000136279.20_3 | NA | NA | DBNL | 0,0243841 | 0,55839331 | protein_coding | protein coding | NA | NA |
| ENSG00000276180.1_2 | NA | NA | HIST1H4I | 0,02440057 | 0,6284341 | protein_coding | protein coding | NA | NA |
| ENSG00000160796.16_2 | NA | NA | NBEAL2 | 0,02447038 | 0,60238913 | protein_coding | protein coding | NA | NA |
| ENSG00000001631.15_3 | NA | NA | KRIT1 | 0,02450648 | 0,43488464 | protein_coding | protein coding | NA | NA |
| ENSG00000114670.13_2 | NA | NA | NEK11 | 0,02450667 | 0,54973535 | protein_coding | protein coding | NA | NA |
| ENSG00000105221.16_3 | NA | NA | AKT2 | 0,02452194 | 0,55794663 | protein_coding | protein coding | NA | NA |
| ENSG00000151725.11_3 | NA | NA | CENPU | 0,02453375 | 0,62148713 | protein_coding | protein coding | NA | NA |
| ENSG00000104824.16_3 | NA | NA | HNRNPL | 0,02455006 | 0,6447104 | protein_coding | protein coding | NA | NA |
| ENSG00000111335.12_2 | NA | NA | OAS2 | 0,0246251 | 0,46608266 | protein_coding | protein coding | NA | NA |
| ENSG00000140284.10_2 | NA | NA | SLC27A2 | 0,02471178 | -0,62414923 | protein_coding | protein coding | NA | NA |
| ENSG00000137628.16_3 | NA | NA | DDX60 | 0,02472289 | 0,5023588 | protein_coding | protein coding | NA | NA |
| ENSG00000168291.12_2 | NA | NA | PDHB | 0,02475157 | -0,66766648 | protein_coding | protein coding | NA | NA |
| ENSG00000159214.12_2 | NA | NA | CCDC24 | 0,02480234 | 0,63089505 | protein_coding | protein coding | NA | NA |
| ENSG00000197226.12_1 | NA | NA | TBC1D9B | 0,02482608 | 0,61202462 | protein_coding | protein coding | NA | NA |
| ENSG00000182511.11_3 | NA | NA | FES | 0,02485983 | 0,66768593 | protein_coding | protein coding | NA | NA |
| ENSG00000277775.1_2 | NA | NA | HIST1H3F | 0,02490905 | 0,56160851 | protein_coding | protein coding | NA | NA |
| ENSG00000165966.14_2 | NA | NA | PDZRN4 | 0,02497564 | -0,59881229 | protein_coding | protein coding | NA | NA |

44

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000105643.9_3 | NA | NA | ARRDC2 | 0,02501791 | 0,5866799 | protein_coding | protein coding | NA | NA |
| ENSG00000268858.2_3 | NA | NA | AL118506.1 | 0,02503476 | 0,54701398 | antisense_RNA | lncRNA | NA | NA |
| ENSG00000185267.9_2 | NA | NA | CDNF | 0,02515016 | -0,65595897 | protein_coding | protein coding | NA | NA |
| ENSG00000114013.15_2 | NA | NA | CD86 | 0,02517986 | 0,60559589 | protein_coding | protein coding | NA | NA |
| ENSG00000112851.14_3 | NA | NA | ERBIN | 0,02522051 | -0,51539335 | protein_coding | protein coding | NA | NA |
| ENSG00000211897.9_3 | NA | NA | IGHG3 | 0,02522957 | 0,44241623 | IG_C_gene | Ig / TcR | NA | NA |
| ENSG00000060237.16_3 | NA | NA | WNK1 | 0,0253253 | 0,6845349 | protein_coding | protein coding | NA | NA |
| ENSG00000130701.3_2 | NA | NA | RBBP8NL | 0,02535073 | 0,54262149 | protein_coding | protein coding | NA | NA |
| ENSG00000006327.13_2 | NA | NA | TNFRSF12A | 0,0253935 | 0,54893205 | protein_coding | protein coding | NA | NA |
| ENSG00000196411.9_2 | NA | NA | EPHB4 | 0,0254393 | 0,72110669 | protein_coding | protein coding | NA | NA |
| ENSG00000106785.14_2 | NA | NA | TRIM14 | 0,02547524 | 0,52295589 | protein_coding | protein coding | NA | NA |
| ENSG00000166881.9_3 | NA | NA | NEMP1 | 0,02548454 | 0,58089083 | protein_coding | protein coding | NA | NA |
| ENSG00000111450.13_3 | NA | NA | STX2 | 0,02553348 | 0,59077381 | protein_coding | protein coding | NA | NA |
| ENSG00000106400.11_2 | NA | NA | ZNHIT1 | 0,02555291 | 0,54307132 | protein_coding | protein coding | NA | NA |
| ENSG00000078098.13_2 | NA | NA | FAP | 0,02555651 | 0,62084635 | protein_coding | protein coding | NA | NA |
| ENSG00000263001.5_2 | NA | NA | GTF2I | 0,02557528 | 0,57029438 | protein_coding | protein coding | NA | NA |
| ENSG00000117054.13_3 | NA | NA | ACADM | 0,02558123 | -0,50888082 | protein_coding | protein coding | NA | NA |
| ENSG00000175198.15_3 | NA | NA | PCCA | 0,0256139 | -0,57927473 | protein_coding | protein coding | NA | NA |
| ENSG00000146828.17_3 | NA | NA | SLC12A9 | 0,02561955 | 0,62415121 | protein_coding | protein coding | NA | NA |
| ENSG00000139636.15_2 | NA | NA | LMBR1L | 0,02565575 | 0,62403477 | protein_coding | protein coding | NA | NA |
| ENSG00000164402.13_4 | NA | NA | Sep 08 | 0,02567024 | 0,56393961 | protein_coding | protein coding | NA | NA |
| ENSG00000072518.20_2 | NA | NA | MARK2 | 0,02567688 | 0,65865738 | protein_coding | protein coding | NA | NA |
| ENSG00000011304.19_4 | NA | NA | PTBP1 | 0,02568194 | 0,65827466 | protein_coding | protein coding | NA | NA |
| ENSG00000151500.14_2 | NA | NA | THYN1 | 0,02577189 | -0,80378675 | protein_coding | protein coding | NA | NA |
| ENSG00000137203.10_2 | NA | NA | TFAP2A | 0,02577805 | 0,60177587 | protein_coding | protein coding | NA | NA |
| ENSG00000166960.16_4 | NA | NA | CCDC178 | 0,02578154 | -0,64793398 | protein_coding | protein coding | NA | NA |
| ENSG00000145214.13_3 | NA | NA | DGKQ | 0,02581447 | 0,49355237 | protein_coding | protein coding | NA | NA |
| ENSG00000184254.16_3 | NA | NA | ALDH1A3 | 0,02585063 | -0,67100471 | protein_coding | protein coding | NA | NA |
| ENSG00000146670.9_3 | NA | NA | CDCA5 | 0,02585783 | 0,62076273 | protein_coding | protein coding | NA | NA |
| ENSG00000185480.11_2 | NA | NA | PARPBP | 0,02588016 | 0,5968472 | protein_coding | protein coding | NA | NA |
| ENSG00000163541.11_2 | NA | NA | SUCLG1 | 0,02592771 | -0,60985132 | protein_coding | protein coding | NA | NA |
| ENSG00000104472.9_3 | NA | NA | CHRAC1 | 0,0261035 | 0,5405175 | protein_coding | protein coding | NA | NA |
| ENSG00000154265.15_3 | NA | NA | ABCA5 | 0,02610917 | -0,6922514 | protein_coding | protein coding | NA | NA |
| ENSG00000173039.18_3 | NA | NA | RELA | 0,02613178 | 0,64479605 | protein_coding | protein coding | NA | NA |
| ENSG00000101391.20_3 | NA | NA | CDK5RAP1 | 0,02624912 | 0,58557469 | protein_coding | protein coding | NA | NA |
| ENSG00000196139.13_3 | NA | NA | AKR1C3 | 0,02634237 | 0,58681619 | protein_coding | protein coding | NA | NA |
| ENSG00000168542.14_3 | NA | NA | COL3A1 | 0,02642442 | 0,67294516 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000183337.16_2 | NA | NA | BCOR | 0,02649856 | 0,6118476 | protein_coding | protein coding | NA | NA |
| ENSG00000214655.10_3 | NA | NA | ZSWIM8 | 0,02652984 | 0,51517103 | protein_coding | protein coding | NA | NA |
| ENSG00000162692.10_2 | NA | NA | VCAM1 | 0,02654155 | 0,6467296 | protein_coding | protein coding | NA | NA |
| ENSG00000124575.6_2 | NA | NA | HIST1H1D | 0,02659085 | 0,62541838 | protein_coding | protein coding | NA | NA |
| ENSG00000152454.3_2 | NA | NA | ZNF256 | 0,02670341 | 0,62244406 | protein_coding | protein coding | NA | NA |
| ENSG00000169155.9_3 | NA | NA | ZBTB43 | 0,02672626 | 0,42469847 | protein_coding | protein coding | NA | NA |
| ENSG00000230454.1_3 | NA | NA | U73166.1 | 0,02675823 | 0,52045963 | lincRNA | lncRNA | NA | NA |
| ENSG00000123136.14_2 | NA | NA | DDX39A | 0,0267829 | 0,63041779 | protein_coding | protein coding | NA | NA |
| ENSG00000125826.20_3 | NA | NA | RBCK1 | 0,0267842 | 0,62567633 | protein_coding | protein coding | NA | NA |
| ENSG00000261342.1_3 | NA | NA | AC006538.1 | 0,02691967 | 0,45860306 | sense_intronic | lncRNA | NA | NA |
| ENSG00000229370.1_3 | NA | NA | AC073062.1 | 0,02693139 | -0,76828339 | lincRNA | lncRNA | NA | NA |
| ENSG00000122557.9_3 | NA | NA | HERPUD2 | 0,02694272 | 0,5858953 | protein_coding | protein coding | NA | NA |
| ENSG00000124659.6_2 | NA | NA | TBCC | 0,02698287 | 0,58130588 | protein_coding | protein coding | NA | NA |
| ENSG00000153006.15_3 | NA | NA | SREK1IP1 | 0,02702954 | -0,45155807 | protein_coding | protein coding | NA | NA |
| ENSG00000116459.10_2 | NA | NA | ATP5F1 | 0,02703065 | -0,49625936 | protein_coding | protein coding | NA | NA |
| ENSG00000135931.17_3 | NA | NA | ARMC9 | 0,02704355 | 0,54297884 | protein_coding | protein coding | NA | NA |
| ENSG00000100526.19_3 | NA | NA | CDKN3 | 0,0270908 | 0,52873427 | protein_coding | protein coding | NA | NA |
| ENSG00000114248.9_3 | NA | NA | LRRC31 | 0,0271647 | 0,45281393 | protein_coding | protein coding | NA | NA |
| ENSG00000101460.12_2 | NA | NA | MAP1LC3A | 0,02720825 | 0,56993217 | protein_coding | protein coding | NA | NA |
| ENSG00000171552.12_3 | NA | NA | BCL2L1 | 0,027213 | 0,54438605 | protein_coding | protein coding | NA | NA |
| ENSG00000105397.13_3 | NA | NA | TYK2 | 0,02721697 | 0,69679464 | protein_coding | protein coding | NA | NA |
| ENSG00000163808.16_3 | NA | NA | KIF15 | 0,02721838 | 0,47475437 | protein_coding | protein coding | NA | NA |
| ENSG00000163872.15_3 | NA | NA | YEATS2 | 0,02749814 | 0,58578085 | protein_coding | protein coding | NA | NA |
| ENSG00000175336.9_2 | NA | NA | APOF | 0,02751457 | -0,63868496 | protein_coding | protein coding | NA | NA |
| ENSG00000139725.7_3 | NA | NA | RHOF | 0,02752153 | 0,62057433 | protein_coding | protein coding | NA | NA |
| ENSG00000136238.17_2 | NA | NA | RAC1 | 0,02752465 | 0,61953207 | protein_coding | protein coding | NA | NA |
| ENSG00000230223.6_3 | NA | NA | ATXN8OS | 0,02754096 | 0,42172609 | antisense_RNA | lncRNA | NA | NA |
| ENSG00000102524.11_3 | NA | NA | TNFSF13B | 0,02763417 | 0,5754188 | protein_coding | protein coding | NA | NA |
| ENSG00000131323.14_3 | NA | NA | TRAF3 | 0,02764261 | 0,63402745 | protein_coding | protein coding | NA | NA |
| ENSG00000112874.9_2 | NA | NA | NUDT12 | 0,02765159 | -0,52757986 | protein_coding | protein coding | NA | NA |
| ENSG00000047648.21_3 | NA | NA | ARHGAP6 | 0,02766243 | -0,53444591 | protein_coding | protein coding | NA | NA |
| ENSG00000112799.8_2 | NA | NA | LY86 | 0,02772516 | 0,63602198 | protein_coding | protein coding | NA | NA |
| ENSG00000261502.3_4 | NA | NA | AC040174.1 | 0,02780197 | -0,9086351 | processed_transcript | lncRNA | NA | NA |
| ENSG00000171703.16_3 | NA | NA | TCEA2 | 0,02780235 | 0,60527443 | protein_coding | protein coding | NA | NA |
| ENSG00000184634.15_3 | NA | NA | MED12 | 0,02791952 | 0,75286094 | protein_coding | protein coding | NA | NA |
| ENSG00000137959.15_3 | NA | NA | IFI44L | 0,02793375 | 0,38581647 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000197472.14_3 | NA | NA | ZNF695 | 0,02809714 | 0,5804235 | protein_coding | protein coding | NA | NA |
| ENSG00000164896.19_3 | NA | NA | FASTK | 0,02812657 | 0,56437698 | protein_coding | protein coding | NA | NA |
| ENSG00000141577.13_3 | NA | NA | CEP131 | 0,02816729 | 0,69372892 | protein_coding | protein coding | NA | NA |
| ENSG00000179262.9_2 | NA | NA | RAD23A | 0,02817349 | 0,72344931 | protein_coding | protein coding | NA | NA |
| ENSG00000188878.19_4 | NA | NA | FBF1 | 0,02819744 | 0,63001638 | protein_coding | protein coding | NA | NA |
| ENSG00000129173.12_2 | NA | NA | E2F8 | 0,02823144 | 0,57103006 | protein_coding | protein coding | NA | NA |
| ENSG00000145423.4_2 | NA | NA | SFRP2 | 0,02830189 | 0,53702165 | protein_coding | protein coding | NA | NA |
| ENSG00000104154.6_2 | NA | NA | SLC30A4 | 0,02830453 | -0,53260705 | protein_coding | protein coding | NA | NA |
| ENSG00000089289.15_2 | NA | NA | IGBP1 | 0,02830992 | -0,56949047 | protein_coding | protein coding | NA | NA |
| ENSG00000090889.11_2 | NA | NA | KIF4A | 0,02831939 | 0,47803168 | protein_coding | protein coding | NA | NA |
| ENSG00000267532.5_4 | NA | NA | MIR497HG | 0,02834919 | 0,56318436 | antisense_RNA | lncRNA | NA | NA |
| ENSG00000156687.10_3 | NA | NA | UNC5D | 0,02846825 | -0,70962822 | protein_coding | protein coding | NA | NA |
| ENSG00000168209.4_2 | NA | NA | DDIT4 | 0,02849047 | 0,56005797 | protein_coding | protein coding | NA | NA |
| ENSG00000143942.4_2 | NA | NA | CHAC2 | 0,02855805 | 0,53798783 | protein_coding | protein coding | NA | NA |
| ENSG00000262712.1_4 | NA | NA | AC012676.1 | 0,0285613 | 0,50935904 | sense_intronic | lncRNA | NA | NA |
| ENSG00000118707.9_3 | NA | NA | TGIF2 | 0,02856528 | 0,62726825 | protein_coding | protein coding | NA | NA |
| ENSG00000115353.10_2 | NA | NA | TACR1 | 0,028614 | 0,47069375 | protein_coding | protein coding | NA | NA |
| ENSG00000259330.2_2 | NA | NA | INAFM2 | 0,02863373 | -0,61041353 | protein_coding | protein coding | NA | NA |
| ENSG00000160710.15_3 | NA | NA | ADAR | 0,02873821 | 0,42627783 | protein_coding | protein coding | NA | NA |
| ENSG00000168661.14_2 | NA | NA | ZNF30 | 0,02878173 | -0,73468665 | protein_coding | protein coding | NA | NA |
| ENSG00000225697.12_3 | NA | NA | SLC26A6 | 0,02886317 | 0,58712641 | protein_coding | protein coding | NA | NA |
| ENSG00000106459.14_2 | NA | NA | NRF1 | 0,02907962 | 0,46488238 | protein_coding | protein coding | NA | NA |
| ENSG00000156026.14_3 | NA | NA | MCU | 0,02926131 | 0,49654612 | protein_coding | protein coding | NA | NA |
| ENSG00000101049.14_3 | NA | NA | SGK2 | 0,02933529 | 0,46096105 | protein_coding | protein coding | NA | NA |
| ENSG00000105993.14_4 | NA | NA | DNAJB6 | 0,02934859 | 0,53371293 | protein_coding | protein coding | NA | NA |
| ENSG00000068308.13_3 | NA | NA | OTUD5 | 0,02936706 | 0,64873684 | protein_coding | protein coding | NA | NA |
| ENSG00000068323.16_2 | NA | NA | TFE3 | 0,02938569 | 0,6321617 | protein_coding | protein coding | NA | NA |
| ENSG00000117114.19_3 | NA | NA | ADGRL2 | 0,02940192 | -0,74128615 | protein_coding | protein coding | NA | NA |
| ENSG00000182325.10_3 | NA | NA | FBXL6 | 0,02944324 | 0,61355646 | protein_coding | protein coding | NA | NA |
| ENSG00000198887.8_3 | NA | NA | SMC5 | 0,02953163 | 0,65899161 | protein_coding | protein coding | NA | NA |
| ENSG00000211662.2_2 | NA | NA | IGLV3-21 | 0,02961363 | 0,49656943 | IG_V_gene | Ig / TcR | NA | NA |
| ENSG00000269781.1 | NA | NA | FLJ20306 | 0,02963678 | 0,45481473 | protein_coding | protein coding | NA | NA |
| ENSG00000169398.19_3 | NA | NA | PTK2 | 0,02968729 | 0,56793293 | protein_coding | protein coding | NA | NA |
| ENSG00000131408.13_4 | NA | NA | NR1H2 | 0,02973774 | 0,54020686 | protein_coding | protein coding | NA | NA |
| ENSG00000205544.3_3 | NA | NA | TMEM256 | 0,02979948 | -0,57421628 | protein_coding | protein coding | NA | NA |
| ENSG00000129351.17_2 | NA | NA | ILF3 | 0,0298094 | 0,71993741 | protein_coding | protein coding | NA | NA |
| ENSG00000230532.1_3 | NA | NA | AC091133.1 | 0,02981658 | 0,51226564 | antisense_RNA | lncRNA | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000153391.15_4 | NA | NA | INO80C | 0,02982433 | -0,51969079 | protein_coding | protein coding | NA | NA |
| ENSG00000163159.12_3 | NA | NA | VPS72 | 0,02992737 | 0,49794188 | protein_coding | protein coding | NA | NA |
| ENSG00000165813.18_3 | NA | NA | CCDC186 | 0,03001466 | -0,67351419 | protein_coding | protein coding | NA | NA |
| ENSG00000103148.15_3 | NA | NA | NPRL3 | 0,03025878 | 0,57778166 | protein_coding | protein coding | NA | NA |
| ENSG00000183087.14_2 | NA | NA | GAS6 | 0,03035801 | 0,59458596 | protein_coding | protein coding | NA | NA |
| ENSG00000038427.15_3 | NA | NA | VCAN | 0,03035902 | 0,5683976 | protein_coding | protein coding | NA | NA |
| ENSG00000092853.13_2 | NA | NA | CLSPN | 0,0303937 | 0,59111527 | protein_coding | protein coding | NA | NA |
| ENSG00000244578.1_2 | NA | NA | LINC01391 | 0,03042403 | 0,37145518 | lincRNA | lncRNA | NA | NA |
| ENSG00000167632.14_3 | NA | NA | TRAPPC9 | 0,03043559 | 0,55692736 | protein_coding | protein coding | NA | NA |
| ENSG00000122824.10_3 | NA | NA | NUDT10 | 0,0304755 | 0,47636034 | protein_coding | protein coding | NA | NA |
| ENSG00000146918.19_2 | NA | NA | NCAPG2 | 0,03051748 | 0,5856419 | protein_coding | protein coding | NA | NA |
| ENSG00000197548.12_2 | NA | NA | ATG7 | 0,03052469 | 0,63709555 | protein_coding | protein coding | NA | NA |
| ENSG00000223685.5_2 | NA | NA | LINC00571 | 0,03054234 | -0,64145098 | lincRNA | lncRNA | NA | NA |
| ENSG00000162148.10_3 | NA | NA | PPP1R32 | 0,03055635 | 0,57852176 | protein_coding | protein coding | NA | NA |
| ENSG00000077274.8_2 | NA | NA | CAPN6 | 0,0306074 | -0,68181076 | protein_coding | protein coding | NA | NA |
| ENSG00000091164.12_3 | NA | NA | TXNL1 | 0,0307583 | -0,54153075 | protein_coding | protein coding | NA | NA |
| ENSG00000169926.10_3 | NA | NA | KLF13 | 0,03084403 | 0,63846534 | protein_coding | protein coding | NA | NA |
| ENSG00000255404.1_3 | NA | NA | AP001266.1 | 0,03087777 | 0,54196312 | antisense_RNA | lncRNA | NA | NA |
| ENSG00000151320.10_3 | NA | NA | AKAP6 | 0,03093006 | 0,36772803 | protein_coding | protein coding | NA | NA |
| ENSG00000106244.12_2 | NA | NA | PDAP1 | 0,03102961 | 0,53889713 | protein_coding | protein coding | NA | NA |
| ENSG00000157800.17_2 | NA | NA | SLC37A3 | 0,03104799 | 0,53419059 | protein_coding | protein coding | NA | NA |
| ENSG00000168078.9_2 | NA | NA | PBK | 0,03107129 | 0,50994764 | protein_coding | protein coding | NA | NA |
| ENSG00000197037.10_3 | NA | NA | ZSCAN25 | 0,03112596 | 0,57798863 | protein_coding | protein coding | NA | NA |
| ENSG00000153187.18_3 | NA | NA | HNRNPU | 0,03113158 | 0,56344395 | protein_coding | protein coding | NA | NA |
| ENSG00000106344.8_3 | NA | NA | RBM28 | 0,03118643 | 0,59429791 | protein_coding | protein coding | NA | NA |
| ENSG00000131591.17_3 | NA | NA | C1orf159 | 0,03120325 | 0,51051041 | protein_coding | protein coding | NA | NA |
| ENSG00000163348.3_3 | NA | NA | PYGO2 | 0,03122664 | 0,61647638 | protein_coding | protein coding | NA | NA |
| ENSG00000140743.7_3 | NA | NA | CDR2 | 0,03128813 | 0,55564709 | protein_coding | protein coding | NA | NA |
| ENSG00000034510.5_2 | NA | NA | TMSB10 | 0,03129879 | 0,5854406 | protein_coding | protein coding | NA | NA |
| ENSG00000107669.17_4 | NA | NA | ATE1 | 0,03134533 | -0,53936205 | protein_coding | protein coding | NA | NA |
| ENSG00000182022.17_3 | NA | NA | CHST15 | 0,03139098 | 0,58830375 | protein_coding | protein coding | NA | NA |
| ENSG00000183751.14_4 | NA | NA | TBL3 | 0,03146338 | 0,60465761 | protein_coding | protein coding | NA | NA |
| ENSG00000185052.11_2 | NA | NA | SLC24A3 | 0,03156437 | 0,53622073 | protein_coding | protein coding | NA | NA |
| ENSG00000223478.1_3 | NA | NA | AL441992.1 | 0,03158544 | -0,68373602 | antisense_RNA | lncRNA | NA | NA |
| ENSG00000174579.3_3 | NA | NA | MSL2 | 0,03168839 | 0,61466546 | protein_coding | protein coding | NA | NA |
| ENSG00000169607.12_2 | NA | NA | CKAP2L | 0,03169258 | 0,58289978 | protein_coding | protein coding | NA | NA |
| ENSG00000196872.11_3 | NA | NA | KIAA1211L | 0,03170648 | 0,51826052 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000261150.2_2 | NA | NA | EPPK1 | 0,0317162 | 0,54649816 | protein_coding | protein coding | NA | NA |
| ENSG00000238835.1 | NA | NA | SCARNA18 | 0,03179144 | -0,58998216 | snoRNA | small ncRNA | NA | NA |
| ENSG00000163655.15_2 | NA | NA | GMPS | 0,0318378 | 0,42027885 | protein_coding | protein coding | NA | NA |
| ENSG00000179833.4_2 | NA | NA | SERTAD2 | 0,03193682 | 0,61828599 | protein_coding | protein coding | NA | NA |
| ENSG00000134460.15_2 | NA | NA | IL2RA | 0,03197772 | 0,53003347 | protein_coding | protein coding | NA | NA |
| ENSG00000130479.10_4 | NA | NA | MAP1S | 0,03202483 | 0,62239478 | protein_coding | protein coding | NA | NA |
| ENSG00000235706.7_3 | NA | NA | DICER1-AS1 | 0,03208225 | 0,53712804 | antisense_RNA | lncRNA | NA | NA |
| ENSG00000176890.15_2 | NA | NA | TYMS | 0,0321806 | 0,53896713 | protein_coding | protein coding | NA | NA |
| ENSG00000062038.13_3 | NA | NA | CDH3 | 0,03222895 | 0,55330419 | protein_coding | protein coding | NA | NA |
| ENSG00000168421.12_3 | NA | NA | RHOH | 0,03227699 | 0,46085745 | protein_coding | protein coding | NA | NA |
| ENSG00000249992.1_2 | NA | NA | TMEM158 | 0,0323139 | -0,66467183 | protein_coding | protein coding | NA | NA |
| ENSG00000044574.7_2 | NA | NA | HSPA5 | 0,03241937 | -0,66685249 | protein_coding | protein coding | NA | NA |
| ENSG00000167702.11_2 | NA | NA | KIFC2 | 0,03242286 | 0,59978654 | protein_coding | protein coding | NA | NA |
| ENSG00000133103.16_2 | NA | NA | COG6 | 0,03245908 | -0,57675894 | protein_coding | protein coding | NA | NA |
| ENSG00000000460.16_4 | NA | NA | C1orf112 | 0,03246371 | 0,48255064 | protein_coding | protein coding | NA | NA |
| ENSG00000158169.12_3 | NA | NA | FANCC | 0,03250997 | 0,52847059 | protein_coding | protein coding | NA | NA |
| ENSG00000102034.16_2 | NA | NA | ELF4 | 0,03252687 | 0,59392181 | protein_coding | protein coding | NA | NA |
| ENSG00000182256.12_3 | NA | NA | GABRG3 | 0,03261446 | -0,61633454 | protein_coding | protein coding | NA | NA |
| ENSG00000211599.2_2 | NA | NA | IGKV5-2 | 0,0326178 | 0,4868929 | IG_V_gene | Ig / TcR | NA | NA |
| ENSG00000259605.3_4 | NA | NA | AC074212.1 | 0,03262913 | 0,51776096 | processed_transcript | lncRNA | NA | NA |
| ENSG00000251396.6_2 | NA | NA | LINC01301 | 0,03271698 | 0,62111249 | lincRNA | lncRNA | NA | NA |
| ENSG00000100368.13_4 | NA | NA | CSF2RB | 0,0327248 | 0,64188384 | protein_coding | protein coding | NA | NA |
| ENSG00000109906.13_3 | NA | NA | ZBTB16 | 0,03272982 | -0,61047176 | protein_coding | protein coding | NA | NA |
| ENSG00000140265.12_2 | NA | NA | ZSCAN29 | 0,03275739 | 0,64107047 | protein_coding | protein coding | NA | NA |
| ENSG00000163719.19_4 | NA | NA | MTMR14 | 0,03278164 | 0,56730472 | protein_coding | protein coding | NA | NA |
| ENSG00000099385.11_4 | NA | NA | BCL7C | 0,03279602 | 0,57742923 | protein_coding | protein coding | NA | NA |
| ENSG00000225855.6_3 | NA | NA | RUSC1-AS1 | 0,03282616 | 0,5274578 | antisense_RNA | lncRNA | NA | NA |
| ENSG00000075223.13_2 | NA | NA | SEMA3C | 0,03285777 | -0,77036466 | protein_coding | protein coding | NA | NA |
| ENSG00000152234.15_3 | NA | NA | ATP5A1 | 0,03289681 | -0,53786399 | protein_coding | protein coding | NA | NA |
| ENSG00000106089.11_2 | NA | NA | STX1A | 0,03295897 | 0,56963179 | protein_coding | protein coding | NA | NA |
| ENSG00000205358.3_2 | NA | NA | MT1H | 0,03296089 | -0,80474821 | protein_coding | protein coding | NA | NA |
| ENSG00000139182.13_2 | NA | NA | CLSTN3 | 0,03302123 | 0,62560923 | protein_coding | protein coding | NA | NA |
| ENSG00000121067.17_3 | NA | NA | SPOP | 0,03310341 | -0,59057456 | protein_coding | protein coding | NA | NA |
| ENSG00000233937.6_4 | NA | NA | AC008443.1 | 0,03313742 | 0,49499538 | processed_transcript | lncRNA | NA | NA |
| ENSG00000075131.9_4 | NA | NA | TIPIN | 0,0331507 | 0,59600047 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000110057.7_3 | NA | NA | UNC93B1 | 0,03315464 | 0,536222 | protein_coding | protein coding | NA | NA |
| ENSG00000155506.16_3 | NA | NA | LARP1 | 0,0331634 | 0,66754415 | protein_coding | protein coding | NA | NA |
| ENSG00000226800.5_3 | NA | NA | CACTIN-AS1 | 0,03317687 | 0,54254571 | antisense_RNA | lncRNA | NA | NA |
| ENSG00000205808.5_2 | NA | NA | PLPP6 | 0,03320319 | -0,69051084 | protein_coding | protein coding | NA | NA |
| ENSG00000167513.8_3 | NA | NA | CDT1 | 0,033238 | 0,5956013 | protein_coding | protein coding | NA | NA |
| ENSG00000145016.15_4 | NA | NA | RUBCN | 0,0332393 | 0,52278914 | protein_coding | protein coding | NA | NA |
| ENSG00000189157.13_4 | NA | NA | FAM47E | 0,0332983 | 0,3883681 | protein_coding | protein coding | NA | NA |
| ENSG00000134759.13_2 | NA | NA | ELP2 | 0,03330987 | -0,52221805 | protein_coding | protein coding | NA | NA |
| ENSG00000144339.11_2 | NA | NA | TMEFF2 | 0,03337335 | -0,57632137 | protein_coding | protein coding | NA | NA |
| ENSG00000162576.16_2 | NA | NA | MXRA8 | 0,03339802 | 0,54698244 | protein_coding | protein coding | NA | NA |
| ENSG00000105655.18_3 | NA | NA | ISYNA1 | 0,03341815 | 0,58813401 | protein_coding | protein coding | NA | NA |
| ENSG00000088035.15_2 | NA | NA | ALG6 | 0,03343025 | -0,57706843 | protein_coding | protein coding | NA | NA |
| ENSG00000103479.15_3 | NA | NA | RBL2 | 0,0334551 | -0,597125 | protein_coding | protein coding | NA | NA |
| ENSG00000156970.12_3 | NA | NA | BUB1B | 0,03346272 | 0,3853645 | protein_coding | protein coding | NA | NA |
| ENSG00000082175.14_2 | NA | NA | PGR | 0,03348957 | -0,58545653 | protein_coding | protein coding | NA | NA |
| ENSG00000185591.9_3 | NA | NA | SP1 | 0,03351015 | 0,63667745 | protein_coding | protein coding | NA | NA |
| ENSG00000160392.13_4 | NA | NA | C19orf47 | 0,03364576 | 0,59976512 | protein_coding | protein coding | NA | NA |
| ENSG00000156531.16_2 | NA | NA | PHF6 | 0,03368288 | 0,48623402 | protein_coding | protein coding | NA | NA |
| ENSG00000134072.10_2 | NA | NA | CAMK1 | 0,03371411 | -0,66884809 | protein_coding | protein coding | NA | NA |
| ENSG00000092531.9_2 | NA | NA | SNAP23 | 0,03371987 | -0,58969605 | protein_coding | protein coding | NA | NA |
| ENSG00000196150.13_3 | NA | NA | ZNF250 | 0,03373485 | 0,47950793 | protein_coding | protein coding | NA | NA |
| ENSG00000113739.10_2 | NA | NA | STC2 | 0,03383954 | 0,46822559 | protein_coding | protein coding | NA | NA |
| ENSG00000152503.9_3 | NA | NA | TRIM36 | 0,03385466 | -0,5183856 | protein_coding | protein coding | NA | NA |
| ENSG00000105519.15_3 | NA | NA | CAPS | 0,03388049 | 0,53299735 | protein_coding | protein coding | NA | NA |
| ENSG00000171812.12_3 | NA | NA | COL8A2 | 0,03391594 | 0,52557219 | protein_coding | protein coding | NA | NA |
| ENSG00000244122.2_3 | NA | NA | UGT1A7 | 0,03396237 | 0,38733385 | protein_coding | protein coding | NA | NA |
| ENSG00000158470.5_2 | NA | NA | B4GALT5 | 0,03396786 | 0,62225063 | protein_coding | protein coding | NA | NA |
| ENSG00000127946.16_3 | NA | NA | HIP1 | 0,0340329 | 0,59409422 | protein_coding | protein coding | NA | NA |
| ENSG00000104979.8_3 | NA | NA | C19orf53 | 0,03407719 | 0,63722372 | protein_coding | protein coding | NA | NA |
| ENSG00000243431.1_2 | NA | NA | RPL5P30 | 0,03408099 | -0,54337504 | processed_pseudogene | pseudogene | NA | NA |
| ENSG00000143575.14_3 | NA | NA | HAX1 | 0,03411423 | 0,55641088 | protein_coding | protein coding | NA | NA |
| ENSG00000163635.17_3 | NA | NA | ATXN7 | 0,03414696 | 0,5720323 | protein_coding | protein coding | NA | NA |
| ENSG00000188747.8_4 | NA | NA | NOXA1 | 0,03421418 | 0,59669745 | protein_coding | protein coding | NA | NA |
| ENSG00000272634.2_3 | NA | NA | AC103710.2 | 0,0342554 | -0,69041678 | transcribed_unprocessed_pseudogene | pseudogene | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000077454.15_3 | NA | NA | LRCH4 | 0,03429013 | 0,60016503 | protein_coding | protein coding | NA | NA |
| ENSG00000168491.9_3 | NA | NA | CCDC110 | 0,03429382 | -0,59439189 | protein_coding | protein coding | NA | NA |
| ENSG00000111912.19_4 | NA | NA | NCOA7 | 0,03431553 | 0,59350338 | protein_coding | protein coding | NA | NA |
| ENSG00000145536.15_2 | NA | NA | ADAMTS16 | 0,03447507 | 0,54104665 | protein_coding | protein coding | NA | NA |
| ENSG00000051341.13_2 | NA | NA | POLQ | 0,03452949 | 0,44124708 | protein_coding | protein coding | NA | NA |
| ENSG00000144535.19_3 | NA | NA | DIS3L2 | 0,03459052 | 0,55685227 | protein_coding | protein coding | NA | NA |
| ENSG00000152931.7_2 | NA | NA | PART1 | 0,03460426 | -0,70641258 | lincRNA | lncRNA | NA | NA |
| ENSG00000170876.7_3 | NA | NA | TMEM43 | 0,03462507 | 0,58571825 | protein_coding | protein coding | NA | NA |
| ENSG00000163462.17_2 | NA | NA | TRIM46 | 0,0346284 | 0,55278855 | protein_coding | protein coding | NA | NA |
| ENSG00000163482.11_3 | NA | NA | STK36 | 0,03466833 | 0,60380021 | protein_coding | protein coding | NA | NA |
| ENSG00000226756.1_3 | NA | NA | AC007365.1 | 0,03468148 | 0,4352907 | lincRNA | lncRNA | NA | NA |
| ENSG00000214106.7_3 | NA | NA | PAXIP1-AS2 | 0,03469888 | 0,55701929 | antisense_RNA | lncRNA | NA | NA |
| ENSG00000266865.6_3 | NA | NA | AC138207.8 | 0,03474231 | -0,58277391 | transcribed_unprocessed_pseudogene | pseudogene | NA | NA |
| ENSG00000137221.14_3 | NA | NA | TJAP1 | 0,03475016 | 0,60555943 | protein_coding | protein coding | NA | NA |
| ENSG00000266094.7_3 | NA | NA | RASSF5 | 0,03475023 | 0,54230401 | protein_coding | protein coding | NA | NA |
| ENSG00000168010.10_3 | NA | NA | ATG16L2 | 0,034772 | 0,59680098 | protein_coding | protein coding | NA | NA |
| ENSG00000214078.12_3 | NA | NA | CPNE1 | 0,03483887 | 0,57056439 | protein_coding | protein coding | NA | NA |
| ENSG00000118257.16_3 | NA | NA | NRP2 | 0,03495631 | 0,56280837 | protein_coding | protein coding | NA | NA |
| ENSG00000076928.17_3 | NA | NA | ARHGEF1 | 0,03497853 | 0,57861561 | protein_coding | protein coding | NA | NA |
| ENSG00000111670.14_3 | NA | NA | GNPTAB | 0,03498855 | 0,55917264 | protein_coding | protein coding | NA | NA |
| ENSG00000086065.13_2 | NA | NA | CHMP5 | 0,03502858 | -0,58919553 | protein_coding | protein coding | NA | NA |
| ENSG00000106266.10_3 | NA | NA | SNX8 | 0,03504772 | 0,58094655 | protein_coding | protein coding | NA | NA |
| ENSG00000171681.12_3 | NA | NA | ATF7IP | 0,03506017 | 0,51219739 | protein_coding | protein coding | NA | NA |
| ENSG00000151148.13_2 | NA | NA | UBE3B | 0,03521365 | 0,62480752 | protein_coding | protein coding | NA | NA |
| ENSG00000135632.11_2 | NA | NA | SMYD5 | 0,03523707 | 0,57535756 | protein_coding | protein coding | NA | NA |
| ENSG00000147655.10_2 | NA | NA | RSPO2 | 0,03527136 | -0,57874969 | protein_coding | protein coding | NA | NA |
| ENSG00000122642.10_2 | NA | NA | FKBP9 | 0,03532173 | 0,59143127 | protein_coding | protein coding | NA | NA |
| ENSG00000254812.1_3 | NA | NA | AC067930.3 | 0,035337 | 0,56174321 | antisense_RNA | lncRNA | NA | NA |
| ENSG00000119640.8_2 | NA | NA | ACYP1 | 0,03534903 | 0,58140735 | protein_coding | protein coding | NA | NA |
| ENSG00000241839.9_3 | NA | NA | PLEKHO2 | 0,03545073 | 0,57751394 | protein_coding | protein coding | NA | NA |
| ENSG00000184838.14_3 | NA | NA | PRR16 | 0,0354631 | 0,54578153 | protein_coding | protein coding | NA | NA |
| ENSG00000139437.17_3 | NA | NA | TCHP | 0,03546918 | 0,63600397 | protein_coding | protein coding | NA | NA |
| ENSG00000138071.13_3 | NA | NA | ACTR2 | 0,03547089 | 0,60751723 | protein_coding | protein coding | NA | NA |
| ENSG00000178537.9_2 | NA | NA | SLC25A20 | 0,03547867 | -0,55175277 | protein_coding | protein coding | NA | NA |
| ENSG00000133247.13_3 | NA | NA | KMT5C | 0,03548888 | 0,49385028 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000122861.15_2 | NA | NA | PLAU | 0,03553117 | 0,5794612 | protein_coding | protein coding | NA | NA |
| ENSG00000168140.4_2 | NA | NA | VASN | 0,03558919 | 0,57976452 | protein_coding | protein coding | NA | NA |
| ENSG00000198169.8_2 | NA | NA | ZNF251 | 0,03563225 | 0,47634292 | protein_coding | protein coding | NA | NA |
| ENSG00000050130.17_2 | NA | NA | JKAMP | 0,03568348 | -0,61452964 | protein_coding | protein coding | NA | NA |
| ENSG00000140750.16_3 | NA | NA | ARHGAP17 | 0,03571578 | 0,64885991 | protein_coding | protein coding | NA | NA |
| ENSG00000141293.15_3 | NA | NA | SKAP1 | 0,03573767 | 0,5369643 | protein_coding | protein coding | NA | NA |
| ENSG00000008710.19_3 | NA | NA | PKD1 | 0,03576094 | 0,59020112 | protein_coding | protein coding | NA | NA |
| ENSG00000112290.12_3 | NA | NA | WASF1 | 0,03577085 | 0,61842092 | protein_coding | protein coding | NA | NA |
| ENSG00000169896.16_2 | NA | NA | ITGAM | 0,0357972 | 0,65782861 | protein_coding | protein coding | NA | NA |
| ENSG00000183624.13_2 | NA | NA | HMCES | 0,03583195 | 0,59338665 | protein_coding | protein coding | NA | NA |
| ENSG00000282851.1_3 | NA | NA | BISPR | 0,0358696 | 0,32801611 | lincRNA | lncRNA | NA | NA |
| ENSG00000171574.17_3 | NA | NA | ZNF584 | 0,0359738 | 0,53602515 | protein_coding | protein coding | NA | NA |
| ENSG00000133624.13_2 | NA | NA | ZNF767P | 0,03599314 | 0,58272625 | transcribed_unprocessed_pseudogene | pseudogene | NA | NA |
| ENSG00000105325.13_2 | NA | NA | FZR1 | 0,03602921 | 0,61949309 | protein_coding | protein coding | NA | NA |
| ENSG00000242173.8_3 | NA | NA | ARHGDIG | 0,03605625 | 0,44257645 | protein_coding | protein coding | NA | NA |
| ENSG00000164855.15_2 | NA | NA | TMEM184A | 0,03617302 | 0,44079354 | protein_coding | protein coding | NA | NA |
| ENSG00000133858.15_2 | NA | NA | ZFC3H1 | 0,03617507 | 0,60602823 | protein_coding | protein coding | NA | NA |
| ENSG00000106617.13_3 | NA | NA | PRKAG2 | 0,03618112 | 0,42098512 | protein_coding | protein coding | NA | NA |
| ENSG00000236753.5_2 | NA | NA | MKLN1-AS | 0,0362039 | -0,68411961 | processed_transcript | lncRNA | NA | NA |
| ENSG00000102265.11_2 | NA | NA | TIMP1 | 0,03629698 | 0,57536167 | protein_coding | protein coding | NA | NA |
| ENSG00000163781.12_3 | NA | NA | TOPBP1 | 0,03632671 | 0,57010144 | protein_coding | protein coding | NA | NA |
| ENSG00000102886.14_2 | NA | NA | GDPD3 | 0,03639687 | 0,56233513 | protein_coding | protein coding | NA | NA |
| ENSG00000092108.20_4 | NA | NA | SCFD1 | 0,0364329 | -0,57817197 | protein_coding | protein coding | NA | NA |
| ENSG00000179097.5_2 | NA | NA | HTR1F | 0,03643776 | 0,35180961 | protein_coding | protein coding | NA | NA |
| ENSG00000165271.16_3 | NA | NA | NOL6 | 0,03652178 | 0,55446277 | protein_coding | protein coding | NA | NA |
| ENSG00000177302.14_3 | NA | NA | TOP3A | 0,03655726 | 0,57838944 | protein_coding | protein coding | NA | NA |
| ENSG00000151014.5_2 | NA | NA | NOCT | 0,0366118 | 0,58390988 | protein_coding | protein coding | NA | NA |
| ENSG00000086544.2_2 | NA | NA | ITPKC | 0,0366138 | 0,50540905 | protein_coding | protein coding | NA | NA |
| ENSG00000129128.12_2 | NA | NA | SPCS3 | 0,03665849 | -0,58623214 | protein_coding | protein coding | NA | NA |
| ENSG00000229689.3_3 | NA | NA | AC009237.3 | 0,03666771 | 0,47672888 | transcribed_unprocessed_pseudogene | pseudogene | NA | NA |
| ENSG00000260236.1_3 | NA | NA | AC099778.1 | 0,03668426 | 0,58933565 | antisense_RNA | lncRNA | NA | NA |
| ENSG00000244165.1_3 | NA | NA | P2RY11 | 0,03669333 | 0,54623097 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000236947.5_3 | NA | NA | AL139412.1 | 0,03670268 | 0,54350856 | lincRNA | lncRNA | NA | NA |
| ENSG00000181754.6_2 | NA | NA | AMIGO1 | 0,03671638 | -0,58793117 | protein_coding | protein coding | NA | NA |
| ENSG00002236756.4_4 | NA | NA | DNAJC9-AS1 | 0,03672237 | 0,4893721 | antisense_RNA | lncRNA | NA | NA |
| ENSG00000086189.9_2 | NA | NA | DIMT1 | 0,03674334 | -0,54172509 | protein_coding | protein coding | NA | NA |
| ENSG00000102738.7_2 | NA | NA | MRPS31 | 0,03674337 | -0,5505947 | protein_coding | protein coding | NA | NA |
| ENSG00000111247.14_2 | NA | NA | RAD51AP1 | 0,03677371 | 0,60238465 | protein_coding | protein coding | NA | NA |
| ENSG00000130813.17_4 | NA | NA | C19orf66 | 0,03681976 | 0,53789546 | protein_coding | protein coding | NA | NA |
| ENSG00000241158.5_4 | NA | NA | ADAMTS9-AS1 | 0,03687977 | -0,61314211 | antisense_RNA | lncRNA | NA | NA |
| ENSG00000172007.5_2 | NA | NA | RAB33B | 0,03701568 | 0,59223109 | protein_coding | protein coding | NA | NA |
| ENSG00000138246.15_3 | NA | NA | DNAJC13 | 0,03703936 | 0,52785749 | protein_coding | protein coding | NA | NA |
| ENSG00000075702.16_3 | NA | NA | WDR62 | 0,03707026 | 0,61251575 | protein_coding | protein coding | NA | NA |
| ENSG00000213478.2_2 | NA | NA | CFL1P2 | 0,0370711 | 0,50698168 | processed_pseudogene | pseudogene | NA | NA |
| ENSG00000008517.16_4 | NA | NA | IL32 | 0,03709991 | 0,58784403 | protein_coding | protein coding | NA | NA |
| ENSG00000049759.17_3 | NA | NA | NEDD4L | 0,0371018 | -0,5725498 | protein_coding | protein coding | NA | NA |
| ENSG00000167749.11_2 | NA | NA | KLK4 | 0,0371037 | -0,58504135 | protein_coding | protein coding | NA | NA |
| ENSG00000165782.10_2 | NA | NA | TMEM55B | 0,03723996 | 0,60794413 | protein_coding | protein coding | NA | NA |
| ENSG00000196236.12_2 | NA | NA | XPNPEP3 | 0,03727767 | 0,56242545 | protein_coding | protein coding | NA | NA |
| ENSG00000055147.18_3 | NA | NA | FAM114A2 | 0,03734111 | -0,59324154 | protein_coding | protein coding | NA | NA |
| ENSG00000132003.9_3 | NA | NA | ZSWIM4 | 0,03734693 | 0,46754301 | protein_coding | protein coding | NA | NA |
| ENSG00000140320.11_3 | NA | NA | BAHD1 | 0,03735441 | 0,54695579 | protein_coding | protein coding | NA | NA |
| ENSG00000113194.12_3 | NA | NA | FAF2 | 0,03740828 | 0,52977617 | protein_coding | protein coding | NA | NA |
| ENSG00000108771.12_3 | NA | NA | DHX58 | 0,03749303 | 0,45864612 | protein_coding | protein coding | NA | NA |
| ENSG00000076108.11_2 | NA | NA | BAZ2A | 0,03749768 | 0,61292508 | protein_coding | protein coding | NA | NA |
| ENSG00000243766.7_3 | NA | NA | HOTTIP | 0,037578 | 0,5232374 | antisense_RNA | lncRNA | NA | NA |
| ENSG00000160908.14_2 | NA | NA | ZNF394 | 0,0375997 | 0,42384686 | protein_coding | protein coding | NA | NA |
| ENSG00000124496.12_2 | NA | NA | TRERF1 | 0,03767894 | 0,54421518 | protein_coding | protein coding | NA | NA |
| ENSG00000139514.12_3 | NA | NA | SLC7A1 | 0,03768051 | -0,62765273 | protein_coding | protein coding | NA | NA |
| ENSG00000001629.9_3 | NA | NA | ANKIB1 | 0,03773449 | 0,52394791 | protein_coding | protein coding | NA | NA |
| ENSG00000161013.16_3 | NA | NA | MGAT4B | 0,03778612 | 0,65858823 | protein_coding | protein coding | NA | NA |
| ENSG00000135968.20_3 | NA | NA | GCC2 | 0,03789313 | -0,5420816 | protein_coding | protein coding | NA | NA |
| ENSG00000163795.13_2 | NA | NA | ZNF513 | 0,03791077 | 0,59318218 | protein_coding | protein coding | NA | NA |
| ENSG00000106665.15_2 | NA | NA | CLIP2 | 0,03792052 | 0,61953486 | protein_coding | protein coding | NA | NA |
| ENSG00000099725.14_2 | NA | NA | PRKY | 0,0379288 | 0,58650749 | transcribed_unprocessed_pseudogene | pseudogene | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000100207.18_4 | NA | NA | TCF20 | 0,03795811 | 0,61036146 | protein_coding | protein coding | NA | NA |
| ENSG00000187017.15_3 | NA | NA | ESPN | 0,03796751 | 0,48584117 | protein_coding | protein coding | NA | NA |
| ENSG00000143333.6_2 | NA | NA | RGS16 | 0,03801061 | 0,52162389 | protein_coding | protein coding | NA | NA |
| ENSG00000132872.11_2 | NA | NA | SYT4 | 0,03801195 | 0,45063739 | protein_coding | protein coding | NA | NA |
| ENSG00000106086.18_3 | NA | NA | PLEKHA8 | 0,03804245 | 0,57572959 | protein_coding | protein coding | NA | NA |
| ENSG00000108821.13_3 | NA | NA | COL1A1 | 0,03808903 | 0,558091 | protein_coding | protein coding | NA | NA |
| ENSG00000104549.11_2 | NA | NA | SQLE | 0,0381199 | 0,59289964 | protein_coding | protein coding | NA | NA |
| ENSG00000173207.12_2 | NA | NA | CKS1B | 0,03814795 | 0,55163107 | protein_coding | protein coding | NA | NA |
| ENSG00000142208.15_3 | NA | NA | AKT1 | 0,03819994 | 0,53762005 | protein_coding | protein coding | NA | NA |
| ENSG00000141198.15_4 | NA | NA | TOM1L1 | 0,03825994 | -0,48497264 | protein_coding | protein coding | NA | NA |
| ENSG00000198742.9_2 | NA | NA | SMURF1 | 0,03826761 | 0,61287012 | protein_coding | protein coding | NA | NA |
| ENSG00000213626.11_2 | NA | NA | LBH | 0,03828355 | 0,48347516 | protein_coding | protein coding | NA | NA |
| ENSG00000174740.7_3 | NA | NA | PABPC5 | 0,03830085 | -0,61281005 | protein_coding | protein coding | NA | NA |
| ENSG00000249307.5_3 | NA | NA | LINC01088 | 0,03833474 | -0,92155985 | antisense_RNA | lncRNA | NA | NA |
| ENSG00000054965.10_2 | NA | NA | FAM168A | 0,03838686 | 0,56013619 | protein_coding | protein coding | NA | NA |
| ENSG00000196235.13_3 | NA | NA | SUPT5H | 0,03840134 | 0,62808898 | protein_coding | protein coding | NA | NA |
| ENSG00000086991.12_2 | NA | NA | NOX4 | 0,03840551 | 0,55003108 | protein_coding | protein coding | NA | NA |
| ENSG00000106100.10_2 | NA | NA | NOD1 | 0,03841251 | 0,4463129 | protein_coding | protein coding | NA | NA |
| ENSG00000131652.13_2 | NA | NA | THOC6 | 0,03841724 | 0,55593822 | protein_coding | protein coding | NA | NA |
| ENSG00000182472.8_3 | NA | NA | CAPN12 | 0,03848692 | 0,60215076 | protein_coding | protein coding | NA | NA |
| ENSG00000157954.14_2 | NA | NA | WIPI2 | 0,03857365 | 0,52618957 | protein_coding | protein coding | NA | NA |
| ENSG00000126709.14_2 | NA | NA | IFI6 | 0,03860355 | 0,42852386 | protein_coding | protein coding | NA | NA |
| ENSG00000186638.16_3 | NA | NA | KIF24 | 0,03864384 | 0,55898881 | protein_coding | protein coding | NA | NA |
| ENSG00000180182.10_2 | NA | NA | MED14 | 0,03871232 | 0,52134285 | protein_coding | protein coding | NA | NA |
| ENSG00000106105.13_2 | NA | NA | GARS | 0,03871961 | 0,52805555 | protein_coding | protein coding | NA | NA |
| ENSG00000102580.14_3 | NA | NA | DNAJC3 | 0,03874447 | -0,50982922 | protein_coding | protein coding | NA | NA |
| ENSG00000134058.11_3 | NA | NA | CDK7 | 0,03876143 | -0,50001611 | protein_coding | protein coding | NA | NA |
| ENSG00000269313.5_2 | NA | NA | MAGIX | 0,03886288 | 0,54889672 | protein_coding | protein coding | NA | NA |
| ENSG00000132793.11_2 | NA | NA | LPIN3 | 0,03890605 | 0,55560149 | protein_coding | protein coding | NA | NA |
| ENSG00000104518.10_3 | NA | NA | GSDMD | 0,03897151 | 0,65896529 | protein_coding | protein coding | NA | NA |
| ENSG00000043143.20_2 | NA | NA | JADE2 | 0,03906426 | 0,57571794 | protein_coding | protein coding | NA | NA |
| ENSG00000197822.10_2 | NA | NA | OCLN | 0,03908854 | 0,55081533 | protein_coding | protein coding | NA | NA |
| ENSG00000117713.18_3 | NA | NA | ARID1A | 0,03910578 | 0,75128133 | protein_coding | protein coding | NA | NA |
| ENSG00000122691.12_3 | NA | NA | TWIST1 | 0,0391233 | 0,403622 | protein_coding | protein coding | NA | NA |
| ENSG00000177963.14_4 | NA | NA | RIC8A | 0,0391581 | 0,57902592 | protein_coding | protein coding | NA | NA |
| ENSG00000144354.13_2 | NA | NA | CDCA7 | 0,0391659 | 0,48310391 | protein_coding | protein coding | NA | NA |
| ENSG00000105122.12_4 | NA | NA | RASAL3 | 0,03922798 | 0,54714839 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000008838.19_3 | NA | NA | MED24 | 0,03923508 | 0,6123147 | protein_coding | protein coding | NA | NA |
| ENSG00000141424.12_3 | NA | NA | SLC39A6 | 0,03931517 | -0,53223222 | protein_coding | protein coding | NA | NA |
| ENSG00000136811.16_4 | NA | NA | ODF2 | 0,03933514 | 0,52271285 | protein_coding | protein coding | NA | NA |
| ENSG00000172572.6_3 | NA | NA | PDE3A | 0,03934862 | 0,44341044 | protein_coding | protein coding | NA | NA |
| ENSG00000064763.10_3 | NA | NA | FAR2 | 0,03944758 | 0,38986676 | protein_coding | protein coding | NA | NA |
| ENSG00000050327.14_3 | NA | NA | ARHGEF5 | 0,03950889 | 0,48609309 | protein_coding | protein coding | NA | NA |
| ENSG00000067365.14_3 | NA | NA | METTL22 | 0,03958639 | 0,52656922 | protein_coding | protein coding | NA | NA |
| ENSG00000162493.16_3 | NA | NA | PDPN | 0,03958997 | 0,54440749 | protein_coding | protein coding | NA | NA |
| ENSG00000149218.4_2 | NA | NA | ENDOD1 | 0,03966382 | -0,65574584 | protein_coding | protein coding | NA | NA |
| ENSG00000147889.17_3 | NA | NA | CDKN2A | 0,03968622 | 0,50247715 | protein_coding | protein coding | NA | NA |
| ENSG00000198826.10_3 | NA | NA | ARHGAP11A | 0,03974364 | 0,55330203 | protein_coding | protein coding | NA | NA |
| ENSG00000172057.9_3 | NA | NA | ORMDL3 | 0,03976183 | 0,59497116 | protein_coding | protein coding | NA | NA |
| ENSG00000130827.6_2 | NA | NA | PLXNA3 | 0,03977412 | 0,57508259 | protein_coding | protein coding | NA | NA |
| ENSG00000187037.8_3 | NA | NA | GPR141 | 0,03979 | 0,58946593 | protein_coding | protein coding | NA | NA |
| ENSG00000244038.9_4 | NA | NA | DDOST | 0,03980095 | -0,54122615 | protein_coding | protein coding | NA | NA |
| ENSG00000213462.4_3 | NA | NA | ERV3-1 | 0,03980949 | 0,50704785 | protein_coding | protein coding | NA | NA |
| ENSG00000124920.13_3 | NA | NA | MYRF | 0,03981388 | 0,50260068 | protein_coding | protein coding | NA | NA |
| ENSG00000105778.18_2 | NA | NA | AVL9 | 0,03996632 | 0,58410736 | protein_coding | protein coding | NA | NA |
| ENSG00000108924.13_3 | NA | NA | HLF | 0,03996765 | -0,62860882 | protein_coding | protein coding | NA | NA |
| ENSG00000185730.7_3 | NA | NA | ZNF696 | 0,04005099 | 0,52775349 | protein_coding | protein coding | NA | NA |
| ENSG00000186312.10_2 | NA | NA | CA5BP1 | 0,04011527 | 0,50757144 | transcribed_unprocessed_pseudogene | pseudogene | NA | NA |
| ENSG00000148300.11_2 | NA | NA | REXO4 | 0,04015416 | 0,49117655 | protein_coding | protein coding | NA | NA |
| ENSG00000160299.16_2 | NA | NA | PCNT | 0,04022685 | 0,61572848 | protein_coding | protein coding | NA | NA |
| ENSG00000144909.7_2 | NA | NA | OSBPL11 | 0,04032208 | 0,59879644 | protein_coding | protein coding | NA | NA |
| ENSG00000168256.17_3 | NA | NA | NKIRAS2 | 0,04037796 | 0,61426219 | protein_coding | protein coding | NA | NA |
| ENSG00000136159.3_2 | NA | NA | NUDT15 | 0,04039098 | -0,57319084 | protein_coding | protein coding | NA | NA |
| ENSG00000130560.8_2 | NA | NA | UBAC1 | 0,04053632 | 0,49082535 | protein_coding | protein coding | NA | NA |
| ENSG00000012048.20_3 | NA | NA | BRCA1 | 0,040556 | 0,397942 | protein_coding | protein coding | NA | NA |
| ENSG00000248801.6_3 | NA | NA | C8orf34-AS1 | 0,04057565 | -0,69996779 | antisense_RNA | lncRNA | NA | NA |
| ENSG00000186174.12_2 | NA | NA | BCL9L | 0,04064385 | 0,56365557 | protein_coding | protein coding | NA | NA |
| ENSG00000141646.13_3 | NA | NA | SMAD4 | 0,04073871 | -0,55178947 | protein_coding | protein coding | NA | NA |
| ENSG00000085365.17_2 | NA | NA | SCAMP1 | 0,04077739 | -0,44062788 | protein_coding | protein coding | NA | NA |
| ENSG00000159763.3_2 | NA | NA | PIP | 0,04079963 | 0,2591827 | protein_coding | protein coding | NA | NA |
| ENSG00000171551.11_2 | NA | NA | ECEL1 | 0,04084878 | 0,57947202 | protein_coding | protein coding | NA | NA |
| ENSG00000235398.4 | NA | NA | LINC00623 | 0,04084897 | 0,5379806 | lincRNA | lncRNA | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000213601.3_2 | NA | NA | KRT18P19 | 0,04089148 | -0,6285447 | processed_pseudogene | pseudogene | NA | NA |
| ENSG00000149243.15_2 | NA | NA | KLHL35 | 0,04092809 | 0,50629791 | protein_coding | protein coding | NA | NA |
| ENSG00000227248.1_3 | NA | NA | FAM155A-IT1 | 0,04093966 | 0,54522882 | sense_intronic | lncRNA | NA | NA |
| ENSG00000163499.11_2 | NA | NA | CRYBA2 | 0,04117045 | 0,48371671 | protein_coding | protein coding | NA | NA |
| ENSG00000198546.14_3 | NA | NA | ZNF511 | 0,04117449 | -0,54630504 | protein_coding | protein coding | NA | NA |
| ENSG00000162889.10_2 | NA | NA | MAPKAPK2 | 0,04120341 | 0,53646505 | protein_coding | protein coding | NA | NA |
| ENSG00000139433.9_3 | NA | NA | GLTP | 0,04120713 | 0,59343763 | protein_coding | protein coding | NA | NA |
| ENSG00000184209.14_3 | NA | NA | SNRNP35 | 0,04125834 | 0,60830686 | protein_coding | protein coding | NA | NA |
| ENSG00000130487.5_2 | NA | NA | KLHDC7B | 0,04130996 | 0,3897293 | protein_coding | protein coding | NA | NA |
| ENSG00000153827.13_3 | NA | NA | TRIP12 | 0,04139623 | 0,60363524 | protein_coding | protein coding | NA | NA |
| ENSG00000124780.13_2 | NA | NA | KCNK17 | 0,0414536 | 0,50376508 | protein_coding | protein coding | NA | NA |
| ENSG00000196428.12_2 | NA | NA | TSC22D2 | 0,04150827 | 0,54147315 | protein_coding | protein coding | NA | NA |
| ENSG00000174943.9_4 | NA | NA | KCTD13 | 0,04152621 | 0,47597248 | protein_coding | protein coding | NA | NA |
| ENSG00000136546.13_2 | NA | NA | SCN7A | 0,04153141 | -0,57698206 | protein_coding | protein coding | NA | NA |
| ENSG00000143149.12_2 | NA | NA | ALDH9A1 | 0,04162151 | -0,51866797 | protein_coding | protein coding | NA | NA |
| ENSG00000119917.13_2 | NA | NA | IFIT3 | 0,04171614 | 0,37986948 | protein_coding | protein coding | NA | NA |
| ENSG00000157837.15_3 | NA | NA | SPPL3 | 0,04180783 | 0,60708774 | protein_coding | protein coding | NA | NA |
| ENSG00000160633.12_2 | NA | NA | SAFB | 0,04181339 | 0,54170373 | protein_coding | protein coding | NA | NA |
| ENSG00000130175.9_4 | NA | NA | PRKCSH | 0,04185154 | 0,56968592 | protein_coding | protein coding | NA | NA |
| ENSG00000205045.8_3 | NA | NA | SLFN12L | 0,0418885 | 0,51474565 | protein_coding | protein coding | NA | NA |
| ENSG00000116209.11_3 | NA | NA | TMEM59 | 0,04191512 | -0,54400011 | protein_coding | protein coding | NA | NA |
| ENSG00000052126.14_3 | NA | NA | PLEKHA5 | 0,04194622 | 0,35401683 | protein_coding | protein coding | NA | NA |
| ENSG00000198276.15_3 | NA | NA | UCKL1 | 0,04195005 | 0,4996659 | protein_coding | protein coding | NA | NA |
| ENSG00000168907.13_2 | NA | NA | PLA2G4F | 0,04201467 | -0,59745136 | protein_coding | protein coding | NA | NA |
| ENSG00000204160.11_3 | NA | NA | ZDHHC18 | 0,04203698 | 0,53698059 | protein_coding | protein coding | NA | NA |
| ENSG00000139880.19_2 | NA | NA | CDH24 | 0,04210717 | 0,5997622 | protein_coding | protein coding | NA | NA |
| ENSG00000130589.16_2 | NA | NA | HELZ2 | 0,04212498 | 0,52846755 | protein_coding | protein coding | NA | NA |
| ENSG00000006194.9_3 | NA | NA | ZNF263 | 0,04212842 | 0,53826492 | protein_coding | protein coding | NA | NA |
| ENSG00000118507.15_3 | NA | NA | AKAP7 | 0,04214879 | -0,67833933 | protein_coding | protein coding | NA | NA |
| ENSG00000178104.19_3 | NA | NA | PDE4DIP | 0,04216934 | 0,54011408 | protein_coding | protein coding | NA | NA |
| ENSG00000283703.1_2 | NA | NA | VSIG10L2 | 0,0421821 | 0,42866427 | protein_coding | protein coding | NA | NA |
| ENSG00000131788.15_3 | NA | NA | PIAS3 | 0,04222736 | 0,50213032 | protein_coding | protein coding | NA | NA |
| ENSG00000123485.11_3 | NA | NA | HJURP | 0,04228382 | 0,52255575 | protein_coding | protein coding | NA | NA |
| ENSG00000008086.11_3 | NA | NA | CDKL5 | 0,04233167 | -0,55202376 | protein_coding | protein coding | NA | NA |
| ENSG00000183549.10_2 | NA | NA | ACSM5 | 0,0423805 | 0,56976839 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000267702.1_4 | NA | NA | AP005131.6 | 0,04240401 | 0,46873777 | sense_intronic | lncRNA | NA | NA |
| ENSG00000149328.14_2 | NA | NA | GLB1L2 | 0,04254419 | -0,57833784 | protein_coding | protein coding | NA | NA |
| ENSG00000133422.12_2 | NA | NA | MORC2 | 0,04273884 | 0,56686944 | protein_coding | protein coding | NA | NA |
| ENSG00000162910.18_2 | NA | NA | MRPL55 | 0,042786 | 0,51801449 | protein_coding | protein coding | NA | NA |
| ENSG00000034713.7_2 | NA | NA | GABARAPL2 | 0,0428148 | -0,58444492 | protein_coding | protein coding | NA | NA |
| ENSG00000143799.12_2 | NA | NA | PARP1 | 0,04282997 | 0,54295465 | protein_coding | protein coding | NA | NA |
| ENSG00000133027.17_2 | NA | NA | PEMT | 0,04285167 | -0,50902847 | protein_coding | protein coding | NA | NA |
| ENSG00000171067.10_3 | NA | NA | C11orf24 | 0,04287143 | 0,52724913 | protein_coding | protein coding | NA | NA |
| ENSG00000144218.18_3 | NA | NA | AFF3 | 0,04293515 | -0,730885 | protein_coding | protein coding | NA | NA |
| ENSG00000116863.10_2 | NA | NA | ADPRHL2 | 0,04295575 | 0,46980505 | protein_coding | protein coding | NA | NA |
| ENSG00000111215.11_4 | NA | NA | PRR4 | 0,04295881 | 0,56425937 | protein_coding | protein coding | NA | NA |
| ENSG00000101004.14_3 | NA | NA | NINL | 0,04301404 | 0,57699558 | protein_coding | protein coding | NA | NA |
| ENSG00000168792.4_2 | NA | NA | ABHD15 | 0,04302358 | 0,56245607 | protein_coding | protein coding | NA | NA |
| ENSG00000104973.16_3 | NA | NA | MED25 | 0,04306521 | 0,4832518 | protein_coding | protein coding | NA | NA |
| ENSG00000142002.16_2 | NA | NA | DPP9 | 0,04312632 | 0,57617541 | protein_coding | protein coding | NA | NA |
| ENSG00000148346.11_3 | NA | NA | LCN2 | 0,04316169 | -0,5399454 | protein_coding | protein coding | NA | NA |
| ENSG00000260802.1_3 | NA | NA | LINC00890 | 0,04322144 | -0,57592928 | protein_coding | protein coding | NA | NA |
| ENSG00000233708.1_3 | NA | NA | AL512353.2 | 0,04324239 | 0,61071193 | processed_pseudogene | pseudogene | NA | NA |
| ENSG00000100888.12_3 | NA | NA | CHD8 | 0,04334097 | 0,57282941 | protein_coding | protein coding | NA | NA |
| ENSG00000122585.7_2 | NA | NA | NPY | 0,04334115 | -0,57135674 | protein_coding | protein coding | NA | NA |
| ENSG00000233155.1_2 | NA | NA | HMGA1P8 | 0,04351432 | 0,54579513 | processed_pseudogene | pseudogene | NA | NA |
| ENSG00000129521.13_3 | NA | NA | EGLN3 | 0,04354547 | 0,58606808 | protein_coding | protein coding | NA | NA |
| ENSG00000101951.16_2 | NA | NA | PAGE4 | 0,04356074 | -0,60408378 | protein_coding | protein coding | NA | NA |
| ENSG00000182890.4_3 | NA | NA | GLUD2 | 0,04362604 | -0,4741927 | protein_coding | protein coding | NA | NA |
| ENSG00000104951.15_3 | NA | NA | IL4I1 | 0,0437209 | 0,51125991 | protein_coding | protein coding | NA | NA |
| ENSG00000113924.11_3 | NA | NA | HGD | 0,04374155 | -0,56697834 | protein_coding | protein coding | NA | NA |
| ENSG00000185513.14_3 | NA | NA | L3MBTL1 | 0,04374983 | 0,47140686 | protein_coding | protein coding | NA | NA |
| ENSG00000140406.3_3 | NA | NA | TLNRD1 | 0,04392099 | 0,5464601 | protein_coding | protein coding | NA | NA |
| ENSG00000007080.10_2 | NA | NA | CCDC124 | 0,04407284 | 0,5125001 | protein_coding | protein coding | NA | NA |
| ENSG00000174799.10_2 | NA | NA | CEP135 | 0,04407897 | 0,46455467 | protein_coding | protein coding | NA | NA |
| ENSG00000122515.14_3 | NA | NA | ZMIZ2 | 0,04411187 | 0,54527657 | protein_coding | protein coding | NA | NA |
| ENSG00000232855.6_4 | NA | NA | AF165147.1 | 0,04411257 | -0,6715272 | lincRNA | lncRNA | NA | NA |
| ENSG00000061273.17_3 | NA | NA | HDAC7 | 0,04411264 | 0,55292174 | protein_coding | protein coding | NA | NA |
| ENSG00000262454.3_3 | NA | NA | MIR193BHG | 0,04417005 | 0,38553562 | lincRNA | lncRNA | NA | NA |
| ENSG00000160072.19_2 | NA | NA | ATAD3B | 0,04421083 | 0,55431071 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000183647.10_2 | NA | NA | ZNF530 | 0,04426665 | 0,54661683 | protein_coding | protein coding | NA | NA |
| ENSG00000127084.17_2 | NA | NA | FGD3 | 0,04433056 | 0,49516528 | protein_coding | protein coding | NA | NA |
| ENSG00000038532.14_3 | NA | NA | CLEC16A | 0,04436325 | 0,5694158 | protein_coding | protein coding | NA | NA |
| ENSG00000175115.11_3 | NA | NA | PACS1 | 0,04440557 | 0,58024845 | protein_coding | protein coding | NA | NA |
| ENSG00000167346.7_4 | NA | NA | MMP26 | 0,04466268 | -0,61223989 | protein_coding | protein coding | NA | NA |
| ENSG00000161677.11_3 | NA | NA | JOSD2 | 0,04466717 | 0,50866737 | protein_coding | protein coding | NA | NA |
| ENSG00000143862.7_2 | NA | NA | ARL8A | 0,04471169 | 0,530022 | protein_coding | protein coding | NA | NA |
| ENSG00000148384.12_3 | NA | NA | INPP5E | 0,04480854 | 0,58962395 | protein_coding | protein coding | NA | NA |
| ENSG00000132024.17_3 | NA | NA | CC2D1A | 0,04482231 | 0,63114029 | protein_coding | protein coding | NA | NA |
| ENSG00000174130.12_3 | NA | NA | TLR6 | 0,04483143 | 0,57357253 | protein_coding | protein coding | NA | NA |
| ENSG00000125970.11_4 | NA | NA | RALY | 0,04486348 | 0,43257333 | protein_coding | protein coding | NA | NA |
| ENSG00000136160.14_3 | NA | NA | EDNRB | 0,04487955 | -0,57222676 | protein_coding | protein coding | NA | NA |
| ENSG00000185800.11_3 | NA | NA | DMWD | 0,04488193 | 0,59912022 | protein_coding | protein coding | NA | NA |
| ENSG00000228140.1_3 | NA | NA | AL031283.1 | 0,04489313 | 0,44711987 | lincRNA | lncRNA | NA | NA |
| ENSG00000247095.2_2 | NA | NA | MIR210HG | 0,04495832 | 0,50077133 | lincRNA | lncRNA | NA | NA |
| ENSG00000166801.15_2 | NA | NA | FAM111A | 0,04496781 | -0,58579673 | protein_coding | protein coding | NA | NA |
| ENSG00000101096.19_2 | NA | NA | NFATC2 | 0,0451015 | 0,49206752 | protein_coding | protein coding | NA | NA |
| ENSG00000196747.4_2 | NA | NA | HIST1H2AI | 0,04514272 | 0,49857988 | protein_coding | protein coding | NA | NA |
| ENSG00000165730.14_2 | NA | NA | STOX1 | 0,04515022 | 0,48616834 | protein_coding | protein coding | NA | NA |
| ENSG00000247137.8_4 | NA | NA | AP000873.2 | 0,04528315 | 0,4618239 | processed_transcript | lncRNA | NA | NA |
| ENSG00000179241.12_3 | NA | NA | LDLRAD3 | 0,04535788 | -0,56271811 | protein_coding | protein coding | NA | NA |
| ENSG00000133106.14_3 | NA | NA | EPSTI1 | 0,04536644 | 0,42737373 | protein_coding | protein coding | NA | NA |
| ENSG00000162104.9_2 | NA | NA | ADCY9 | 0,04550633 | 0,50860777 | protein_coding | protein coding | NA | NA |
| ENSG00000205364.3_2 | NA | NA | MT1M | 0,04554112 | -0,70633092 | protein_coding | protein coding | NA | NA |
| ENSG00000087510.6_2 | NA | NA | TFAP2C | 0,04561679 | 0,53075545 | protein_coding | protein coding | NA | NA |
| ENSG00000124570.17_2 | NA | NA | SERPINB6 | 0,0457271 | -0,46741214 | protein_coding | protein coding | NA | NA |
| ENSG00000072501.17_2 | NA | NA | SMC1A | 0,04574525 | 0,48965376 | protein_coding | protein coding | NA | NA |
| ENSG00000146281.5_2 | NA | NA | PM20D2 | 0,04585028 | -0,51741487 | protein_coding | protein coding | NA | NA |
| ENSG00000005206.16_1 | NA | NA | SPPL2B | 0,04602932 | 0,4922383 | protein_coding | protein coding | NA | NA |
| ENSG00000111252.10_2 | NA | NA | SH2B3 | 0,04603228 | 0,55496975 | protein_coding | protein coding | NA | NA |
| ENSG00000123240.16_3 | NA | NA | OPTN | 0,0460755 | 0,57981883 | protein_coding | protein coding | NA | NA |
| ENSG00000069424.14_2 | NA | NA | KCNAB2 | 0,04607998 | 0,49912061 | protein_coding | protein coding | NA | NA |
| ENSG00000130299.16_3 | NA | NA | GTPBP3 | 0,04619261 | 0,52227955 | protein_coding | protein coding | NA | NA |
| ENSG00000260954.1_4 | NA | NA | AL133297.1 | 0,04634589 | 0,43653454 | sense_intronic | lncRNA | NA | NA |
| ENSG00000018625.14_2 | NA | NA | ATP1A2 | 0,04634831 | -0,56936417 | protein_coding | protein coding | NA | NA |
| ENSG00000017427.15_3 | NA | NA | IGF1 | 0,04635696 | -0,74284644 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000151136.14_3 | NA | NA | BTBD11 | 0,04644374 | -0,5809721 | protein_coding | protein coding | NA | NA |
| ENSG00000120334.15_2 | NA | NA | CENPL | 0,04648797 | 0,53790484 | protein_coding | protein coding | NA | NA |
| ENSG00000092051.16_3 | NA | NA | JPH4 | 0,04649137 | -0,61034001 | protein_coding | protein coding | NA | NA |
| ENSG00000197894.10_3 | NA | NA | ADH5 | 0,0465017 | -0,70143571 | protein_coding | protein coding | NA | NA |
| ENSG00000267311.1_4 | NA | NA | AC007673.1 | 0,04658554 | -0,60529216 | lincRNA | lncRNA | NA | NA |
| ENSG00000091140.13_2 | NA | NA | DLD | 0,04663836 | -0,55382918 | protein_coding | protein coding | NA | NA |
| ENSG00000250982.2_2 | NA | NA | GAPDHP35 | 0,04665766 | 0,52869039 | processed_pseudogene | pseudogene | NA | NA |
| ENSG00000112079.8_3 | NA | NA | STK38 | 0,04666289 | 0,58722536 | protein_coding | protein coding | NA | NA |
| ENSG00000218891.4_2 | NA | NA | ZNF579 | 0,04666308 | 0,60144568 | protein_coding | protein coding | NA | NA |
| ENSG00000173391.8_3 | NA | NA | OLR1 | 0,04668001 | 0,55903322 | protein_coding | protein coding | NA | NA |
| ENSG00000159110.19_3 | NA | NA | IFNAR2 | 0,04668563 | 0,56707835 | protein_coding | protein coding | NA | NA |
| ENSG00000108561.8_3 | NA | NA | C1QBP | 0,04669429 | -0,5025373 | protein_coding | protein coding | NA | NA |
| ENSG00000123066.7_3 | NA | NA | MED13L | 0,0467116 | 0,57228969 | protein_coding | protein coding | NA | NA |
| ENSG00000125386.15_4 | NA | NA | FAM193A | 0,0467175 | 0,62766797 | protein_coding | protein coding | NA | NA |
| ENSG00000164050.12_2 | NA | NA | PLXNB1 | 0,04675889 | 0,58559944 | protein_coding | protein coding | NA | NA |
| ENSG00000198673.10_3 | NA | NA | FAM19A2 | 0,04675953 | 0,42397985 | protein_coding | protein coding | NA | NA |
| ENSG00000126062.3_2 | NA | NA | TMEM115 | 0,04676548 | 0,51179558 | protein_coding | protein coding | NA | NA |
| ENSG00000134398.14_3 | NA | NA | ERN2 | 0,04687199 | -0,61102002 | protein_coding | protein coding | NA | NA |
| ENSG00000120458.10_2 | NA | NA | MSANTD2 | 0,04692872 | 0,49544605 | protein_coding | protein coding | NA | NA |
| ENSG00000086696.10_3 | NA | NA | HSD17B2 | 0,04696399 | 0,47264587 | protein_coding | protein coding | NA | NA |
| ENSG00000206418.3_2 | NA | NA | RAB12 | 0,04714138 | 0,5845907 | protein_coding | protein coding | NA | NA |
| ENSG00000072210.18_3 | NA | NA | ALDH3A2 | 0,04715918 | -0,57165166 | protein_coding | protein coding | NA | NA |
| ENSG00000168137.15_3 | NA | NA | SETD5 | 0,04719294 | 0,57375297 | protein_coding | protein coding | NA | NA |
| ENSG00000267838.2_3 | NA | NA | AC245884.8 | 0,04739189 | 0,53882478 | lincRNA | lncRNA | NA | NA |
| ENSG00000264538.6_2 | NA | NA | SUZ12P1 | 0,04739319 | 0,57384327 | transcribed_unprocessed_pseudogene | pseudogene | NA | NA |
| ENSG00000091583.10_3 | NA | NA | APOH | 0,04743045 | 0,56873313 | protein_coding | protein coding | NA | NA |
| ENSG00000198677.10_2 | NA | NA | TTC37 | 0,04752254 | -0,51002189 | protein_coding | protein coding | NA | NA |
| ENSG00000167397.14_3 | NA | NA | VKORC1 | 0,04773081 | 0,55606942 | protein_coding | protein coding | NA | NA |
| ENSG00000178184.15_2 | NA | NA | PARD6G | 0,04776051 | -0,4552983 | protein_coding | protein coding | NA | NA |
| ENSG00000101181.17_3 | NA | NA | MTG2 | 0,04776682 | 0,56153366 | protein_coding | protein coding | NA | NA |
| ENSG00000257167.2_3 | NA | NA | TMPO-AS1 | 0,04781843 | 0,50991765 | antisense_RNA | lncRNA | NA | NA |
| ENSG00000233912.1_4 | NA | NA | AC026202.2 | 0,04787167 | -0,44625109 | antisense_RNA | lncRNA | NA | NA |
| ENSG00000187097.12_3 | NA | NA | ENTPD5 | 0,04805357 | -0,5047975 | protein_coding | protein coding | NA | NA |
| ENSG00000111906.17_2 | NA | NA | HDDC2 | 0,04807816 | -0,53242322 | protein_coding | protein coding | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000144504.15_3 | NA | NA | ANKMY1 | 0,0480971 | 0,53418947 | protein_coding | protein coding | NA | NA |
| ENSG00000105552.14_3 | NA | NA | BCAT2 | 0,04811552 | -0,54705531 | protein_coding | protein coding | NA | NA |
| ENSG00000127511.9_3 | NA | NA | SIN3B | 0,04811868 | 0,52642679 | protein_coding | protein coding | NA | NA |
| ENSG00000101447.14_2 | NA | NA | FAM83D | 0,04814142 | 0,54128309 | protein_coding | protein coding | NA | NA |
| ENSG00000171747.8_2 | NA | NA | LGALS4 | 0,04815057 | 0,5938642 | protein_coding | protein coding | NA | NA |
| ENSG00000064309.14_2 | NA | NA | CDON | 0,04822183 | 0,44030631 | protein_coding | protein coding | NA | NA |
| ENSG00000148926.9_3 | NA | NA | ADM | 0,04826237 | 0,55779992 | protein_coding | protein coding | NA | NA |
| ENSG00000105401.8_3 | NA | NA | CDC37 | 0,04830573 | 0,63501101 | protein_coding | protein coding | NA | NA |
| ENSG00000115267.5_2 | NA | NA | IFIH1 | 0,04845119 | 0,44418828 | protein_coding | protein coding | NA | NA |
| ENSG00000138834.12_3 | NA | NA | MAPK8IP3 | 0,04851911 | 0,50530052 | protein_coding | protein coding | NA | NA |
| ENSG00000139354.10_3 | NA | NA | GAS2L3 | 0,04872508 | 0,6039567 | protein_coding | protein coding | NA | NA |
| ENSG00000021645.18_3 | NA | NA | NRXN3 | 0,04875823 | 0,40128402 | protein_coding | protein coding | NA | NA |
| ENSG00000187626.8_2 | NA | NA | ZKSCAN4 | 0,04876599 | 0,5800227 | protein_coding | protein coding | NA | NA |
| ENSG00000115145.9_2 | NA | NA | STAM2 | 0,0487912 | -0,51159246 | protein_coding | protein coding | NA | NA |
| ENSG00000183808.11_3 | NA | NA | RBM12B | 0,04889183 | 0,53061673 | protein_coding | protein coding | NA | NA |
| ENSG00000118960.12_3 | NA | NA | HS1BP3 | 0,04889829 | 0,57519798 | protein_coding | protein coding | NA | NA |
| ENSG00000103876.11_3 | NA | NA | FAH | 0,048913 | -0,48497157 | protein_coding | protein coding | NA | NA |
| ENSG00000084463.7_2 | NA | NA | WBP11 | 0,04892519 | 0,30467623 | protein_coding | protein coding | NA | NA |
| ENSG00000181192.11_3 | NA | NA | DHTKD1 | 0,04895084 | -0,55680905 | protein_coding | protein coding | NA | NA |
| ENSG00000144401.14_2 | NA | NA | METTL21A | 0,04895981 | 0,54089188 | protein_coding | protein coding | NA | NA |
| ENSG00000237517.9_3 | NA | NA | DGCR5 | 0,04896895 | 0,46269673 | transcribed_unprocessed_pseudogene | pseudogene | NA | NA |
| ENSG00000007047.14_3 | NA | NA | MARK4 | 0,04897541 | 0,57531786 | protein_coding | protein coding | NA | NA |
| ENSG00000196547.14_3 | NA | NA | MAN2A2 | 0,04902171 | 0,54576327 | protein_coding | protein coding | NA | NA |
| ENSG00000129007.14_3 | NA | NA | CALML4 | 0,04906841 | 0,25954899 | protein_coding | protein coding | NA | NA |
| ENSG00000125885.13_4 | NA | NA | MCM8 | 0,04909925 | 0,5351483 | protein_coding | protein coding | NA | NA |
| ENSG00000182798.10_3 | NA | NA | MAGEB17 | 0,04913351 | 0,50405791 | protein_coding | protein coding | NA | NA |
| ENSG00000263155.5_4 | NA | NA | MYZAP | 0,04922239 | -0,62757901 | protein_coding | protein coding | NA | NA |
| ENSG00000130595.18_4 | NA | NA | TNNT3 | 0,04930545 | 0,42088156 | protein_coding | protein coding | NA | NA |
| ENSG00000011451.17_3 | NA | NA | WIZ | 0,04930998 | 0,64671945 | protein_coding | protein coding | NA | NA |
| ENSG00000164932.12_2 | NA | NA | CTHRC1 | 0,04939095 | 0,53741685 | protein_coding | protein coding | NA | NA |
| ENSG00000103485.17_3 | NA | NA | QPRT | 0,04943368 | 0,57076633 | protein_coding | protein coding | NA | NA |
| ENSG00000070047.11_2 | NA | NA | PHRF1 | 0,0494507 | 0,62648941 | protein_coding | protein coding | NA | NA |
| ENSG00000197329.11_2 | NA | NA | PELI1 | 0,04947151 | 0,53479379 | protein_coding | protein coding | NA | NA |
| ENSG00000141030.12_2 | NA | NA | COPS3 | 0,04953633 | -0,48640871 | protein_coding | protein coding | NA | NA |
| ENSG00000260646.1_3 | NA | NA | AL031705.1 | 0,04957859 | 0,42489016 | lincRNA | lncRNA | NA | NA |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| ENSG00000269893.6_2 | NA | NA | SNHG8 | 0,04958736 | -0,53530914 | lincRNA | lncRNA | NA | NA |
| ENSG00000161999.11_3 | NA | NA | JMJD8 | 0,04963484 | 0,53759098 | protein_coding | protein coding | NA | NA |
| ENSG00000157404.15_2 | NA | NA | KIT | 0,04974377 | -0,55979066 | protein_coding | protein coding | NA | NA |
| ENSG00000189143.9_3 | NA | NA | CLDN4 | 0,04981994 | 0,53849259 | protein_coding | protein coding | NA | NA |
| ENSG00000184343.10_3 | NA | NA | SRPK3 | 0,04996185 | 0,45050678 | protein_coding | protein coding | NA | NA |
| ENSG00000149599.15_3 | NA | NA | DUSP15 | 0,04998761 | -0,56279629 | protein_coding | protein coding | NA | NA |
| ENSG00000166823.5_2 | NA | NA | MESP1 | 0,0499955 | -0,57562016 | protein_coding | protein coding | NA | NA |
| NA | XLOC_158557 | TCONS_00321576 | NA | 0,00063265 | -0,85954611 | unknown | unknown | chr4:42211109-42265231 | + |
| NA | XLOC_158557 | TCONS_00321584 | NA | 0,00063265 | -0,85954611 | unknown | unknown | chr4:42228967-42265231 | + |
| NA | XLOC_158557 | TCONS_00321573 | NA | 0,00063265 | -0,85954611 | unknown | unknown | chr4:42211109-42216388 | + |
| NA | XLOC_158557 | TCONS_00321575 | NA | 0,00063265 | -0,85954611 | unknown | unknown | chr4:42211109-42241657 | + |
| NA | XLOC_158557 | TCONS_00321570 | NA | 0,00063265 | -0,85954611 | unknown | unknown | chr4:42198942-42202448 | + |
| NA | XLOC_158557 | TCONS_00321571 | NA | 0,00063265 | -0,85954611 | unknown | unknown | chr4:42198942-42257631 | + |
| NA | XLOC_158557 | TCONS_00321574 | NA | 0,00063265 | -0,85954611 | unknown | unknown | chr4:42211109-42241657 | + |
| NA | XLOC_135891 | TCONS_00276376 | NA | 0,00738972 | 0,54994332 | unknown | unknown | chr21:43194288-43196469 | + |
| NA | XLOC_135891 | TCONS_00276371 | NA | 0,00738972 | 0,54994332 | unknown | unknown | chr21:43187323-43188248 | + |
| NA | XLOC_135891 | TCONS_00276374 | NA | 0,00738972 | 0,54994332 | unknown | unknown | chr21:43188304-43196469 | + |
| NA | XLOC_135891 | TCONS_00276372 | NA | 0,00738972 | 0,54994332 | unknown | unknown | chr21:43187331-43188248 | + |
| NA | XLOC_135891 | TCONS_00276373 | NA | 0,00738972 | 0,54994332 | unknown | unknown | chr21:43187331-43196469 | + |
| NA | XLOC_189524 | TCONS_00369677 | NA | 0,00841318 | 0,47487523 | unknown | unknown | chr5:171232932-171273023 | - |
| NA | XLOC_189524 | TCONS_00369682 | NA | 0,00841318 | 0,47487523 | unknown | unknown | chr5:171245243-171273768 | - |
| NA | XLOC_016951 | TCONS_00034988 | NA | 0,01444182 | 0,30819622 | unknown | unknown | chr1:184609513-184641539 | - |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| NA | XLOC_016951 | TCONS_00034995 | NA | 0,01444182 | 0,30819622 | unknown | unknown | chr1:184630780-184641517 | - |
| NA | XLOC_016951 | TCONS_00034994 | NA | 0,01444182 | 0,30819622 | unknown | unknown | chr1:184630780-184641517 | - |
| NA | XLOC_016951 | TCONS_00034993 | NA | 0,01444182 | 0,30819622 | unknown | unknown | chr1:184630780-184641517 | - |
| NA | XLOC_016951 | TCONS_00034997 | NA | 0,01444182 | 0,30819622 | unknown | unknown | chr1:184630780-184641539 | - |
| NA | XLOC_016951 | TCONS_00034996 | NA | 0,01444182 | 0,30819622 | unknown | unknown | chr1:184630780-184641539 | - |
| NA | XLOC_078679 | TCONS_00149490 | NA | 0,01922875 | 0,4277114 | unknown | unknown | chr15:39739241-39745702 | - |
| NA | XLOC_078679 | TCONS_00149485 | NA | 0,01922875 | 0,4277114 | unknown | unknown | chr15:39723736-39742486 | - |
| NA | XLOC_078679 | TCONS_00149486 | NA | 0,01922875 | 0,4277114 | unknown | unknown | chr15:39723736-39756740 | - |
| NA | XLOC_040861 | TCONS_00078963 | NA | 0,01948539 | 0,47758274 | unknown | unknown | chr11:65573119-65585707 | - |
| NA | XLOC_040861 | TCONS_00078961 | NA | 0,01948539 | 0,47758274 | unknown | unknown | chr11:65573119-65585564 | - |
| NA | XLOC_040861 | TCONS_00078962 | NA | 0,01948539 | 0,47758274 | unknown | unknown | chr11:65573119-65585700 | - |
| NA | XLOC_026322 | TCONS_00049915 | NA | 0,02299877 | 0,46586538 | unknown | unknown | chr10:5502171-5503506 | - |
| NA | XLOC_026322 | TCONS_00049916 | NA | 0,02299877 | 0,46586538 | unknown | unknown | chr10:5502171-5508933 | - |
| NA | XLOC_139458 | TCONS_00284385 | NA | 0,02787765 | 0,54352809 | unknown | unknown | chr22:18550412-18554051 | - |
| NA | XLOC_139458 | TCONS_00284386 | NA | 0,02787765 | 0,54352809 | unknown | unknown | chr22:18550412-18554092 | - |
| NA | XLOC_006945 | TCONS_00014671 | NA | 0,02841858 | 0,62274863 | unknown | unknown | chr1:184081741-184091687 | + |
| NA | XLOC_006945 | TCONS_00014676 | NA | 0,02841858 | 0,62274863 | unknown | unknown | chr1:184088054-184091687 | + |
| NA | XLOC_006945 | TCONS_00014675 | NA | 0,02841858 | 0,62274863 | unknown | unknown | chr1:184088054-184090586 | + |
| NA | XLOC_158075 | TCONS_00320769 | NA | 0,02902115 | 0,3794339 | unknown | unknown | chr4:28291298-28338123 | + |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| NA | XLOC_158075 | TCONS_00320776 | NA | 0,02902115 | 0,3794339 | unknown | unknown | chr4:28331251-28338123 | + |
| NA | XLOC_158075 | TCONS_00320773 | NA | 0,02902115 | 0,3794339 | unknown | unknown | chr4:28325274-28327442 | + |
| NA | XLOC_158075 | TCONS_00320774 | NA | 0,02902115 | 0,3794339 | unknown | unknown | chr4:28325336-28338123 | + |
| NA | XLOC_104865 | TCONS_00216430 | NA | 0,03236359 | 0,38550281 | unknown | unknown | chr19:1246354-1248318 | - |
| NA | XLOC_231989 | TCONS_00441345 | NA | 0,03730235 | 0,31955139 | unknown | unknown | chr8:116193990-116268841 | - |
| NA | XLOC_231989 | TCONS_00441344 | NA | 0,03730235 | 0,31955139 | unknown | unknown | chr8:116193990-116268841 | - |
| NA | XLOC_199739 | TCONS_00385636 | NA | 0,03764499 | 0,52991976 | unknown | unknown | chr6:6798930-6801406 | - |
| NA | XLOC_199739 | TCONS_00385630 | NA | 0,03764499 | 0,52991976 | unknown | unknown | chr6:6789200-6794121 | - |
| NA | XLOC_199739 | TCONS_00385629 | NA | 0,03764499 | 0,52991976 | unknown | unknown | chr6:6789200-6791151 | - |
| NA | XLOC_030880 | TCONS_00057388 | NA | 0,03943907 | 0,29095279 | unknown | unknown | chr10:113276721-113547691 | - |
| NA | XLOC_030880 | TCONS_00057476 | NA | 0,03943907 | 0,29095279 | unknown | unknown | chr10:113544074-113545772 | - |
| NA | XLOC_179726 | TCONS_00354238 | NA | 0,04077875 | -0,62583334 | unknown | unknown | chr5:141445047-141448212 | + |
| NA | XLOC_179726 | TCONS_00354237 | NA | 0,04077875 | -0,62583334 | unknown | unknown | chr5:141445047-141448212 | + |
| NA | XLOC_178755 | TCONS_00352176 | NA | 0,04082666 | 0,40504836 | unknown | unknown | chr5:119537531-119556211 | + |
| NA | XLOC_178755 | TCONS_00352185 | NA | 0,04082666 | 0,40504836 | unknown | unknown | chr5:119780114-119797371 | + |
| NA | XLOC_246301 | TCONS_00463215 | NA | 0,04435218 | 0,43241447 | unknown | unknown | chr9:122548013-122554542 | - |
| NA | XLOC_246301 | TCONS_00463216 | NA | 0,04435218 | 0,43241447 | unknown | unknown | chr9:122550827-122554542 | - |
| NA | XLOC_131963 | TCONS_00269355 | NA | 0,04540418 | -0,59131818 | unknown | unknown | chr20:58611186-58630270 | + |
| NA | XLOC_131963 | TCONS_00269354 | NA | 0,04540418 | -0,59131818 | unknown | unknown | chr20:58611186-58630270 | + |

| Ensembl_ID | Gene_ID | Transcript_ID | Gene_Symbol | tail_based_Fdr | weighted_log HR | Biotype | Biotype_Cluster | Position | Strand |
|---|---|---|---|---|---|---|---|---|---|
| NA | XLOC_222230 | TCONS_00424987 | NA | 0,04601354 | 0,44829888 | unknown | unknown | chr8:23935040-24020985 | + |
| NA | XLOC_086821 | TCONS_00170694 | NA | 0,04712453 | -0,73895783 | unknown | unknown | chr16:63188093-63213039 | - |

**Table 3**

| ID | ProstaTrend score | biochemicalRecurrence | bcrTime (days) |
|---|---|---|---|
| X53eacf69.b50d.4050.af83.d4a9aa5605df | 0,01266451 | FALSE | 805 |
| c6172d43.6f62.420b.a22f.9f28673e561d | -0,01119452 | FALSE | 4604 |
| f42884a2.7a05.4cac.985c.b5488cb32bdb | -0,05386046 | FALSE | 518 |
| X140c214a.c5db.4580.b9eb.c25f9d69211e | -0,21978053 | FALSE | 554 |
| X68dba1a6.5a69.490d.8d92.be2bc0828df3 | -0,24622512 | FALSE | 1184 |
| c13bfc0c.de93.4904.8f0a.7d63a4e46831 | -0,03114551 | FALSE | 1384 |
| X4c61943c.702d.4d0b.9f38.2997c5deb0ae | 0,09244066 | FALSE | 182 |
| ab2bdcca.6c49.434d.bc7b.5e89708dd627 | 0,17446423 | FALSE | 995 |
| c827acc6.8c94.4e2f.9eeb.283cc2476060 | -0,11350905 | FALSE | 145 |
| X1cc487f4.6a5e.4402.8ddf.85346c71ddf8 | 0,04215859 | FALSE | 1014 |
| f5516033.4759.440c.bc7f.d561bd6305f7 | -0,03024049 | FALSE | 1338 |
| b187472a.04ab.4991.b5c3.ea17d62f3a88 | 0,26566694 | FALSE | 3253 |
| X83fd57c2.d699.485f.b39c.6a8998285ff1 | -0,13597635 | FALSE | 417 |
| X0169b0f8.d12e.4944.ae8c.7141347cd461 | 0,3666697 | FALSE | 709 |
| X8d787c3d.11ec.4e12.b722.ca78a9b2c50e | -0,00788842 | FALSE | 170 |
| f8b69065.5dc8.4392.915b.1bc3c6059725 | 0,12038141 | FALSE | 1195 |
| f96498cc.5436.46ff.9c1c.73cbf078d079 | 0,10768653 | FALSE | 882 |
| X50b59441.8f4d.4ec1.a410.3c7b1567dfca | -0,07915654 | FALSE | 324 |
| f76af4bc.b7da.4e4c.9158.6988094fb4b7 | -0,04643407 | FALSE | 295 |
| X9f7c22a6.eaad.4842.b5aa.2248d4286789 | 0,09182014 | FALSE | 637 |
| f88fcf42.cadb.4be3.a09d.ba20c2247823 | 0,22984177 | FALSE | 972 |
| a316b78d.a63b.4616.b3cc.2d3618134f89 | 0,02655875 | TRUE | 786 |
| X09790590.7ae1.45db.9a0b.9e9e1c93854d | -0,22038374 | FALSE | 2056 |
| X083b4b88.d288.4ca1.b52e.14dfcea758b6 | -0,18768493 | FALSE | 452 |
| X9a3ae902.21d1.404a.9799.ab707618458d | 0,32291743 | FALSE | 589 |
| X449c910a.9493.477f.bb3e.2d281e8d8ded | -0,13339255 | FALSE | 397 |
| X9cdcffcc.1ed6.400e.be79.aeb54ec3c99c | 0,03262865 | FALSE | 110 |
| e8e9a0ac.6ce8.4698.9daf.d7b4478915b1 | 0,43773787 | FALSE | 686 |
| b18d28f5.bade.4225.b2bf.4534410cbc1e | 0,04834977 | FALSE | 2364 |
| X931af2bf.3f7c.4d3c.ba4b.3bbb30bd1e2a | 0,12314357 | TRUE | 752 |
| X723b8d9f.4f37.4081.849e.caa060f298d1 | -0,12599414 | FALSE | 848 |
| X196d6e43.c916.48ce.a58a.843ef13005f5 | 0,09272478 | FALSE | 1259 |
| f459e048.8807.404e.acd5.c97dcdb385b2 | -0,0433282 | FALSE | 308 |
| X9f089f72.125d.4e8b.af68.0c952e9b2244 | -0,2448196 | FALSE | 1109 |
| e6b6cced.c361.435d.a61c.e57b24aaba08 | -0,08254783 | FALSE | 2542 |
| X5389cb15.76dc.4bd3.948a.a94fa01b4a4f | -0,08210564 | FALSE | 1949 |
| X9f0b719f.c9ef.4971.a895.14b4634eba24 | -0,01594692 | FALSE | 483 |
| af4162fd.8ecd.4a6e.8dfa.770f8f7d0df2 | 0,1465537 | FALSE | 209 |
| X89344308.d9b9.402d.a763.7a5e83d06569 | -0,37933669 | FALSE | 278 |
| d91fd3ba.d209.4547.9294.5a102c2ad57b | -0,03042509 | FALSE | 191 |
| fbeb79a3.8408.4384.84b3.5205c7639929 | -0,05659524 | FALSE | 1842 |
| X3c17b4d2.85d0.4e6e.8f53.9c9b746f0917 | 0,19127046 | FALSE | 2068 |
| b33eebec.4b2a.4872.bdf3.92a9bc2774cf | 0,04406705 | TRUE | 329 |
| X8982e051.d476.4b65.a541.663f3f2892f2 | 0,06355037 | FALSE | 175 |
| X70d515bc.4ccd.4dc1.bb8f.0e2ff65b87c5 | -0,1143342 | FALSE | 943 |
| X33818bd9.d030.4b7d.ba89.639ae678e2ad | -0,00553638 | FALSE | 176 |
| X1d9a798b.cbbc.4551.ad48.f804a6a0d8de | 0,01413397 | TRUE | 1506 |
| dbd793a8.1b32.480d.9366.bb7b45c14549 | 0,0800748 | FALSE | 167 |
| X1241a34b.e299.49e0.b8db.c1293017b0b7 | -0,18743848 | FALSE | 1501 |
| X1768ed1c.76c3.4992.816d.bf0bcd8fb331 | 0,12297607 | TRUE | 218 |
| X86409fcc.d962.41d6.87cf.6db35eb7be2b | 0,10558817 | TRUE | 473 |
| ccd746e5.7974.49e4.befe.05f4ef1f5ed9 | -0,02274617 | FALSE | 270 |
| X5d392f6c.d936.479a.9991.53902919b63f | 0,15836444 | TRUE | 1376 |

| ID | ProstaTrend score | biochemicalRecurrence | bcrTime (days) |
|---|---|---|---|
| ddb80bec.78e0.403d.8f14.131d0d32cf11 | -0,1990645 | FALSE | 285 |
| d742eebc.242b.4c5e.8429.7d67b6173e79 | 0,07410416 | FALSE | 263 |
| d0f9a9c1.433f.4c56.91b8.ea8381eb0cc7 | -0,1319643 | FALSE | 671 |
| d6b8349b.d829.4b8b.90b9.0e5900b90492 | -0,11545964 | FALSE | 917 |
| X0af86aba.8244.4a90.ba34.33e2331d0ab2 | -0,20141912 | TRUE | 189 |
| X1e2261f6.1053.47dd.8128.afb3d34729fa | -0,16246971 | FALSE | 162 |
| X80aa8fed.1d46.4e3c.b93d.8ac1b778b6a6 | -0,13243066 | FALSE | 972 |
| X56b1d8a7.1827.43ae.a08d.6f8419fc5fd0 | -0,00746181 | FALSE | 1001 |
| b0a12dfa.fb35.4b78.9886.47354d0f521e | -0,30394208 | FALSE | 273 |
| X90c1a5fd.552c.4edf.a51f.99f6dc57554d | -0,20861707 | FALSE | 486 |
| X8e1c619f.9c79.4968.8d13.f457f83ea840 | 0,00027337 | FALSE | 459 |
| cbccf354.b6fa.41cb.8ff6.d1b380e501aa | -0,11229769 | FALSE | 176 |
| X2240eb93.791a.4d1e.b89e.5e483fa3e9f5 | 0,38971649 | TRUE | 637 |
| X161dc244.0ea6.4d46.b1df.03efdfbff115 | 0,16790848 | FALSE | 405 |
| X554cce35.3074.41f7.ab68.974a9307e879 | 0,35341964 | FALSE | 62 |
| X768911a0.ac76.4ced.acf3.e3480c506a8b | -0,08424084 | FALSE | 615 |
| X7a0c0e5e.a498.4b52.951f.3fbb5eac60fe | -0,1599889 | FALSE | 202 |
| X4d15b276.42e8.4d9b.b1d1.de6d9028d69d | -0,13480353 | FALSE | 230 |
| caab94fd.80c1.448c.9600.6490351468dd | -0,14847363 | FALSE | 526 |
| da85e3dc.8955.4490.b90f.f8cb86f238b1 | 0,2070685 | FALSE | 549 |
| X687173e8.1b1b.4daf.a114.0ab8ad8375d0 | -0,12354201 | FALSE | 31 |
| X37dc07d5.b80d.4b71.ba83.9de5f475efda | 0,36338493 | FALSE | 350 |
| X7935ade5.92b8.49b6.9ce4.0fe2927902fd | -0,03496328 | FALSE | 310 |
| X5bdc302e.94f7.45af.96ee.2ea25c4ad3d5 | 0,17215343 | FALSE | 132 |
| db36e05c.3a5a.4c5f.ad51.43193d967fa6 | -0,02109283 | FALSE | 530 |
| bf3bdbbd.e639.4a52.b9f2.3f02bc475066 | -0,27805488 | FALSE | 371 |
| X9bc50510.26be.44f8.b81d.49be4b0d3f18 | -0,21645104 | FALSE | 1215 |
| X7f8c4021.5d13.4323.8f9d.8704ae8e47a8 | -0,14386954 | FALSE | 700 |
| d4374b78.cfc3.40e1.8f25.1c3217003495 | -0,23145698 | FALSE | 365 |
| cf07746e.d21a.4d7e.9bff.1526d62ac000 | 0,11451708 | FALSE | 412 |
| ba5c394f.6645.40c0.8c6c.d49afbc63a59 | -0,20359867 | FALSE | 783 |
| X0021aa6d.3e7d.4f82.a4d9.77171a599775 | 0,18049792 | FALSE | 121 |
| X046395fd.299c.4639.9ce8.072cd7263718 | 0,2892765 | FALSE | 28 |
| b8294676.1393.4dc7.85e2.af0b865c0d4b | -0,32529212 | FALSE | 448 |
| c83615eb.9452.415d.9d54.3172f9b508bb | 0,5328095 | FALSE | 244 |
| X85f8f1ac.16c9.4625.9b79.4728ef7188b6 | 0,07252379 | TRUE | 198 |
| X6dace0fb.826b.466c.a284.749022ca41b1 | -0,22899344 | FALSE | 1961 |
| X0fb021dc.dc06.4b8f.bc40.50aa2808ee90 | -0,01726681 | FALSE | 91 |
| X75552368.9d49.447d.a59e.e9763f2e4cdd | 0,10422917 | TRUE | 215 |
| X58e733fb.f36f.46ec.98b6.c0bb47acc319 | 0,10761706 | FALSE | 396 |
| X4ca75b49.9a93.4972.b852.9d4821321807 | -0,39961739 | FALSE | 458 |
| X7bcfa805.138e.4ef9.bf8c.327accd14005 | -0,3390933 | FALSE | 132 |
| fafde957.1ae0.4952.be02.63336a383131 | -0,03890827 | TRUE | 559 |
| d60a0f58.a953.495c.9b3e.1a9d5ea7d1fa | 0,02994123 | FALSE | 857 |
| fc44d09d.fcf0.4cd8.ad2c.2f56637e8d47 | 0,12738045 | FALSE | 999 |
| bcca1e02.6bdc.4082.b4cc.7646ccf62f32 | -0,17814844 | FALSE | 822 |
| c1a5f47e.4290.4ea7.ae45.028879eda0ed | -0,22856909 | FALSE | 123 |
| ced9ddf3.89b4.4510.ab4b.cbe32d9039f8 | -0,1780631 | FALSE | 907 |
| ff41604c.f1e3.43aa.b085.cb953ef11e29 | -0,12186806 | FALSE | 804 |
| a257a966.15dc.445c.b29c.e48591f44c7e | 0,22694412 | FALSE | 1659 |
| X027e7fb6.605b.45a9.8b5b.c8adf7976660 | 0,03314359 | FALSE | 1102 |
| X568db38f.d1d3.4d4b.b171.3590deddf6f0 | 0,18647155 | TRUE | 429 |
| X68796415.e8cb.4d7c.91d5.579647f37e25 | -0,07011119 | FALSE | 834 |
| X6afa1c47.6459.4fde.bc19.e2b96dcbaa9d | 0,21823724 | FALSE | 485 |
| X295e5368.29f7.4ec9.bbde.9cec0d3be8fb | -0,18637738 | FALSE | 448 |
| dca31650.cad5.4fd4.8816.ed571afb54ad | -0,23386256 | FALSE | 112 |
| bd77c761.c3f6.4be3.bfb2.ffc6e12cb1a3 | -0,07922562 | FALSE | 889 |
| X6a08c20f.f647.48cc.9c6f.eefb6da31ec2 | 0,17898896 | FALSE | 223 |
| f7b8f6ba.1c67.4738.858e.ec84915e0948 | -0,02601896 | FALSE | 1399 |

| ID | ProstaTrend score | biochemicalRecurrence | bcrTime (days) |
|---|---|---|---|
| X22ab8f43.2462.4902.ada7.7a7ec7fbc51c | -0,26842052 | FALSE | 457 |
| X2cc19db3.7e6c.43a4.a5db.fca5533e2b0f | 0,16918024 | TRUE | 310 |
| c31df1a8.6a00.4721.9d89.018a0da55e80 | 0,12931485 | FALSE | 1621 |
| X0ce5ab7b.0010.4553.a085.75b5e2efc9ae | 0,05219418 | TRUE | 180 |
| X02502979.8348.46f3.8a01.4028ddcdbcbd | 0,31309118 | FALSE | 428 |
| X0e2376cf.0c11.4ce2.ba35.96f5194b7104 | 0,0782636 | FALSE | 1173 |
| X3e2d4fdd.acba.4394.afa0.e7b5e150c3ea | -0,00692274 | FALSE | 396 |
| X1ee743ac.11cf.428f.9638.2812946ff38a | -0,22358821 | FALSE | 775 |
| f16673b9.5a56.457d.a85f.229e81a8a4f5 | 0,04477149 | FALSE | 2170 |
| X5da88297.0f33.47ff.81ca.748d691cfe32 | 0,25922666 | TRUE | 765 |
| ea217462.0178.47f1.87d2.049ec77cfb22 | 0,02454724 | TRUE | 84 |
| a11daa48.bdd3.4cc1.a4d8.b16c3bec848b | -0,06826653 | FALSE | 598 |
| X442e7bff.8453.49dd.8b22.b04e226b3fcf | -0,12270002 | FALSE | 567 |
| X2c8f0ab5.cf44.40bb.b9f9.daf5bb66bb7c | -0,20237035 | FALSE | 614 |
| X309b6de1.4442.4259.be57.3ce9993a9018 | 0,08388312 | FALSE | 321 |
| X294fb99b.f5a1.4565.902a.dbe6d6b5f5ba | 0,00674219 | FALSE | 437 |
| c78c0c4e.6669.42d9.8fe3.1379accb2b00 | -0,09703668 | FALSE | 314 |
| X5b844b3c.e3dc.43db.8825.4bd85c12e2e5 | -0,23342214 | FALSE | 212 |
| fb967d00.eb5a.407c.b712.0e5014cae86c | 0,30574526 | FALSE | 602 |
| a8fda00e.a53f.47b2.82c1.7b15bef0ef06 | 0,20639129 | FALSE | 96 |
| X152cc35b.4bb8.4ca7.ab6e.536e388d80cd | 0,1904656 | TRUE | 740 |
| X1e9cfeda.eeff.458e.ab59.8e8fe6c756a9 | -0,08417868 | FALSE | 362 |
| bf36ed70.dd1a.49ba.81f8.8e705666b618 | 0,09229634 | FALSE | 3487 |
| e857e43d.cc5f.4fc1.ab90.0d6ebdf906d1 | -0,13022881 | FALSE | 974 |
| e3ebab82.0c64.417f.b8b9.da0c32482539 | 0,05607313 | FALSE | 729 |
| a8329b1a.c613.425d.8726.6e8de23fe54a | -0,01979317 | FALSE | 822 |
| X1c2c7841.ff7d.4ce5.9a5f.db4c8596f5df | -0,20628571 | FALSE | 851 |
| X1fc91a38.29a6.4325.a8e2.8ebf3c1b35d9 | 0,3243863 | FALSE | 323 |
| e789a2c6.32b8.44a0.939a.def0c5dbba39 | -0,25864579 | FALSE | 79 |
| X662d84f3.a9b3.4d13.bce2.53ee1efa6815 | -0,13410796 | FALSE | 187 |
| X39a4ef2d.33fc.4bb3.8a5c.5b36dccbcae2 | 0,00250547 | FALSE | 474 |
| a37161ec.8a26.4fea.9f97.206b89bee6db | 0,00794218 | TRUE | 442 |
| X01bcef33.9987.49b2.a3bc.a2fe8fc40563 | -0,02266333 | FALSE | 1000 |
| X3932d5bd.8c71.430e.a723.3d6a617a457a | 0,13431867 | FALSE | 724 |
| X9fa4d0b8.280a.4785.b325.144924a75c30 | -0,04171479 | FALSE | 1008 |
| be497d4b.0d7a.49f2.a71a.1ea597e15676 | -0,10064805 | FALSE | 32 |
| X966b0ab3.2c53.44fc.a867.690ff64a9168 | -0,37740911 | FALSE | 850 |
| d47a679d.0eb9.4b39.a787.5f085c44f6ee | -0,11141955 | FALSE | 57 |
| af9a40a0.ea09.4aba.8cf5.c60eab1d92ee | 0,11451695 | FALSE | 1879 |
| X39cd3712.1dfc.40a7.b0a8.a87cbe4f8fc0 | -0,16979815 | FALSE | 1375 |
| X0c5aa19a.88c4.47e9.9f78.287074b1e191 | -0,0019982 | FALSE | 533 |
| X2476d745.50ba.4488.b90b.20fdb9b7afe5 | 0,00339486 | TRUE | 1009 |
| X40841d0b.2fee.4f82.a801.1a458d76ab05 | 0,27961533 | FALSE | 93 |
| X9f881762.34ba.40a9.8271.63c5d3a35d9b | 0,243445 | FALSE | 1563 |
| f81853f4.1ba5.4b9c.9b34.c6cf8b53d600 | 0,14818414 | FALSE | 269 |
| X5d371173.1cbb.4811.a9af.0beaeb13714c | -0,12573411 | FALSE | 31 |
| X0c4f1f4c.cd77.49fa.86a1.025ead78300e | -0,00662012 | FALSE | 668 |
| X6a92360e.ae54.46b0.95aa.4161620d632f | -0,01412014 | FALSE | 738 |
| efee5692.64fc.46eb.9d20.46f2090c65d2 | 0,09612976 | FALSE | 350 |
| X5fad56e2.139a.4d53.9844.26ceffe8a5f7 | -0,07407246 | FALSE | 1033 |
| X8e1d154b.6afd.4800.a8e0.1e58896ebc8b | -0,21681147 | FALSE | 1332 |
| X4dc4f20b.29c0.4c88.a9a9.73b850b8aaa9 | -0,01563802 | FALSE | 724 |
| X82beabd5.73b6.4cb1.8b54.ad490cfcc04f | 0,03163506 | FALSE | 606 |
| X61223fbb.abbd.4c17.b54d.9b4d6fe959e4 | -0,11922588 | FALSE | 1608 |
| edadb5bf.e858.427d.8b17.f95ba6a6e04d | 0,01110778 | TRUE | 626 |
| f69bedb8.913e.42ac.b9e5.bbdee8c3e2c1 | -0,15695967 | FALSE | 664 |
| X300ecd12.29f0.46dc.8697.5600ff768611 | -0,07940581 | FALSE | 523 |
| X9bcbcf4b.e727.4de2.99f0.23fa1e3c7f00 | -0,13022354 | FALSE | 183 |
| b57c6e3d.3c11.42ef.99ee.d463d495a9ab | 0,09500071 | FALSE | 61 |

| ID | ProstaTrend score | biochemicalRecurrence | bcrTime (days) |
|---|---|---|---|
| cf4f7acf.4ae8.4de8.8c8b.8a8ab9023cbe | -0,1242812 | FALSE | 356 |
| X09211e49.e1be.41cf.8c59.d7e6fa5d54f2 | -0,01845826 | FALSE | 488 |
| a7871851.76a9.4b91.a4e5.05bd8f4a2d65 | -0,03886305 | FALSE | 1339 |
| X326de35c.3b96.43fc.8268.cb68a9f9540f | -0,00255355 | FALSE | 295 |
| X46d9ab52.41a7.4f58.b505.81f8e9a53b13 | 0,1909536 | FALSE | 790 |
| X83b5f9f5.945b.48b7.a1b5.c65688aca56b | -0,20286457 | FALSE | 841 |
| X87876b23.f561.4405.a247.06c54e8db349 | 0,02962444 | FALSE | 1611 |
| X855f5506.0f78.44cb.a610.fd160b4a659c | -0,16486132 | FALSE | 101 |
| e096d636.a693.40e8.8266.2e078596b74f | 0,45143928 | FALSE | 1065 |
| X017c457c.5a5d.403a.9201.ce8365cc08e0 | -0,04526995 | FALSE | 302 |
| d821bd34.ee64.429c.a806.e1dcf50d48b5 | -0,16762768 | FALSE | 279 |
| X872bde64.2da7.4b64.be60.961b8564fc1b | 0,0525303 | FALSE | 211 |
| b529e9b2.d3ca.444c.b3b3.9c8dfb86a3c0 | -0,21045824 | FALSE | 356 |
| e971b82b.4a7b.4fc1.bfe1.2f5b72897fec | 0,07005553 | FALSE | 776 |
| X53c47f05.bf62.4085.a013.3a4b308e86be | -0,07989247 | FALSE | 91 |
| X6a447bae.5df6.46d7.804e.575221380d54 | -0,14303767 | TRUE | 941 |
| a97b7802.ca2b.4a4b.a39d.0a5e48711949 | -0,07827975 | FALSE | 249 |
| a94862d0.e649.45d6.9cab.3af4d63564ee | -0,10435789 | FALSE | 993 |
| e6dfb032.58a7.4e4e.b14f.1c391eebec92 | -0,08408392 | FALSE | 1157 |
| X6a5d21d3.9004.4cea.a2c4.133e914f589c | -0,0195504 | FALSE | 32 |
| X89a641c4.74ea.4f4a.8d2f.8adca3608623 | -0,17107647 | FALSE | 926 |
| X2dae139f.1f00.4916.af87.b8a72e6fe13b | 0,18005313 | FALSE | 663 |
| b12709c4.1ecc.4dcf.96e9.366415dbbecb | 0,03033594 | FALSE | 636 |
| X5135a6a3.aa0d.4d45.8b11.fd03876c6258 | -0,21537017 | FALSE | 395 |
| X9e9f64c5.4c11.4df5.81a0.46a4ac3580d3 | 0,0649288 | FALSE | 91 |
| b59777f4.95d3.46be.8208.a7c04b3140dc | -0,09112797 | FALSE | 1718 |
| bc12158d.9bfe.4c63.b12f.90251587b88d | -0,1811222 | FALSE | 1441 |
| X6da91052.f931.40b9.be1e.7fa3e9bb68f7 | -0,057193 | FALSE | 2576 |
| X8e37a3d8.01e2.4f3e.b8a6.de48e62e9770 | 0,31855793 | TRUE | 338 |
| X11cf4550.2080.43df.841c.c013d98e37b0 | 0,20341072 | FALSE | 487 |
| X2c4156de.1e08.40dc.802b.f575f9bea90a | 0,17245067 | FALSE | 542 |
| e09e7160.94da.4582.a939.51f3986da8c4 | -0,0074925 | FALSE | 158 |
| X876a1e36.fb72.4fab.b69d.355acabee7f3 | 0,00380783 | FALSE | 261 |
| X4d7a83be.fcad.475f.bf4a.6ba354755af9 | -0,18607038 | FALSE | 304 |
| X1311a7d1.cc47.49a7.9624.5e8962ed8806 | 0,066241 | TRUE | 1328 |
| af354027.d898.44b7.8ac1.eb3ebf07454c | -0,03236443 | FALSE | 256 |
| X30dc8120.d30e.4eb7.849b.1f9e5375cbfd | -0,11574065 | FALSE | 168 |
| b055526f.6b23.45a3.a356.03ef60078111 | 0,4720704 | FALSE | 427 |
| X7e3a5c0b.f50c.425c.954b.a7ce59a313b1 | 0,02726321 | FALSE | 526 |
| X22c1fa1f.8d08.42e2.ba65.756df100f411 | -0,14851517 | FALSE | 1403 |
| X3b7e626d.f31c.497e.810d.fb7c13872a03 | 0,06685615 | FALSE | 721 |
| f98879f0.0798.4646.a277.725c1d05192f | -0,208049 | FALSE | 1770 |
| X8cc08948.9ce0.4250.a269.d929d5de39e5 | -0,05003344 | FALSE | 663 |
| e9a615b2.8e97.4143.b221.deda830b0806 | 0,01658768 | FALSE | 1126 |
| e79862e3.cb96.4ecb.98c8.c5258992c7ec | 0,07848353 | TRUE | 648 |
| X8a8b6b66.94d5.464b.aae3.3908b573ea4d | -0,03282795 | FALSE | 930 |
| X791c023a.e994.4b93.bdff.2b59cb5ea875 | -0,14776044 | FALSE | 91 |
| b51add64.6d2d.48e4.9a0c.a7f760de8950 | -0,14532608 | FALSE | 1860 |
| X334b96f5.b6e5.483f.a855.bb2367c47b32 | -0,14888828 | FALSE | 519 |
| X500b3dfa.fd7f.4bfb.8fda.3bfd3dc75fa3 | -0,05121412 | FALSE | 1572 |
| fced33b4.2c8f.407d.9b4a.f416a5ba7580 | -0,25050563 | FALSE | 1237 |
| fe487cc3.379d.4a53.a7e3.7769317718c2 | 0,00309815 | TRUE | 990 |
| fd9842b9.97da.4268.8068.28bbcd2420aa | -0,00367756 | FALSE | 2309 |
| X2aacdd95.7a2b.4f2f.b3e8.b89e04f9d463 | -0,09663154 | FALSE | 390 |
| X02478525.18c2.4661.9546.c12cae4271aa | -0,10419797 | FALSE | 624 |
| X0b9a1aae.432d.4766.bcf3.8fdceaca59c0 | -0,1097999 | FALSE | 1669 |
| eee92fb4.507e.42d1.9e48.f2920d4be42e | 0,19145325 | TRUE | 132 |
| X7e8b0f1c.cda8.4122.9176.b2d3681b4ebc | -0,16252396 | FALSE | 1925 |
| e52c3b95.e3de.43e8.ae1f.e23c4fd686d7 | 0,03329349 | FALSE | 279 |

| ID | ProstaTrend score | biochemicalRecurrence | bcrTime (days) |
|---|---|---|---|
| f1fcbaee.161e.41d2.bd9b.0c12f75d384f | 0,27029268 | TRUE | 86 |
| X49a3c7c4.4184.480f.8da0.484f5ab766b0 | -0,09992712 | FALSE | 1254 |
| X6768577c.f449.4f00.967a.ef68091f8f21 | -0,29797151 | FALSE | 782 |
| X47796408.e269.46cf.9aed.0d55162edcbb | -0,22796784 | FALSE | 595 |
| X208ef97d.5bbe.4ea0.afac.c71994aa593e | -0,00900907 | FALSE | 923 |
| a7051a73.72e1.485e.b114.7ee5051721e3 | 0,29478614 | FALSE | 134 |
| X69e8ff05.e31f.4e2e.8e4d.a00e20c968de | 0,15662764 | FALSE | 1261 |
| X917a0676.33b8.4108.bee6.095eecca1b1a | -0,00600746 | TRUE | 1194 |
| c4be32bb.d980.4b16.8ac9.d897c57b0dcf | 0,05721464 | FALSE | 187 |
| X3b97963f.b054.4cd8.aa51.e2a9f87c5b06 | 0,30916604 | FALSE | 128 |
| ef712a4f.5c67.4bcf.8719.a3778dbb890f | -0,1386856 | FALSE | 156 |
| X3c895149.46ec.474e.be20.fa4077eae75a | -0,11209354 | FALSE | 665 |
| c26bc295.4944.4953.8b68.1d5acf018361 | -0,05214894 | FALSE | 2211 |
| X65adb593.bb9d.4986.b38c.346e06932389 | -0,27465588 | FALSE | 304 |
| f0ff111b.1210.44c9.9120.ef7eddbbb623 | -0,0483411 | FALSE | 378 |
| baeffbf5.636c.4d64.b7d6.edcb91fceef3 | -0,07150353 | FALSE | 1272 |
| X9cf2a5a7.10cb.4a2e.b3e3.96045d13806c | 0,02117678 | FALSE | 31 |
| a3325a39.519f.420f.aef3.173f9e764da9 | 0,08977153 | FALSE | 131 |
| dcdfcb54.f24e.474a.8254.3f4ec3b2329d | 0,09119533 | FALSE | 91 |
| e3a5bd05.8cd3.4a63.a95e.6946a2d62232 | 0,1082672 | TRUE | 1925 |
| X55850461.c8a0.40b2.947b.8a7cbcf1d667 | -0,3387308 | FALSE | 196 |
| X6e621733.d346.4e30.bb9d.c85542b9a62f | -0,0960812 | FALSE | 76 |
| X5fb306e1.69f8.402d.9994.ec427fae5c69 | 0,15914627 | TRUE | 344 |
| e91c0303.6dd8.4d8c.a87f.ec6dab06f14e | -0,19063502 | FALSE | 1815 |
| X6577d05f.507e.46c6.b853.c2d062c5dde6 | 0,06634846 | FALSE | 512 |
| X21ebc131.0bdc.4dcb.900f.6da23466ad6a | 0,25022983 | FALSE | 393 |
| X9d5180ef.8c67.4bb9.b94f.04c503df6809 | -0,079336 | TRUE | 664 |
| X4c585af2.374f.45b7.80e7.d408799a3778 | -0,06888431 | FALSE | 822 |
| X96e75404.fc6f.4a3d.8edb.ae8f13048b31 | 0,06690739 | FALSE | 1117 |
| c65a3085.d849.4a52.8ede.d719d524524e | -0,3832915 | FALSE | 1061 |
| a3aad406.7607.4a23.9a44.0235334a53e2 | -0,20250988 | FALSE | 114 |
| X449fc55f.6a91.4f7f.9f80.ea6718d40c6e | -0,12158388 | FALSE | 386 |
| X2e8ac4a5.9da9.4b53.a293.616bedc2b796 | -0,18476672 | FALSE | 731 |
| X2f9f0580.3d06.4698.9f68.07b6b7f953f2 | -0,02924983 | FALSE | 217 |
| X514a93da.9c6d.4030.8ce2.d470861690d3 | 0,14063278 | FALSE | 1004 |
| X6fbf62b9.e790.4d92.a06f.91d8b03067a9 | 0,03363591 | FALSE | 1359 |
| X09f7ed63.183f.4159.9603.b36a9dfeca81 | -0,28179346 | FALSE | 972 |
| X017f1cd1.6003.4945.9ead.71c7aaaa1fc2 | 0,01417046 | FALSE | 842 |
| X824d450d.96e6.4ecd.9193.1cb47f4df83b | 0,22843474 | FALSE | 1439 |
| a94003bb.555d.4a61.8b2b.8125021c7553 | -0,03767007 | FALSE | 1253 |
| X6c7a4e11.b60a.4d87.9e7b.8a5093b49114 | -0,15770873 | FALSE | 617 |
| X0ecbfe7c.9fc5.4aa7.ad19.7a4bbaebe2bc | 0,09006177 | FALSE | 1386 |
| X2ed28850.36e0.4d48.96b8.416dabf57984 | 0,0370126 | TRUE | 204 |
| bdde7040.da1e.4f55.ad80.8da448282338 | 0,025278 | FALSE | 868 |
| X796ae53c.6829.4c91.9aaa.61c965b4b863 | -0,12766592 | FALSE | 1491 |
| X3cba8225.e66a.4b7d.a2c9.781c5cf15c39 | -0,10008482 | TRUE | 353 |
| X3dc6bae7.099f.4794.8583.f8903935305a | -0,03115484 | FALSE | 1280 |
| X2ad19836.1d07.4809.bc19.ffb71d812a69 | -0,03453954 | TRUE | 75 |
| X0f0c68db.cfb7.4017.9248.00410688a4cc | -0,02495689 | FALSE | 518 |
| da7a39df.730d.4653.8543.3792626b4e68 | 0,07642164 | FALSE | 178 |
| X31a4ac16.5eea.429a.929d.7bf445cd354d | 0,03341471 | FALSE | 662 |
| a4a420c8.a195.4077.b621.6f8bfa02b39b | -0,16391971 | FALSE | 476 |
| X9a24c74d.c93d.4feb.a999.cc657988cf59 | 0,02011091 | FALSE | 1891 |
| ab801648.fc2d.4c26.a0c3.942273627406 | -0,0010796 | FALSE | 788 |
| X96e7b38e.3100.49ee.8bf6.ed2bd4c4f2f2 | -0,17097542 | TRUE | 77 |
| c570b43b.afdb.4851.bafb.d0d1ec94e146 | -0,09673244 | FALSE | 1144 |
| X5da27db1.a960.4fa1.8d5b.ab2a7ef8bf49 | 0,1821241 | FALSE | 115 |
| X3b94c9f7.702e.4381.8fae.a3bf83577ba9 | -0,15159878 | FALSE | 481 |
| f643ec29.44c0.421b.a71c.42ed1243e504 | -0,02797046 | FALSE | 674 |

| ID | ProstaTrend score | biochemicalRecurrence | bcrTime (days) |
|---|---|---|---|
| X2452d829.320d.4881.a30e.fd056a631689 | 0,13871293 | FALSE | 113 |
| e202f97d.682c.49b8.aa03.e0118cfc87e4 | -0,28559317 | FALSE | 405 |
| fffaeee5.ad28.4ad0.87f2.4a3751976710 | 0,1172813 | TRUE | 197 |
| aa1f36f1.f18b.47a4.a1a4.130e384ba1fd | 0,20580038 | FALSE | 1124 |
| X21246b27.f4bd.433d.9a16.877feeed6dda | -0,2120006 | FALSE | 62 |
| X7e2f69a5.c03f.4c3b.9e26.39541bd90b78 | 0,1989655 | TRUE | 148 |
| a9fe3990.ab6f.436e.8073.b41fe7c2cf0e | 0,03402721 | FALSE | 643 |
| X5ea40cd0.1bbb.4efa.ab08.48bd9db590d0 | -0,12735667 | FALSE | 206 |
| fd68ea12.5c75.4bda.9946.279de3645a57 | -0,08747069 | FALSE | 2704 |
| X883ba65b.2958.4aff.9deb.e46743515bd2 | -0,11103382 | FALSE | 98 |
| X0f66dcb9.5f5b.4154.b784.e014f5c19b08 | 0,17370834 | FALSE | 854 |
| X92228116.53f0.49ec.9be1.4e487a6a5513 | -0,05654277 | FALSE | 295 |
| a48c1dbe.2163.4470.98e9.c638718f0fc7 | 0,37467788 | FALSE | 295 |
| b6b04c56.a41c.4d0b.82fa.76252407995a | -0,13605918 | FALSE | 213 |
| X64da5efc.47be.491d.b4b7.f0ae25b78f76 | 0,11584841 | FALSE | 2183 |
| X054a65fd.419f.442f.85da.e89f03ac2e88 | -0,20597569 | FALSE | 44 |
| b3b5d7e6.6ac1.4342.a4bf.aed2d834cb49 | 0,06564918 | FALSE | 774 |
| X581d16c4.038b.4d23.a989.e3455cc35f3c | -0,14443141 | FALSE | 212 |
| c4fb0452.2639.4021.b2ce.95647639b346 | -0,04909162 | FALSE | 762 |
| X1ab5ef46.4053.4f02.91d1.26541619140c | 0,22748963 | FALSE | 422 |
| X21c2cf99.21bf.4a0d.a312.19f312cd0f89 | -0,07968765 | FALSE | 66 |
| X02662f3c.f7ea.4251.bec8.f4ed6bdd1ff5 | -0,17519749 | FALSE | 731 |
| a0aac555.a15c.42d4.affa.a1ce5886dba8 | -0,02768208 | FALSE | 52 |
| X2cd0d540.55d2.4cf0.a734.71a6713c459a | -0,15656836 | FALSE | 922 |
| d4a4473e.3095.4461.aee2.8844f1470b27 | -0,09225461 | FALSE | 122 |
| X1b3ecd57.0440.47fc.8239.a6cd02ff3c1f | -0,13713943 | FALSE | 104 |
| X5ddaa9cb.f3a4.4e7b.8ed7.1d25820ece22 | 0,07211542 | TRUE | 196 |
| X218b3a45.cf88.4c93.93ef.f80e1812dcdc | -0,0124331 | FALSE | 60 |
| X33af60a6.4c1c.4dcf.abd2.7af4ab4b4b34 | 0,18640179 | FALSE | 2859 |
| X7d8cb922.946f.440c.b8d7.45cfe1bcd917 | -0,1260385 | FALSE | 2553 |
| e0e4b5db.6cc9.436c.be9b.ffc9a049069a | -0,10107394 | FALSE | 1247 |
| X8b522062.7dc5.4e57.83c0.10007bb55c9a | -0,04481944 | FALSE | 54 |
| X04b39224.14f5.47a9.b6b4.b5ab0c03765c | 0,52900438 | TRUE | 124 |
| X30f911b6.5bff.4a11.ac31.a786d672c9bc | 0,21747442 | TRUE | 679 |
| X31b45ace.34b1.491b.a926.7731df03b8da | 0,12369733 | FALSE | 1690 |
| X62eef687.e0d6.4232.a550.f62f10d373fb | -0,06880172 | FALSE | 352 |
| X7219d88d.e73b.4e2b.9da6.43b8ab38c046 | 0,44358403 | TRUE | 170 |
| d8779603.a18c.478e.a381.5ac204e9bc69 | -0,05427177 | FALSE | 204 |
| ddc6fa19.cb88.4fbe.a049.f671fee34f6b | 0,0610405 | FALSE | 287 |
| a3fd7f43.14e5.4dd8.9fa7.4b0084c0b047 | 0,09831684 | FALSE | 2040 |
| X1e6ba6f2.9ed4.422a.99a1.3c9f5c30c658 | 0,01251773 | FALSE | 1780 |
| cbf92875.c4c1.48ac.9d39.6aff23ce6967 | -0,10349731 | FALSE | 394 |
| X6866332e.ed55.4549.94cf.3c748ae2860e | 0,05700412 | FALSE | 3524 |

**Table 4**

| ID | score | DoD | Time |
|---|---|---|---|
| RIB0034827 | -0,05789742 | TRUE | 5,75 |
| RIB0034830 | -0,0927188 | FALSE | 7,5 |
| RIB0034227 | 0,157458 | FALSE | 15,17 |
| RIB0034207 | -0,05751573 | FALSE | 17,67 |
| RIB0034226 | -0,29029778 | FALSE | 11,83 |
| RIB0034303 | 0,43112756 | FALSE | 7,5 |
| RIB0034370 | 0,02653453 | TRUE | 2,17 |
| RIB0034373 | -0,16865389 | FALSE | 13,5 |
| RIB0034340 | -0,19655314 | FALSE | 11,42 |
| RIB0034372 | 0,15830856 | FALSE | 16,08 |
| RIB0034383 | -0,0477193 | FALSE | 14 |

| ID | score | DoD | Time |
|---|---|---|---|
| RIB0034385 | 0,33226352 | TRUE | 2,25 |
| RIB0034321 | -0,30761114 | FALSE | 9,83 |
| RIB0034483 | -0,2038041 | FALSE | 11,5 |
| RIB0034457 | -0,27047346 | FALSE | 11,17 |
| RIB0034485 | 0,16136275 | TRUE | 5,67 |
| RIB0034486 | 0,09473917 | FALSE | 10,08 |
| RIB0034477 | -0,01601988 | FALSE | 13,83 |
| RIB0034448 | -0,03331082 | TRUE | 4,5 |
| RIB0034471 | -0,31611722 | FALSE | 11,25 |
| RIB0034491 | 0,59098609 | TRUE | 3 |
| RIB0034942 | -0,06342756 | FALSE | 9,42 |
| RIB0034986 | 0,2157573 | TRUE | 11,83 |
| RIB0034913 | 0,40795809 | TRUE | 2,58 |
| RIB0034496 | -0,04660878 | FALSE | 9 |
| RIB0034489 | -0,0612437 | FALSE | 10,42 |
| RIB0034434 | -0,10203968 | FALSE | 10,58 |
| RIB0034497 | -0,16136283 | FALSE | 16,17 |
| RIB0034798 | -0,01599411 | FALSE | 12,5 |
| RIB0034731 | 0,16176971 | TRUE | 5,83 |
| RIB0034702 | -0,11889468 | FALSE | 9,75 |
| RIB0034918 | 0,48671153 | TRUE | 4,92 |
| RIB0034224 | 0,18108529 | FALSE | 16 |
| RIB0034214 | 0,01867188 | FALSE | 18,5 |
| RIB0034271 | -0,08930621 | FALSE | 15,58 |
| RIB0034258 | 0,05696045 | FALSE | 14,67 |
| RIB0034239 | 0,17545716 | TRUE | 7,17 |
| RIB0034276 | 0,19644116 | TRUE | 3,92 |
| RIB0034235 | -0,00710036 | FALSE | 15 |
| RIB0034299 | -0,15984869 | FALSE | 15,67 |
| RIB0034812 | -0,01306805 | FALSE | 9,33 |
| RIB0034875 | 0,32007422 | FALSE | 9,92 |
| RIB0034780 | -0,32837208 | FALSE | 5,42 |
| RIB0034794 | 0,35669265 | FALSE | 8,5 |
| RIB0034779 | -0,21654543 | FALSE | 8,42 |
| RIB0034777 | -0,03040986 | FALSE | 7,67 |
| RIB0034767 | -0,07727856 | TRUE | 7,5 |
| RIB0034886 | -0,1670564 | FALSE | 10,33 |
| RIB0034733 | -0,14612466 | TRUE | 7,5 |
| RIB0034776 | -0,16581573 | FALSE | 10,75 |
| RIB0034772 | 0,02472629 | FALSE | 9,25 |
| RIB0034759 | -0,08672834 | FALSE | 11 |
| RIB0034783 | 0,19991793 | FALSE | 10,17 |
| RIB0034841 | 0,04298723 | FALSE | 9,83 |
| RIB0034822 | 0,02622539 | FALSE | 9,33 |
| RIB0034826 | 0,00416608 | FALSE | 9,58 |
| RIB0034819 | -0,04877873 | FALSE | 9,17 |
| RIB0034818 | -0,13179823 | FALSE | 9,92 |
| RIB0034821 | -0,00089025 | FALSE | 6,5 |
| RIB0034864 | 0,09761921 | FALSE | 9,75 |
| RIB0034810 | -0,00591527 | FALSE | 6,92 |
| RIB0034900 | -0,02190402 | FALSE | 8,25 |
| RIB0034254 | 0,1615697 | FALSE | 14,83 |
| RIB0034269 | -0,19527884 | FALSE | 14,42 |
| RIB0034282 | -0,29878035 | FALSE | 13,17 |
| RIB0034233 | 0,17694829 | FALSE | 7,75 |
| RIB0034274 | -0,12017116 | FALSE | 15,75 |
| RIB0034289 | 0,18938624 | FALSE | 10,92 |
| RIB0034242 | 0,03209577 | FALSE | 14,5 |
| RIB0034208 | 0,00586279 | FALSE | 14,58 |
| RIB0034202 | 0,54033332 | TRUE | 3 |

| ID | score | DoD | Time |
|---|---|---|---|
| RIB0034312 | -0,23324581 | FALSE | 7,17 |
| RIB0034388 | 0,0331019 | FALSE | 14,42 |
| RIB0034361 | -0,05486132 | FALSE | 15,17 |
| RIB0034353 | 0,01057841 | FALSE | 11,25 |
| RIB0034378 | 0,07011301 | FALSE | 9 |
| RIB0034360 | 0,03752312 | FALSE | 9,67 |
| RIB0034392 | -0,04338172 | FALSE | 10,67 |
| RIB0034356 | -0,24279187 | FALSE | 10,08 |
| RIB0034359 | -0,15776497 | FALSE | 11,5 |
| RIB0034320 | 0,1625773 | FALSE | 11,33 |
| RIB0034390 | -0,06606545 | FALSE | 11,92 |
| RIB0034377 | 0,08598428 | TRUE | 4,5 |
| RIB0034323 | -0,06391046 | TRUE | 13,08 |
| RIB0034315 | -0,02720597 | FALSE | 8,08 |
| RIB0034342 | -0,05660906 | FALSE | 14,58 |
| RIB0034306 | 0,02515252 | FALSE | 15,83 |
| RIB0034387 | -0,09241328 | FALSE | 11,75 |
| RIB0034459 | 0,15011723 | FALSE | 15,42 |
| RIB0034326 | -0,01080281 | FALSE | 9,33 |
| RIB0034479 | -0,02030004 | FALSE | 12,17 |
| RIB0034458 | -0,26975921 | FALSE | 9,75 |
| RIB0034484 | -0,11303634 | FALSE | 10,75 |
| RIB0034422 | 0,43178883 | TRUE | 11,33 |
| RIB0034500 | -0,18991177 | FALSE | 11,33 |
| RIB0034425 | -0,03325826 | FALSE | 8,33 |
| RIB0034475 | -0,33124847 | FALSE | 10,25 |
| RIB0034423 | 0,03256894 | FALSE | 9,58 |
| RIB0034432 | -0,17597713 | FALSE | 8,58 |
| RIB0034478 | -0,28777526 | FALSE | 11 |
| RIB0034462 | 0,09348845 | FALSE | 11,17 |
| RIB0034429 | 0,03320036 | TRUE | 9,17 |
| RIB0034408 | -0,19825076 | FALSE | 14,42 |
| RIB0034428 | -0,0927142 | FALSE | 9,67 |
| RIB0034450 | 0,0894301 | FALSE | 9,25 |
| RIB0034480 | -0,03457921 | FALSE | 7,75 |
| RIB0034966 | -0,13038993 | FALSE | 10,92 |
| RIB0034989 | -0,0143567 | FALSE | 9,92 |
| RIB0034904 | -0,02259085 | FALSE | 9,25 |
| RIB0034959 | -0,34784808 | FALSE | 8,58 |
| RIB0034976 | 0,08573291 | FALSE | 9,5 |
| RIB0034961 | -0,39381562 | FALSE | 9 |
| RIB0034964 | -0,10067456 | FALSE | 15,5 |
| RIB0034905 | 0,04200649 | FALSE | 9,92 |
| RIB0034951 | -0,06248583 | FALSE | 9,83 |
| RIB0034936 | -0,24486439 | FALSE | 9,33 |
| RIB0034464 | 0,21390358 | TRUE | 8,08 |
| RIB0034470 | -0,00212083 | FALSE | 9,42 |
| RIB0034469 | 0,24416094 | TRUE | 1,75 |
| RIB0034455 | 0,13190175 | FALSE | 8,83 |
| RIB0034415 | 0,11077901 | FALSE | 11,25 |
| RIB0034414 | -0,09734744 | FALSE | 12,67 |
| RIB0034403 | -0,23660935 | FALSE | 10,17 |
| RIB0034409 | -0,25741511 | FALSE | 9,67 |
| RIB0034401 | -0,18702123 | FALSE | 8,25 |
| RIB0034756 | -0,0698472 | FALSE | 8,5 |
| RIB0034711 | -0,11427597 | FALSE | 7,83 |
| RIB0034727 | -0,00501315 | TRUE | 14,83 |
| RIB0034762 | 0,0289554 | FALSE | 7,17 |
| RIB0034747 | -0,33187558 | FALSE | 7,75 |
| RIB0034751 | 0,20623219 | FALSE | 11,75 |

| ID | score | DoD | Time |
|---|---|---|---|
| RIB0034736 | 0,27911479 | FALSE | 9,58 |
| RIB0034729 | -0,12219397 | FALSE | 7,5 |
| RIB0034683 | -0,17262447 | FALSE | 8,83 |
| RIB0034914 | -0,07394617 | FALSE | 12,33 |
| RIB0034880 | -0,12808717 | FALSE | 10,08 |
| RIB0034849 | 0,05472914 | FALSE | 7,58 |
| RIB0034882 | -0,27831219 | FALSE | 11,25 |
| RIB0034858 | 0,34480312 | TRUE | 4,75 |
| RIB0034878 | -0,17058148 | FALSE | 9,92 |
| RIB0034833 | 0,05538858 | FALSE | 9,08 |
| RIB0034984 | 0,31767099 | FALSE | 11,08 |
| RIB0034972 | -0,05405458 | FALSE | 10,5 |
| RIB0034979 | 0,10648813 | FALSE | 9,5 |
| RIB0034985 | -0,20125755 | FALSE | 8,67 |
| RIB0034946 | -0,16743797 | FALSE | 10,5 |
| RIB0034944 | 0,10402747 | FALSE | 7,58 |
| RIB0034927 | -0,2178406 | TRUE | 4,92 |
| RIB0034954 | -0,36889795 | FALSE | 9,08 |
| RIB0034956 | -0,14408289 | FALSE | 9,92 |
| RIB0034916 | 0,07529848 | FALSE | 7,5 |
| RIB0034983 | 0,00829798 | FALSE | 11 |
| RIB0034938 | 0,41425397 | TRUE | 4,5 |
| RIB0034921 | -0,09130398 | FALSE | 7,67 |
| RIB0034928 | 0,25300678 | FALSE | 10,42 |
| RIB0034957 | -0,2598783 | FALSE | 8,08 |
| RIB0034939 | -0,20514336 | FALSE | 5,75 |
| RIB0034941 | -0,1629562 | FALSE | 11,25 |
| RIB0034998 | -0,15290247 | FALSE | 7,5 |
| RIB0034204 | 0,02026786 | FALSE | 16,33 |
| RIB0034297 | -0,0406671 | FALSE | 16,25 |
| RIB0034244 | -0,13457864 | FALSE | 17,33 |
| RIB0034265 | -0,23422099 | FALSE | 14 |
| RIB0034223 | -0,30002604 | FALSE | 14,08 |

**Table 5**

| | | logHR [95% CI] | p-value |
|---|---|---|---|
| univariate | **ProstaTrend** | **4.11 [2.38-5.84]** | **3.3e-06** |
| | PSA (log scale) | 0.38 [-0.05-0.80] | 0.084 |
| | Gleason Grading (RP GG≥3) | 2.53 [1.11-3.95] | 0.0005 |
| | Pathological stage (stage≥T3) | 2.46 [1.03-3.88] | 0.0007 |
| | pN | 1.05 [0.27-1.83] | 0.008 |
| | Age (continious) | 0.04 [-0.01-0.08] | 0.14 |
| | Resection status (R+) | 0.53 [-0.08-1.14] | 0.09 |
| pairwise | **ProstaTrend** PSA | **3.59 [1.74-5.44]** 0.36 [-0.05-0.77] | **0.0001** 0.087 |
| | **ProstaTrend** Gleason GG≥3 | **2.58 [0.64-4.52]** 2.09 [0.62-3.55] | **0.009** 0.005 |
| | **ProstaTrend** Pathological stage | **3.01 [1.20-4.82]** 2.11 [0.67-3.55] | **0.001** 0.004 |
| | **ProstaTrend** pN | **3.35 [1.41-5.28]** 0.55 [-0.27-1.37] | **0.0007** 0.19 |
| | **ProstaTrend** Age | **3.99 [2.23-5.75]** 0.02 [-0.03-0.06] | **9.1e-06** 0.50 |
| | **ProstaTrend** Resection status | **3.98 [2.19-5.76]** 0.21 [-0.43-0.84] | **1.2e-05** 0.52 |
| complex model | **ProstaTrend** Gleason score Pathological stage | **2.00 [0.01-3.98]** 1.65 [0.16-3.14] 1.76 [0.31-3.21] | **0.049** 0.030 0.017 |

Europäisches
Patentamt

European
Patent Office

Office européen
des brevets

# EUROPEAN SEARCH REPORT

Application Number

EP 20 17 0321

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/303002 A1 (SHAK STEVEN [US] ET AL) 9 October 2014 (2014-10-09) * MYBL2, MMP11, NEIL3, FANCA; claims 1,14; tables 10, A * | 1-9 | INV.<br>C12Q1/6886<br>G16B25/10 |
| X | US 2015/167103 A1 (STONE STEVEN [US] ET AL) 18 June 2015 (2015-06-18) * MCM2, MYBL2, PLK4, FANCA; claim 5; tables 1, 23 * | 1-9 | |
| X | CA 2 891 653 A1 (MYRIAD GENETICS INC [US]) 22 May 2014 (2014-05-22) * MCM2, MYBL2, PLK4, FANCA; claims 1,13; table 9 * | 1-9 | |
| X | KLEIN ERIC A ET AL: "Decipher Genomic Classifier Measured on Prostate Biopsy Predicts Metastasis Risk", UROLOGY, BELLE MEAD, NJ, US, vol. 90, 22 January 2016 (2016-01-22), pages 148-152, XP029483982, ISSN: 0090-4295, DOI: 10.1016/J.UROLOGY.2016.01.012 * the whole document * | 1,2,5-9 | |
| X | DEJAN KNEZEVIC ET AL: "Analytical validation of the Oncotype DX prostate cancer assay - a clinical RT-PCR assay optimized for prostate needle biop", BMC GENOMICS, BIOMED CENTRAL, vol. 14, no. 1, 8 October 2013 (2013-10-08), page 690, XP021165824, ISSN: 1471-2164, DOI: 10.1186/1471-2164-14-690 * the whole document * | 1,2,5-9 | |

TECHNICAL FIELDS SEARCHED (IPC)

C12Q
G06F
G16B

-/--

The present search report has been drawn up for all claims

| Place of search | Date of completion of the search | Examiner |
|---|---|---|
| The Hague | 11 September 2020 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

**EUROPEAN SEARCH REPORT**

Application Number

EP 20 17 0321

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | J CUZICK ET AL: "Prognostic value of an RNA expression signature derived from cell cycle proliferation genes in patients with prostate cancer: a retrospective study", THE LANCET. ONCOLOGY, vol. 12, no. 3, 1 March 2011 (2011-03-01), pages 245-255, XP055275747, DOI: 10.1016/S1470-2045(10)70295-3 * Prolaris; the whole document * | 1,2,5-9 | |
| T | KREUZ MARKUS ET AL: "ProstaTrend-A Multivariable Prognostic RNA Expression Score for Aggressive Prostate Cancer", EUROPEAN UROLOGY, ELSEVIER, AMSTERDAM, NL, vol. 78, no. 3, 4 July 2020 (2020-07-04), pages 452-459, XP086250312, ISSN: 0302-2838, DOI: 10.1016/J.EURURO.2020.06.001 [retrieved on 2020-07-04] | | |

TECHNICAL FIELDS SEARCHED (IPC)

The present search report has been drawn up for all claims

| Place of search | Date of completion of the search | Examiner |
|---|---|---|
| The Hague | 11 September 2020 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 0321

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2020

| Patent document cited in search report | Publication date | Patent family member(s) | | Publication date |
|---|---|---|---|---|
| US 2014303002 A1 | 09-10-2014 | AU | 2013215448 A1 | 21-08-2014 |
| | | AU | 2018201688 A1 | 05-04-2018 |
| | | AU | 2020202164 A1 | 16-04-2020 |
| | | CA | 2863040 A1 | 08-08-2013 |
| | | CO | 7051010 A2 | 10-09-2014 |
| | | DK | 3179393 T3 | 27-07-2020 |
| | | EP | 2809812 A1 | 10-12-2014 |
| | | EP | 3179393 A2 | 14-06-2017 |
| | | HK | 1201881 A1 | 11-09-2015 |
| | | IL | 233709 A | 28-09-2017 |
| | | IL | 254255 A | 29-11-2018 |
| | | JP | 6351112 B2 | 04-07-2018 |
| | | JP | 2015511814 A | 23-04-2015 |
| | | JP | 2017113008 A | 29-06-2017 |
| | | JP | 2019013255 A | 31-01-2019 |
| | | MX | 351626 B | 23-10-2017 |
| | | MX | 366164 B | 01-07-2019 |
| | | NZ | 627887 A | 26-08-2016 |
| | | NZ | 722902 A | 22-12-2017 |
| | | SG | 10201605210P A | 30-08-2016 |
| | | SG | 10201912312Y A | 27-02-2020 |
| | | SG | 11201404390W A | 28-08-2014 |
| | | US | 2013196321 A1 | 01-08-2013 |
| | | US | 2014303002 A1 | 09-10-2014 |
| | | WO | 2013116144 A1 | 08-08-2013 |
| US 2015167103 A1 | 18-06-2015 | CA | 2804391 A1 | 12-01-2012 |
| | | EP | 2591126 A2 | 15-05-2013 |
| | | US | 2012053253 A1 | 01-03-2012 |
| | | US | 2015167103 A1 | 18-06-2015 |
| | | US | 2016355884 A1 | 08-12-2016 |
| | | US | 2018334722 A1 | 22-11-2018 |
| | | WO | 2012006447 A2 | 12-01-2012 |
| CA 2891653 A1 | 22-05-2014 | CA | 2891653 A1 | 22-05-2014 |
| | | EP | 2920322 A1 | 23-09-2015 |
| | | US | 2015247208 A1 | 03-09-2015 |
| | | WO | 2014078700 A1 | 22-05-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CULLEN et al.** *Eur Urol,* 2015
- **ERHO et al.** *PLoS ONE,* 2013
- **CUZICK et al.** *Br J Cancer,* 2015
- **2011.** *Lancet Oncol*
- **MCSHANE et al.** *J Natl Cancer Inst.,* 2005
- **HANAHAN et al.** *Cell,* 2011
- **LOEFFLER-WIRTH et al.** *Genome Med.,* 2019